# EUROPÄISCHE PATENTSCHRIFT

(11) **EP 3 313 501 B1**
(45) Veröffentlichungstag und Bekanntmachung des Hinweises auf die Patenterteilung: **01.01.2025**
(21) Anmeldenummer: 16734614.7
(22) Anmeldetag: 29.06.2016
(51) Int. Cl.: A61M 39/10, F16L 37/12, G01L 19/00, G01L 19/06, G01L 19/14

(54) **VERBINDUNGSSYSTEM**
CONNECTION SYSTEM
SYSTÈME D'ASSEMBLAGE

(30) Priorität: 29.06.2015 DE 202015103406 U
(43) Veröffentlichungstag der Anmeldung: 02.05.2018
(73) Patentinhaber: Sartorius Stedim Biotech GmbH, 37079 Göttingen (DE)
(72) Erfinder: SCHOLZ, Jochen, 37077 Göttingen (DE); PURMANN, Sebastian, 37073 Göttingen (DE); BATES, Michael, Gloucestershire GL5 2BL (GB)
(74) Vertreter: Novagraaf International SA
(86) Internationale Anmeldenummer: PCT/EP2016/065182
(87) Internationale Veröffentlichungsnummer: WO 2017/001504

(56) Entgegenhaltungen:
- EP-A1- 2 252 202
- WO-A1-2006/117138
- WO-A1-2007/063390
- DE-A1- 102008 015 322
- GB-A- 393 699
- US-A1- 2003 030 272
- US-A1- 2003 200 812
- US-A1- 2013 042 692

## Beschreibung

### TECHNISCHES GEBIET

Die vorliegende Offenbarung betrifft ein Verbindungssystem, insbesondere ein Verbindungssystem zum lösbar kraftschlüssigen Koppeln eines Körpers mit einem fluidführenden System über eine Membran oder zum lösbaren kraftschlüssigen Koppeln zweier fluidführender Körper. In einigen Ausführungsformen dient das Verbindungssystem dem Koppeln eines Drucksensors und eines Druckaufnehmers. Offenbart ist auch ein Verfahren, in dem das Verbindungssystem eingesetzt wird.

### HINTERGRUND

Die folgende Diskussion des Hintergrunds der Offenbarung wird lediglich bereitgestellt, um dem Leser das Verständnis zu erleichtern, und ist kein Eingeständnis, Stand der Technik der zu beschreiben oder darzustellen.

Für biotechnologische Herstellungsverfahren sind sterile und aseptische Verbindungen zwischen Reaktionsgefäßen und Apparaturen, Messgeräten etc. von hoher Bedeutung. Da Prozesse unter aseptischen Bedingungen zu erfolgen haben, müssen sterile Verbindungen und Leitungen auf eine Weise vorgesehen sein, dass sowohl im Betrieb als auch beim vorherigen Verbinden keine Kontaminationen durch Umwelteinflüsse erfolgen können. Beispielsweise erfordern Einwegkunststoffbeutel mit flexiblen Thermoplastleitungen Verbindungen, die sicherstellen, dass sowohl Beutel als auch Leitungen steril bleiben.

Drucksensoren werden für eine Vielzahl von technischen Strömungsvorgängen zur Überwachung und Regelung eingesetzt. Mit der Verbreitung von Einwegvorrichtungen in der biopharmazeutischen Industrie wird auch der Einsatz von Einwegsensoren zur Druckmessung immer wichtiger. Wichtige Einsatzgebiete sind dabei unter anderem Cross-Flow oder Filtrationsanwendungen. Das Europäische Patent EP 2 252 202 offenbart ein Verbindungselement, das für einen Einwegdruckaufnehmer geeignet ist. Das entsprechende Systemelement dient der lösbaren abgedichteten Verbindung eines Messwertaufnehmers mit einem Fluidsystem. Der Druckaufnehmer gibt als durchströmter abgeschlossener Teil des Fluidsystems die Druckkräfte über eine Membran an einen Messaufnehmer, z.B. mit Piezo-Kraftmesser, weiter.

Einweg-Drucksensoren sind typischerweise aus Kunststoff gefertigt, während der betreffende Messkopf in der Regel Metall wie z.B. Edelstahl aufweist. Die Verbindung eines Einweg-Drucksensors mit einem fest installierten Messkopf aus Metall muss einerseits leicht durchzuführen sein und andererseits eine feste und definierte Verpressung zueinander gewährleisten.

Zu diesem Zweck werden üblicherweise Hilfsvorrichtungen bzw. Hilfsmittel wie z.B. eine Haube und Verriegelungsstifte vorgesehen, um die erforderliche Messgenauigkeit und Reproduzierbarkeit zu erreichen. Diese Hilfsvorrichtungen sind allerdings in der Regel komplex in der Handhabung und bergen das Risiko einer Fehlbedienung durch den Anwender. Auf diese Weise kann es zu ungenauen Messungen kommen. Auch ist der Zeitaufwand für die Installation bzw. De-Installation hoch.

DE 10 2008 015322 offenbart ein Systemelement zur lösbaren abgedichteten Verbindung eines Messwertaufnehmers mit einem Fluidsystem, mit einer mit dem Fluidsystem durchströmbar verbindbaren Messkammer. WO 2007/063390 offenbart eine abnehmbare hydraulische Schnellverbindung zum Verbinden einer ersten und einer zweiten Leitung, von denen mindestens eine durch eine Kunststoffleitung definiert ist. US 2003/030272 offenbart eine Vorrichtung zum Herstellen einer aseptischen/sterilen Verbindung. Die Vorrichtung weist eine im Wesentlichen flexible, im Wesentlichen transparente sterile Barriere auf, die ein terminales Ende einer Leitung umschließt, sowie eine elastische, verformbare Trägerkarte, die weiterhin einen mit einem Trennpapier bedeckten Kleberand und eine Rollmembran mit einem kontinuierlichen, entfernbaren, nachgiebigen, flexiblen Streifenmaterial aufweist. WO 2006/117138 offenbart einen Verbinder mit einer Fluiddurchführung, einer Kontakteinrichtung mit einer im wesentlichen planen Kontaktfläche und einer Fluiddurchführungsöffnung, welche zur Durchführung von Fluid ausgelegt ist.

GB 393 699 offenbart Kupplungsköpfe für biegsame Rohrkupplungen mit einer Verriegelungsvorrichtung, die dazu ausgelegt ist, die relative Drehung des Kupplungskopfes relativ zu seinem Gegenkupplungskopf in einer Richtung zu verhindern. US 2003/200812 offenbart einen Fluiddrucksensor, der ein Gehäuse mit einer ersten Membran, einen im Inneren des Gehäuses angeordneten Wandler, einen abnehmbar mit dem Gehäuse verbundenen Körper mit einem Fluideinlass und einem Fluidauslass, sowie eine zweite Membran, die ein integriertes Dichtungselement enthält und mindestens einen Teil der ersten Membran berührt, wenn der Körper mit dem Gehäuse verbunden ist. US 2013/0042692 offenbart eine Membran für einen Druckdom, die eine elastische kreisförmige Wand aufweist, die geeignet ist, eine Seite des Druckdoms zu schließen, um eine Trennung zwischen dem Inneren des Druckdoms und der Außenseite zu definieren, sowie einen kreisförmigen Rand, der dazu geeignet ist, mit einem Hauptkörper des Druckdoms verbunden zu werden.

### ZUSAMMENFASSUNG

Die vorliegende Erfindung betrifft ein Verbindungssystem zum lösbar kraftschlüssigen Koppeln sowie ein darauf beruhendes Verfahren gemäß den unabhängigen Ansprüchen.

Eine hier offenbarte Verbindung bietet in typischen Ausführungsformen eine robuste und definierte mechanische Ankopplung einer Einweg-Komponente an einen wiederverwendbaren Körper wie einen Messaufnehmer bei einfacher Montage. In einigen Ausführungsformen ist diese Montage sogar mit einer Hand sicher und zuverlässig durchführbar. Eine hier offenbarte Verbindung bietet typischerweise auch eine robuste und definierte mechanische Ankopplung zweier Einweg-Komponenten aneinander.

Gemäß einem ersten Aspekt ist ein Verbindungssystem zum lösbar kraftschlüssigen Koppeln eines ersten Körpers an ein fluidführendes System über eine fluiddichte Membran offenbart. Ein entsprechendes Fluid kann ein Gas oder eine Flüssigkeit sein. Das Verbindungssystem weist die fluiddichte Membran auf. Das Verbindungssystem weist des Weiteren einen zweiten Körper auf. Der zweite Körper weist einen Innenraum auf, der an das fluidführende System gekoppelt ist. Der zweite Körper ist typischerweise mit dem fluidführenden System verbunden. In diesem Fall ist der Innenraum des zweiten Körpers in der Regel mit dem Fluid des fluidführenden Systems gefüllt. Der zweite Körper weist auch eine Mündung auf. Die Mündung kommuniziert mit dem Innenraum des zweiten Körpers. Diese Mündung kann beispielsweise ein Auslass, inklusive einer Öffnung, sein. Diese Mündung ist in der Regel eine Mündung des Innenraums des zweiten Körpers. Der Innenraum des zweiten Körpers mündet typischerweise in eine Öffnung. Darüber hinaus weist das Verbindungssystem ein scheibenförmiges Halteelement auf, das auch in den zweiten Körper als flanschartiges Ende integriert sein kann. In diesem Fall definiert das zweite Ende des zweiten Körpers ein flanschartiges, scheibenförmiges Halteelement mit einem peripheren Außenrand. Der periphere Außenrand ist dem Innenraum des zweiten Körpers abgewandt. Weiterhin weist das Verbindungssystem ein lösbares Verbindungselement auf.

Das Verbindungssystem gemäß dem ersten Aspekt ist dazu ausgelegt, ein kraftschlüssiges Koppeln mit einem ersten Körper zu ermöglichen. Ein solcher erster Körper weist ein erstes Ende mit einem Flansch auf. Der zweite Körper weist ein zweites Ende mit einem Innenraum und einer umlaufenden Wand auf. Wie bereits angegeben, weist das Verbindungssystem in einigen Ausführungsformen ein scheibenförmiges Halteelement auf, während in einigen Ausführungsformen der zweite Körper in ein flanschartiges, scheibenförmiges Halteelement mündet. In Ausführungsformen, in denen das scheibenförmige Halteelement ein eigenständiges Bauteil definiert, weist es einen Durchlass auf. Ein solches eigenständiges scheibenförmiges Halteelement weist eine erste Seite, eine zweite Seite und einen peripheren Außenrand auf. Die erste Seite und/oder der Durchlass des scheibenförmigen Halteelements ist bzw. sind an die Membran gekoppelt. Beispielsweise können die erste Seite und/oder der Durchlass des scheibenförmigen Halteelements mit der Membran verbunden sein. In Ausführungsformen, in denen das scheibenförmige Halteelement ein eigenständiges Bauteil definiert, ist die erste Seite des scheibenförmiges Halteelement weiterhin typischerweise mit dem zweiten Ende des zweiten Körpers in Kontakt. Die zweite Seite des Halteelements ist in der Regel für einen flächigen Kontakt mit dem Flansch des ersten Körpers ausgelegt, beispielsweise auf dem Flansch aufzuliegen. Sie kann dazu ausgelegt sein, flächig mit dem Flansch des ersten Körpers verbunden zu werden. Auch in Ausführungsformen, in denen das flanschartige scheibenförmige Halteelement das zweite Ende des zweiten Körpers definiert, weist das Halteelement in der Regel eine Seite auf, die für einen flächigen Kontakt mit dem Flansch des ersten Körpers ausgelegt ist, beispielsweise auf dem Flansch aufzuliegen und/oder flächig mit dem Flansch des ersten Körpers verbunden zu werden. In Ausführungsformen, in denen das flanschartige scheibenförmige Halteelement das zweite Ende des zweiten Körpers definiert, kontaktiert in der Regel das flanschartige scheibenförmige Halteelement die Membran.

In einem montierten Zustand ist der erste Körper an den zweiten Körper gekoppelt und durch Arretierung am zweiten Körper fixiert. Dabei ist in der Regel zumindest teilweise zwischen dem zweiten Ende, das vom zweiten Körper definiert wird, und dem ersten Ende, das vom ersten Körper definiert wird, die Membran angeordnet. In Ausführungsformen, in denen ein scheibenförmiges Halteelement ein eigenes Bauteil definiert, ist auch dieses scheibenförmige Halteelement zwischen dem zweiten Ende, das vom zweiten Körper definiert wird, und dem ersten Ende, das vom ersten Körper definiert wird, angeordnet. Ein solcher erster Körper weist ein erstes Ende mit einem Flansch auf. In Ausführungsformen, in denen ein flanschartiges scheibenförmiges Halteelement das zweite Ende des zweiten Körpers definiert, ist in der Regel die Geometrie dieses zweiten Endes an die Geometrie des ersten Endes angepasst. In einigen Ausführungsformen ist die Geometrie dieses zweiten Endes an die Geometrie des ersten Endes so weit angepasst, so dass ein fluiddichtes Koppeln möglich ist. Auch in einigen Ausführungsformen, in denen ein scheibenförmiges Halteelement ein eigenständiges Bauteil definiert, kann die Geometrie des zweiten Endes an die Geometrie des ersten Endes angepasst sein.

In einigen Ausführungsformen, beispielsweise in Ausführungsformen, in denen das zweite Ende des zweiten Körpers ein flanschartiges, scheibenförmiges Halteelement definiert, enthält der erste Körper typischerweise einen Innenraum. In einem hier offenbarten Verbindungssystem, z.B. in einem System, in dem das zweite Ende des zweiten Körpers ein flanschartiges, scheibenförmiges Halteelement definiert, kann der der erste Körper auch fluidführend sein. In einigen Ausführungsformen, beispielsweise in Ausführungsformen, in denen das zweite Ende des zweiten Körpers ein flanschartiges, scheibenförmiges Halteelement definiert, enthält der erste Körper typischerweise einen Innenraum und ist fluidführend. In einigen Ausführungsformen enthält der erste Körper einen Innenraum, ist jedoch nicht fluidführend. Beispielsweise kann in Ausführungsformen, in denen das scheibenförmige Halteelement ein eigenes Bauteil definiert, der erste Körper einen Innenraum aufweisen, jedoch nicht fluidführend sein. In einigen Ausfiihrungsformen definiert das scheibenförmige Halteelement ein eigenes Bauteil und der erste Körper weist keinen Innenraum auf.

Wie bereits angegeben, enthält das Verbindungssystem des Weiteren ein lösbares Verbindungselement. Das lösbare Verbindungselement ist dazu ausgelegt, den peripheren Außenrand des scheibenförmigen Halteelements bzw. des flanschartigen scheibenförmigen Halteelements und den Flansch des ersten Körpers zumindest teilweise zu umgreifen. Auf diese Weise lässt sich das scheibenförmige Halteelement mit Hilfe des Verbindungselements kraftschlüssig am ersten Körper fixieren.

In typischen Ausführungsformen weist die umlaufende Wand des zweiten Körpers einen um die Mündung, z.B. die Öffnung, umlaufenden Membrankontaktabschnitt auf, der die Membran kontaktiert oder mit der Membran verbunden ist. Der umlaufende Membrankontaktabschnitt ist in der Regel so ausgerichtet, dass er dem ersten Körper zugewandt ist. Der umlaufende Membrankontaktabschnitt kann in einigen Ausführungsformen vollständig die Seite der umlaufenden Wand definieren, die dem Flansch des ersten Körpers zugewandt ist. In solchen Ausführungsformen kontaktiert typischerweise die gesamte dem Flansch des ersten Körpers zugewandte Seite der umlaufenden Wand die Membran oder ist an diese gekoppelt. Es kann auch im Wesentlichen die gesamte dem Flansch des ersten Körpers zugewandte Seite der umlaufenden Wand die Membran kontaktieren. Beispielsweise kontaktiert im Wesentlichen die gesamte dem Flansch des ersten Körpers zugewandte Seite der umlaufenden Wand die Membran oder es kontaktiert die gesamte dem Flansch des ersten Körpers zugewandte Seite der umlaufenden Wand vollständig die Membran. In einigen Ausführungsformen definiert der umlaufende Membrankontaktabschnitt nicht vollständig die Seite der umlaufenden Wand, die dem Flansch des ersten Körpers zugewandt ist. In solchen Ausfiihrungsformen weist diese Seite der umlaufenden Wand einen weiteren Wandabschnitt auf. Beispielsweise kann in Ausführungsformen, in denen der zweite Körper in ein flanschartiges, scheibenförmiges Halteelement mündet, das flanschartige, scheibenförmige Halteelement den um die Mündung umlaufenden Membrankontaktabschnitt sowie einen weiteren Abschnitt enthalten, der nicht die Membran kontaktiert. Dieser weitere Abschnitt, der nicht die Membran kontaktiert, grenzt in einigen Ausführungsformen an den Membrankontaktabschnitt an. In einigen Ausführungsformen grenzt dieser weitere Abschnitt nicht an den Membrankontaktabschnitt an. In Ausführungsformen, in denen neben dem Membrankontaktabschnitt ein weiterer Wandabschnitt der umlaufenden Wand dem Flansch des ersten Körpers zugewandt ist, kontaktiert in der Regel nur ein Teilbereich der dem Flansch des ersten Körpers zugewandten Seite der umlaufenden Wand die Membran.

In einigen Ausführungsformen, in denen ein scheibenförmiges Halteelement vorhanden ist, das ein eigenständiges Bauteil definiert, weist der zweite Körper des Weiteren einen um die umlaufende Wand umlaufenden Rücksprung auf. In einigen Ausführungsformen, in denen das scheibenförmige Halteelement ein flanschartiges Ende des zweiten Körpers definiert, ist auf der umlaufenden Wand ein Rücksprung angeordnet.

In einigen Ausführungsformen, in denen ein eigenständiges Halteelement als unabhängiges Bauteil vorhanden ist, ist auf der ersten Seite des scheibenförmigen Halteelements eine umlaufende Wand angeordnet, die mit der umlaufenden Wand des zweiten Körpers verbunden ist. Die umlaufende Wand auf der ersten Seite des scheibenförmigen Halteelements und die umlaufende Wand des zweiten Körpers weisen in typischen Ausführungsformen Geometrien auf, die einen umlaufen Kontakt der beiden Wände erlauben. Beispielsweise kann die umlaufende Wand des scheibenförmigen Halteelements eine Fläche aufweisen, die die umlaufende Wand des zweiten Körpers im Wesentlichen vollständig umlaufend kontaktiert. Ebenso kann die umlaufende Wand des zweiten Körpers eine Fläche aufweisen, die die umlaufende Wand des scheibenförmigen Halteelements im Wesentlichen vollständig umlaufend kontaktiert. In einigen Ausführungsformen weist die umlaufende Wand des scheibenförmigen Halteelements eine Fläche auf, die die umlaufende Wand des zweiten Körpers vollständig umlaufend kontaktiert. In einigen Ausführungsformen weist die umlaufende Wand des zweiten Körpers eine Fläche auf, die die umlaufende Wand des scheibenförmigen Halteelements vollständig umlaufend kontaktiert. In einigen Ausführungsformen weist die umlaufende Wand des scheibenförmigen Halteelements eine Fläche auf, die eine Fläche der umlaufenden Wand des zweiten Körpers im Wesentlichen vollständig umlaufend kontaktiert. In einigen Ausführungsformen weist die umlaufende Wand des scheibenförmigen Halteelements eine Fläche auf, die eine Fläche der umlaufenden Wand des zweiten Körpers vollständig umlaufend kontaktiert. In einigen Ausfiihrungsformen kann eine umlaufende Fläche der umlaufenden Wand des zweiten Körpers im Wesentlichen vollständig mit der ersten Seite des scheibenförmigen Halteelements verbunden sein. Eine umlaufende Fläche der umlaufenden Wand des zweiten Körpers kann auch vollständig mit der ersten Seite des scheibenförmigen Halteelements verbunden sein. In einigen Ausführungsformen kann eine umlaufende Fläche der umlaufenden Wand auf der ersten Seite des scheibenförmigen Halteelements im Wesentlichen vollständig mit dem zweiten Körper verbunden sein. In einigen Ausfiihrungsformen ist eine umlaufende Fläche der umlaufenden Wand auf der ersten Seite des scheibenförmigen Halteelements vollständig mit dem zweiten Körper verbunden. Auch kann eine umlaufende Fläche der umlaufenden Wand des zweiten Körpers im Wesentlichen vollständig mit dem scheibenförmigen Halteelement verbunden sein. In einigen Ausführungsformen ist eine umlaufende Fläche der umlaufenden Wand des zweiten Körpers vollständig mit dem scheibenförmigen Halteelement verbunden.

Die Seite des Flansches des ersten Körpers, mit dem die zweite Seite des Halteelements bzw. das in den zweiten Körper integrierte flanschartige, scheibenförmige Halteelement flächig verbunden werden kann, definiert eine Ebene. Diese Ebene liegt dementsprechend parallel zu einer Ebene, die vom Halteelement definiert wird. Die Ebenen des Flansches und des Halteelements stehen in einigen Ausführungsformen im Wesentlichen senkrecht zu einer Achse, die von der umlaufenden Wand des zweiten Körpers definiert wird, die den Innenraum, z.B. den Zylinderinnenraum des zweiten Körpers umgibt.

Im Verbindungssystems gemäß dem ersten Aspekt ist die Membran typischerweise zwischen der umlaufenden Wand des zweiten Körpers und dem scheibenförmigen Halteelement angeordnet. In der Regel ist die fluiddichte Membran mit der umlaufenden Wand des zweiten Körpers im Bereich eines umlaufenden Membrankontaktabschnitts in flächigem Kontakt. Die fluiddichte Membran kann auf der umlaufenden Wand des zweiten Körpers im Bereich eines umlaufenden Membrankontaktabschnitts aufliegen.

In einigen Ausführungsformen des Verbindungssystems gemäß dem ersten Aspekt greifen das scheibenförmige Halteelement und das Verbindungselement arretierbar ineinander. In einigen derartigen Ausführungsformen kann das Verbindungssystem, wenn es den ersten Körper aufweist, in einem offenen und in einem geschlossenen Zustand vorliegen. Im offenen Zustand ist das lösbare Verbindungselement an das scheibenförmige Halteelement gekoppelt, typischerweise beweglich gekoppelt. Das lösbare Verbindungselement kann beispielsweise gegenüber dem scheibenförmigen Halteelement verschiebbar oder verdrehbar sein. In einem solchen offenen Zustand ist der erste Körper an das scheibenförmige Halteelement und/oder die Membran gekoppelt. Der erste Körper kann mit dem scheibenförmigen Halteelement und/oder der Membran auch bereits lösbar verbunden sein. Im geschlossenen Zustand sind das scheibenförmige Halteelement und das Verbindungselement arretiert, so dass das Verbindungselement und das scheibenförmige Halteelement aneinander kraftschlüssig gekoppelt sind. In der Regel sind im geschlossenen Zustand das scheibenförmige Halteelement und das Verbindungselement miteinander lösbar verbunden. In diesem Zustand ist das Verbindungselement gegenüber dem scheibenförmigen Halteelement nicht mehr beweglich.

Wie bereits erläutert, weist in einigen Ausführungsformen das Verbindungssystem gemäß dem ersten Aspekt auch den ersten Körper auf. Die zweite Seite des Halteelements ist in einem solchen Fall typischerweise in flächigem Kontakt mit dem Flansch des ersten Körpers. Die zweite Seite des Halteelements kann beispielsweise auf dem Flansch des ersten Körpers aufliegen. Das lösbare Verbindungselement umgreift in solchen Ausführungsformen im arretierten Zustand zumindest teilweise den peripheren Außenrand des Halteelements und den Flansch des ersten Körpers.

In einigen Ausführungsformen des Verbindungssystems gemäß dem ersten Aspekt ist auf der umlaufenden Wand des zweiten Körpers eine umlaufende Wulst angeordnet. Die umlaufende Wulst kann beispielsweise im Membrankontaktabschnitt angeordnet sein. In einigen Ausführungsformen, in denen eine umlaufende Wulst vorhanden ist, kann der übrige Teil der umlaufenden Wand als ein um die umlaufende Wand umlaufender Rücksprung aufgefasst werden. Die fluiddichte Membran kann in solchen Ausführungsformen die umlaufende Wulst kontaktieren, beispielsweise mit der umlaufenden Wulst verbunden sein. Die fluiddichte Membran liegt in einigen Ausführungsformen auf der umlaufenden Wand des zweiten Körpers auf. In einigen Ausführungsformen ist die Membran zwischen der umlaufenden Wulst und dem Halteelement angeordnet. In einigen Ausführungsformen ist die umlaufende Wand des Halteelements mit dem Rücksprung des zweiten Körpers verbunden.

In einigen Ausführungsformen des Verbindungssystems gemäß dem ersten Aspekt weist der erste Körper ein erstes zylindrisches Ende mit einem Flansch auf. In einigen Ausführungsformen weist der zweite Körper ein zweites zylindrisches Ende mit einem Zylinder-Innenraum und einer umlaufenden Wand auf. Die umlaufende Wand kann in einigen Ausführungsformen ringförmig umlaufend sein. In einigen Ausführungsformen weisen der erste Körper ein erstes zylindrisches Ende und der zweite Körper ein zweites zylindrisches Ende mit einem Zylinder-Innenraum auf.

Wie bereits angegeben weist das Verbindungssystem ein scheibenförmiges Halteelement auf, das in einigen Ausführungsformen ein eigenständiges Bauteil ist. Das scheibenförmige Halteelement weist einen Durchlass wie beispielsweise eine Öffnung auf. Der Durchlass ist in einigen Ausführungsformen eine runde Öffnung, beispielsweise eine kreisrunde Öffnung oder eine ovale Öffnung. In einigen Ausführungsformen ist auf der ersten Seite des scheibenförmigen Halteelements eine ringförmige Wand angeordnet. Die ringförmige Wand kann in einigen Ausführungsformen zum Durchlass des scheibenförmigen Halteelements einheitlich beabstandet sein.

In Ausführungsformen, in denen auf der umlaufenden Wand des zweiten Körpers eine umlaufende Wulst angeordnet ist, kann diese beispielsweise eine Ringwulst sein. Eine umlaufende Wulst auf der umlaufenden Wand des zweiten Körpers definiert in einigen Ausführungsformen einen Rand des Innenraums des zweiten Körpers. Typischerweise weist eine umlaufende Wand einer solchen umlaufenden Wulst einen oder mehrere Abschnitte auf, die die Membran kontaktieren. In einigen Ausführungsformen kontaktiert die umlaufende Wand einer entsprechenden umlaufenden Wulst im Wesentlichen vollständig die Membran. In einigen Ausführungsformen kontaktiert die umlaufende Wand einer umlaufenden Wulst vollständig die Membran. In einigen Ausführungsformen ist die Membran zwischen einer solchen umlaufenden Wulst und dem scheibenförmigen Halteelement angeordnet.

In einigen Ausführungsformen, in denen das scheibenförmige Halteelement ein eigenständiges Bauteil definiert, ist eine umlaufende Fläche auf der zweiten Seite des Halteelements angeordnet, die den Flansch des ersten Körpers im Wesentlichen umlaufend kontaktiert. In einigen Ausführungsformen kontaktiert eine umlaufende Fläche auf der zweiten Seite des Halteelements vollständig den Flansch des ersten Körpers. In einigen Ausführungsformen, in denen das scheibenförmige Halteelement das zweite Ende des zweiten Körpers definiert, ist eine umlaufende Fläche auf dem Halteelement angeordnet, die den Flansch des ersten Körpers im Wesentlichen umlaufend kontaktiert. In einigen Ausführungsformen kontaktiert eine umlaufende Fläche auf dem Halteelement vollständig den Flansch des ersten Körpers.

Das lösbare Verbindungselement kontaktiert sowohl das scheibenförmige Halteelement als auch den Flansch des ersten Körpers. Dabei umgreift das lösbare Verbindungselement zumindest teilweise den peripheren Außenrand des scheibenförmigen Halteelements und den Flansch des ersten Körpers. In einigen Ausführungsformen weist das scheibenförmige Halteelement eine Fläche auf, die zumindest teilweise einen Abschnitt des Flansches des ersten Körpers kontaktiert. In einigen Ausfiihrungsformen weist das scheibenförmige Halteelement eine Fläche auf, die im Wesentlichen auf der gesamten Fläche einen Abschnitt des Flansches des ersten Körpers kontaktiert. Das scheibenförmige Halteelement kann beispielsweise eine Fläche aufweisen, die mit einer korrespondierenden Fläche des Flansches des ersten Körpers in Kontakt ist. Beispielsweise kann das scheibenförmige Halteelement eine Fläche aufweisen, die auf einer korrespondierenden Fläche des Flansches des ersten Körpers aufliegt. Eine derartige Fläche ist in einigen Ausführungsformen etwa einen Quadratmillimeter bis etwa 10 Quadratzentimeter groß. In einigen Ausführungsformen ist eine solche Fläche etwa 50 Quadratmillimeter bis etwa 20 Quadratzentimeter groß. In einigen Ausführungsformen ist eine solche Fläche etwa einen Quadratmillimeter bis etwa 50 Quadratzentimeter groß. In einigen Ausführungsformen kontaktiert eine solche Fläche des scheibenförmigen Halteelements die gesamte Fläche eines Abschnitt des Flansches des ersten Körpers vollständig.

Eine Fläche des scheibenförmigen Halteelements, die zumindest teilweise einen Abschnitt des Flansches des ersten Körpers kontaktiert, kann jede beliebige Form aufweisen. So kann eine derartige Fläche in einigen Ausführungsformen rund, z.B. kreisrund oder oval sein. Eine entsprechende Fläche kann auch ein beliebiges Vieleck, wie z.B. ein Dreieck oder ein Fünfeck sein. In einigen Ausfiihrungsformen ist eine Fläche des scheibenförmigen Halteelements, die zumindest teilweise einen Abschnitt des Flansches des ersten Körpers kontaktiert, von quadratischer oder rechteckiger Form. Eine solche Fläche kann auch sechs- oder höhereckig sein.

Der periphere Außenrand des scheibenförmigen Halteelements kann in einigen Ausführungsformen eine flache Wand definieren. In einigen Ausführungsformen kann der periphere Außenrand einen vollständig oder teilweise umlaufenden Vorsprung definieren. Ein solcher Vorsprung kann beispielsweise radial parallel zur Ebene des Flansches nach außen ragen. Ein solcher Vorsprung kann auch relativ zur Ebene des Flansches geneigt sein, beispielsweise in Richtung des zweiten Körpers.

In einigen Ausführungsformen definiert das zweite Ende des zweiten Körpers eine Abzweigung des fluidführenden zweiten Körpers. Das beispielsweise zylindrische Ende weist die umlaufende Wand auf. Ein entsprechendes zylindrisches Ende kann auch die umlaufende Wulst sowie einen wie oben beschriebenen Rücksprung aufweisen. In einigen Ausfiihrungsformen liegt die Membran zumindest teilweise am Rücksprung des zweiten Körpers an. Dazu kann die Membran eine Wulst aufweisen, wie im Europäischen Patent EP 2 252 202 beschrieben.

In einigen Ausführungsformen des Verbindungssystems des ersten Aspekts ist der zweite fluidführende Körper ein Gehäuse mit einem Mittel zur abgedichteten fluiden Verbindung mit einem Fluidsystem.

Das scheibenförmige Halteelement kann in Draufsicht auf die erste oder die zweite Seite jede beliebige Form haben. In einigen Ausführungsformen hat das scheibenförmige Halteelement, von der ersten oder der zweiten Seite aus gesehen, einen ovalen oder kreisförmigen Umriss. In einigen Ausführungsformen weist der Umriss des scheibenförmigen Halteelements, von der ersten oder der zweiten Seite aus gesehen, eine oder mehrere Ecken auf. Der Umriss kann beispielsweise dreieckig, rechteckig, quadratisch, fünf- oder sechseckig sein. In einigen Ausführungsformen definiert das scheibenförmige Halteelement einen Haltering.

Auch die umlaufende Wand des zweiten Körpers kann in Draufsicht jede beliebige Form haben. Die Dicke der umlaufenden Wand des zweiten Körpers kann beliebig variieren. In einer Ausführungsform ist die umlaufende Wand des zweiten Körpers von im Wesentlichen einheitlicher Dicke. In einer Ausführungsform ist die umlaufende Wand des zweiten Körpers von einheitlicher Dicke.

In einigen Ausfiihrungsformen des Verbindungssystems des ersten Aspekts ist der erste Körper ein Messwertaufnehmer und der zweite Körper eine mit einem Fluidsystem durchströmbar verbindbare Messkammer. In solchen Ausführungsformen ist die Membran typischerweise wesentlich nachgiebiger als die Wandung des Innenraums des zweiten Körpers und ermöglicht eine Übertragung von Druckkräften aus der Messkammer auf den Messwertaufnehmer. Die erste Seite und der Durchlass des scheibenförmigen Halteelements sind in solchen Ausführungsformen in der Regel mit der Membran verbunden. Das scheibenförmige Halteelement weist eine erste Seite, eine zweite Seite und einen peripheren Außenrand auf. Die erste Seite des scheibenförmigen Halteelements ist an die Membran und an die umlaufende Wand der Messkammer gekoppelt. Die zweite Seite des scheibenförmigen Halteelements ist dazu ausgelegt, an den Flansch des Messwertaufnehmers zu koppeln. Typischerweise ist die zweite Seite des scheibenförmigen Halteelements dazu ausgelegt, den Flansch des Messwertaufnehmers zu kontaktieren. Das scheibenförmige Halteelement erlaubt es, mit Hilfe eines lösbaren Verbindungselements kraftschlüssig am Messwertaufnehmer fixiert zu werden. In Ausführungsformen, in denen das Verbindungssystem den Messwertaufnehmer aufweist, ist die zweite Seite des scheibenförmigen Halteelements in der Regel an den Messwertaufnehmer gekoppelt. Das scheibenförmige Halteelement ist dann mit Hilfe eines lösbaren Verbindungselements kraftschlüssig am Messwertaufnehmer fixiert. In einigen Ausführungsformen liegt die erste Seite des scheibenförmigen Halteelements an der Membran und an der umlaufenden Wand der Messkammer an.

In einigen Ausführungsformen ist der erste Körper ein Messwertaufnehmer und der zweite Körper eine mit einem Fluidsystem durchströmbar verbindbare Messkammer, die eine umlaufende Wandung mit einer Aussparung aufweist. Die Aussparung weist einen Rand auf, der mit der flüssigkeitsdichten Membran in flächigem Kontakt ist. Auf einem solchen Rand kann beispielsweise eine flüssigkeitsdichte Membran aufliegen. Die Membran ist wesentlich nachgiebiger als die Wandungen der Messkammer. Die Membran ermöglicht in der Regel eine Übertragung von Druckkräften aus der Messkammer auf den Messwertaufnehmer. Die Membran ist zwischen dem Rand der Messkammer und einem scheibenförmigen Halteelement in Form eines Halterings eingelegt. Der Haltering weist eine erste Seite, eine zweite Seite und einen peripheren Außenrand auf. Die erste Seite ist an die Membran und an die Wandung der Messkammer gekoppelt. In einigen Ausführungsformen ist die erste Seite mit der Membran und der Wandung der Messkammer verbunden. Die zweite Seite des Halterings ist an den Messwertaufnehmer gekoppelt. Der Haltering ist mit Hilfe eines lösbaren Verbindungselements kraftschlüssig am Messwertaufnehmer fixiert. In einigen Ausführungsformen des Verbindungssystems des ersten Aspekts ist die zweite Seite des Halterings mit dem Messwertaufnehmer in flächigem Kontakt. In einigen Ausführungsformen liegt die zweite Seite des Halterings am Messwertaufnehmer an. In einigen Ausführungsformen umgreift das Verbindungselement den peripheren Außenrand des Halterings zumindest teilweise. In einigen Ausführungsformen weist der Messwertaufnehmer einen Flansch auf. Das Verbindungselement umgreift in solchen Ausfiihrungsformen zumindest teilweise den Flansch.

In einigen Ausführungsformen des Verbindungssystems des ersten Aspekts umgreift das lösbare Verbindungselement einen oder mehrere Abschnitte des peripheren Außenrands des scheibenförmigen Halteelements. In diesen Ausführungsformen umgreift das lösbare Verbindungselement in der Regel einen oder mehrere entsprechende angrenzende Abschnitte des Flansches des ersten Körpers. Beispielsweise kann der periphere Außenrand des scheibenförmigen Halteelements einen Umfang haben, von dem ein bestimmter Anteil wie etwa 75 % oder etwa 25 % durch einen oder mehrere Bereiche des lösbaren Verbindungselements umgriffen wird.

Der Durchlass des scheibenförmigen Halteelements stellt typischerweise eine Verbindung des Innenraums des zweiten Körpers mit der Membran her. Der Durchlass des scheibenförmigen Halteelements ist typischerweise eine Öffnung. In einigen Ausführungsformen kann der Durchlass aus mehreren Öffnungen bestehen. Eine entsprechende Öffnung kann jede beliebige Form und Größe aufweisen, solange die gewünschte Funktion der Anordnung der Körper und/oder der Membran mit der gewählten Form erreicht wird. Die maximale Größe des Durchlasses ist dann durch die Dimensionen des Innenraums des zweiten Körpers definiert. In einigen Ausführungsformen hat eine entsprechende Öffnung eine runde Form, beispielsweise elliptische oder Kreisform. In einigen Ausfiihrungsformen hat eine solche Öffnung eine dreieckige, rechteckige, quadratische, fünf-, sechseckige oder höhereckige Form. Dabei kann die Form eines solchen Durchlasses, z.B. einer Öffnung, unabhängig von der Form anderer Bestandteile des scheibenförmigen Halteelements oder der Form des Umrisses des scheibenförmigen Halteelements gewählt werden.

Die auf der ersten Seite des scheibenförmigen Halteelements angeordnete umlaufende Wand ist in typischen Ausführungsformen des Verbindungssystems des ersten Aspekts zum zentralen Durchlass im Wesentlich einheitlich beabstandet.

Wie bereits im Vorangehenden angegeben, definiert das scheibenförmige Halteelement eine Ebene. Diese Ebene ist typischerweise parallel zu einer Ebene, die von der Membran definiert wird. In einigen Ausführungsformen ist die zweite Seite des scheibenförmigen Halteelements, die für einen flächigen Kontakt mit dem Flansch des ersten Körpers ausgelegt ist bzw. die auf dem Flansch des ersten Körpers aufliegt, zumindest im Wesentlichen planar.

In typischen Ausführungsformen ist die Membran dauerhaft mit der umlaufenden Wand des zweiten Körpers und dem scheibenförmigen Halteelement verbunden. In einigen Ausführungsformen ist die Membran mit der umlaufenden Wand des zweiten Körpers und dem scheibenförmigen Halteelement verschweißt, verklebt oder versiegelt, oder an die umlaufende Wand angeklemmt. Die Membran kann auch durch Mehrkomponentenspritzguss und/oder Flüssig-Silikon, auch LSR (liquid silicon rubber) genannt, mit der umlaufenden Wand des zweiten Körpers und dem scheibenförmigen Halteelement verbunden sein. In einigen Ausführungsformen kann die Membran mit der umlaufenden Wand des zweiten Körpers und dem scheibenförmigen Halteelement durch eine Kombination mehrerer der genannten Mittel mit der umlaufenden Wand des zweiten Körpers verbunden sein.

In einigen Ausführungsformen ist das scheibenförmige Halteelement mit der umlaufenden Wand des zweiten Körpers verschweißt, verklebt oder versiegelt. Das scheibenförmige Halteelement kann auch an die umlaufende Wand des zweiten Körpers angeklemmt sein, durch Mehrkomponentenspritzguss und/oder Flüssig-Silikon der umlaufenden Wand des zweiten Körpers verbunden sein. Auch durch eine Kombination mehrerer der genannten Mittel kann das scheibenförmige Halteelement mit der umlaufenden Wand des zweiten Körpers verbunden sein.

Gemäß einem zweiten Aspekt wird ein Verbindungssystem zum lösbar kraftschlüssigen Koppeln des Innenraums eines ersten fluidführenden Körpers mit dem Innenraum eines zweiten fluidführenden Körpers bereitgestellt. Das Verbindungssystem kann ein System zum lösbaren abgedichteten Koppeln des Innenraums des ersten fluidführenden Körpers mit dem Innenraum eines zweiten fluidführenden Körpers sein. Der erste Körper weist ein erstes zylindrisches Ende mit einem Flansch auf. Der zweite Körper weist ein zweites zylindrisches Ende auf, das in ein flanschartiges, scheibenförmiges Halteelement mündet. Das scheibenförmige Halteelement weist eine zentrale Mündung des Innenraums des zweiten fluidführenden Körpers auf, beispielsweise einen Auslass. Das scheibenförmige Halteelement weist eine erste Seite, eine zweite Seite und einen peripheren Außenrand auf. Auf der ersten Seite des Halteelements ist ein ringförmiger Vorsprung angeordnet, der zur zentralen Mündung im Wesentlich einheitlich beabstandet ist. Die erste Seite des Halteelements ist für einen flächigen Kontakt mit dem Flansch des ersten Körpers ausgelegt. Die erste Seite des Halteelements liegt beispielsweise auf dem Flansch des ersten Körpers auf. Das Verbindungssystem weist ein Verbindungselement auf, das den peripheren Außenrand des Halteelements und den Flansch des ersten Körpers zumindest teilweise umgreift. Dadurch ist das scheibenförmige Halteelement mit Hilfe des lösbaren Verbindungselements flüssigkeitsdicht am ersten Körper fixiert.

In einigen Ausführungsformen weist das Verbindungssystem gemäß dem zweiten Aspekt weiterhin eine Membran auf. Die Membran ist zwischen dem Flansch des erste Körpers und der ersten Seite des Halteelements des zweiten Körpers angeordnet. Die Membran kann in einigen Ausfiihrungsformen lösbar zwischen ersten Körper und dem zweiten Körper angeordnet sein.

Das flanschartige, scheibenförmige Halteelement weist einen Durchlass bzw. einen zentralen Auslass auf. Der Durchlass des flanschartigen, scheibenförmigen Halteelements ist in der Regel eine Öffnung. In einigen Ausführungsformen kann der Durchlass aus mehreren Öffnungen bestehen. Eine entsprechende Öffnung kann jede beliebige Form und Größe aufweisen, solange die gewünschte Funktion der Verbindung der fluidführenden Körper erreicht wird. Die maximale Größe des Durchlasses ist dann durch die Dimensionen des Innenraums des zweiten Körpers definiert. In einigen Ausfiihrungsformen hat eine entsprechende Öffnung eine runde Form, beispielsweise elliptische oder Kreisform. In einigen Ausführungsformen hat eine solche Öffnung eine dreieckige, rechteckige, quadratische, fünf-, sechseckige oder höhereckige Form. Die Form des Durchlasses oder Auslasses eines solchen flanschartigen, scheibenförmigen Halteelements, z.B. einer Öffnung, kann unabhängig von der Form anderer Bestandteile des flanschartigen scheibenförmigen Halteelements oder der Form des Umrisses des flanschartigen scheibenförmigen Halteelements gewählt werden.

Das flanschartige, scheibenförmige Halteelement kann in Draufsicht auf die erste oder die zweite Seite jede beliebige Form haben. In einigen Ausführungsformen hat das flanschartige scheibenförmige Halteelement, von der ersten oder der zweiten Seite aus gesehen, einen ovalen oder kreisförmigen Umriss. In einigen Ausführungsformen weist der Umriss des scheibenförmigen Halteelements, von der ersten oder der zweiten Seite aus gesehen, eine oder mehrere Ecken auf. Der Umriss kann beispielsweise dreieckig, rechteckig, quadratisch, fünf- oder sechseckig sein. In einigen Ausfiihrungsformen definiert das scheibenförmige Halteelement einen Haltering.

Das flanschartige, scheibenförmige Halteelement weist in einigen Ausführungsformen eine Fläche auf, die zumindest teilweise einen Abschnitt des Flansches des ersten Körpers kontaktiert. In einigen Ausführungsformen weist das flanschartige, scheibenförmige Halteelement eine Fläche auf, die im Wesentlichen auf der gesamten Fläche einen Abschnitt des Flansches des ersten Körpers kontaktiert. Das flanschartige, scheibenförmige Halteelement kann beispielsweise eine Fläche aufweisen, die für einen flächigen Kontakt mit einer korrespondierenden Fläche des Flansches des ersten Körpers ausgelegt ist. Eine solche Fläche des flanschartigen, scheibenförmigen Halteelements kann beispielsweise eine Fläche aufweisen, die auf einer korrespondierenden Fläche des Flansches des ersten Körpers aufliegt. Eine derartige Fläche ist in einigen Ausführungsformen etwa einen Quadratmillimeter bis etwa 10 Quadratzentimeter groß. In einigen Ausfiihrungsformen ist eine solche Fläche etwa 50 Quadratmillimeter bis etwa 20 Quadratzentimeter groß. In einigen Ausfiihrungsformen ist eine solche Fläche etwa einen Quadratmillimeter bis etwa 50 Quadratzentimeter groß. In einigen Ausführungsformen kontaktiert eine solche Fläche des flanschartigen, scheibenförmigen Halteelements die gesamte Fläche eines Abschnitts des Flansches des ersten Körpers vollständig.

In einigen Ausführungsformen des Verbindungssystems gemäß dem zweiten Aspekt sind der erste fluidführende Körper und der zweite fluidführende Körper beweglich miteinander verbunden. Der erste und der zweite fluidführende Körper können verschwenkbar miteinander verbunden sein. So können beispielsweise der erste und der zweite fluidführende Körper über ein oder mehrere Scharniere miteinander verbunden sein.

In einigen Ausführungsformen des Verbindungssystems gemäß dem zweiten Aspekt liegt das flanschartige, scheibenförmige Halteelement lösbar auf dem Flansch des ersten Körpers auf. Das flanschartige, scheibenförmige Halteelement kann an den Flansch des ersten Körpers gekoppelt sein. In einigen Ausführungsformen ist das flanschartige, scheibenförmige Halteelement lösbar an den Flansch des ersten Körpers gekoppelt. In einigen Ausfiihrungsformen sind das flanschartige, scheibenförmige Halteelement und der Flansch des ersten Körpers beweglich aneinander gekoppelt.

Die erste Seite des flanschartigen, scheibenförmigen Halteelements definiert eine Ebene. Sofern eine Membran im Verbindungssystem vorhanden ist, ist die Ebene, die durch die erste Seite des Halteelements definiert ist, typischerweise parallel zu einer Ebene, die von der Membran definiert ist.

In einigen Ausführungsformen des Verbindungssystems gemäß dem ersten oder dem zweiten Aspekt ist das lösbare Verbindungselement mit dem Flansch des ersten Körpers verschraubbar. In einigen Ausführungsformen definiert das lösbare Verbindungselement eine Klemmvorrichtung.

In einigen Ausführungsformen des Verbindungssystems gemäß dem ersten oder dem zweiten Aspekt ist das scheibenförmige Halteelement mit dem Verbindungselement über eine Schnappverbindung verbunden. Typischerweise enthält dazu das Verbindungselement ein nachgebendes Element, das mit dem scheibenförmigen Halteelement interagiert. Das Verbindungselement kann auch ein entsprechendes Element aufweisen, das elastisch ist. In einigen ausführungsformen ist zumindest im Wesentlichen das gesamte Verbindungselement nachgebend.

In einigen Ausführungsformen des Verbindungssystems gemäß dem ersten oder dem zweiten Aspekt weist die für einen flächigen Kontakt mit dem Flansch des ersten Körpers ausgelegte Seite bzw. die auf dem Flansch des ersten Körpers aufliegende Seite des scheibenförmigen Halteelements eine periphere Führungskante auf. Die Führungskante ist dazu angepasst, die Außenseite des Flansches zu kontaktieren. In einigen Ausführungsformen ist die periphere Führungskante dem peripheren Außenrand des scheibenförmigen Halteelements benachbart. In einigen Ausführungsformen grenzt die periphere Führungskante an den peripheren Außenrand des scheibenförmigen Halteelements an.

In einigen Ausführungsformen weist die Seite des scheibenförmigen Halteelements, die für einen flächigen Kontakt mit dem Flansch des ersten Körpers ausgelegt ist bzw. dazu angepasst ist, den Flansch des ersten Körpers zu kontaktieren oder auf dem Flansch des ersten Körpers aufzuliegen, ein peripheres Hakenelement auf. Das periphere Hakenelement ist dazu angepasst, die Außenseite des Flansches des ersten Körpers zumindest teilweise zu umfassen. Das periphere Hakenelement kann in einigen Ausführungsformen dem peripheren Außenrand des scheibenförmigen Halteelements benachbart sein. In einigen Ausführungsformen grenzt das periphere Hakenelement an den peripheren Außenrand des scheibenförmigen Halteelements an. In einigen Ausführungsformen kann das periphere Hakenelement auf der peripheren Führungskante angeordnet sein. In einigen Ausfiihrungsformen kann das periphere Hakenelement in die periphere Führungskante integriert sein. Ein entsprechendes Hakenelement kann im Wesentlichen aus einem festen, unnachgiebigen Material bestehen, es kann auch vollständig aus einem festen, unnachgiebigen Material bestehen.

In einigen Ausführungsformen weist die Seite des scheibenförmigen Halteelements, die für einen flächigen Kontakt mit dem Flansch des ersten Körpers ausgelegt ist bzw. dazu angepasst ist, den Flansch des ersten Körpers zu kontaktieren oder auf diesem Flansch aufzuliegen, zwei periphere Hakenelemente auf. Die beiden peripheren Hakenelemente sind an einander gegenüberliegenden Enden des scheibenförmigen Halteelements angeordnet. Die beiden peripheren Hakenelemente sind beide auf der für einen flächigen Kontakt mit dem Flansch des ersten Körpers ausgelegten Seite bzw. auf der auf dem Flansch des ersten Körpers aufliegenden Seite des scheibenförmigen Halteelements angeordnet. Beide peripheren Hakenelemente können dem peripheren Außenrand des scheibenförmigen Halteelements benachbart sein. Beide peripheren Hakenelemente können an den peripheren Außenrand des scheibenförmigen Halteelements angrenzen.

In einigen Ausführungsformen weist das Verbindungselement einen Spannhebel auf, der dazu angepasst ist, den Flansch des ersten Körpers zumindest teilweise zu umgreifen. Das Verbindungselement kann auch im Wesentlichen aus einem Spannhebel bestehen, der dazu angepasst ist, den Flansch des ersten Körpers zumindest teilweise zu umgreifen. In einigen Ausführungsformen definiert ein entsprechender Spannhebel das Verbindungselement. Das Verbindungselement besteht in einer Ausfiihrungsform aus einem solchen Spannhebel.

Der Spannhebel kann in einigen Ausführungsformen einen peripheren Außenrand des scheibenförmigen Halteelements umgreifen. Typischerweise ist der Spannhebel in solchen Ausführungsformen auf der dem Flansch abgewandten Seite des scheibenförmigen Halteelements mit dem Halteelement verbunden. Der Spannhebel kann dann so ausgelegt sein, dass er in beweglicher Weise lösbar den peripheren Außenrand des scheibenförmigen Halteelements umgreifen kann. In einigen Ausführungsformen, in denen die für einen flächigen Kontakt mit dem Flansch des ersten Körpers ausgelegte Seite bzw. die auf dem Flansch des ersten Körpers aufliegende Seite des scheibenförmigen Halteelements eine periphere Führungskante aufweist, kann die periphere Führungskante Aussparungen enthalten, in die der Spannhebel greifen kann. In einigen Ausführungsformen, in denen die für einen flächigen Kontakt mit dem Flansch des ersten Körpers ausgelegte Seite des scheibenförmigen Halteelements eine periphere Führungskante aufweist, kann der Spannhebel dazu ausgelegt sein, die periphere Führungskante zu umgreifen.

In einigen Ausführungsformen weist das scheibenförmige Halteelement zwei oder mehr Spannhebel auf, die dazu angepasst sind, den Flansch des ersten Körpers zumindest teilweise zu umgreifen. Die Spannhebel können an Positionen an einem peripheren Rand des scheibenförmigen Halteelements axial gegeneinander versetzt angeordnet sein. Zwei Spannhebel können beispielsweise an einander gegenüberliegenden Enden des scheibenförmigen Halteelements angeordnet sein. Die zwei oder mehr Spannhebel sind dazu angepasst, den Flansch des ersten Körpers zumindest teilweise zu umgreifen. In einigen Ausführungsformen besteht das scheibenförmige Halteelement zumindest im Wesentlichen aus zwei Spannhebeln. In einigen Ausführungsformen weist das scheibenförmige Halteelement drei oder mehr Spannhebel auf.

In einigen Ausführungsformen weist das Verbindungselement einen Spannhebel und ein Hakenelement auf. In einigen Ausführungsformen weist das Verbindungselement mehrere Spannhebel und mehrere Hakenelemente auf.

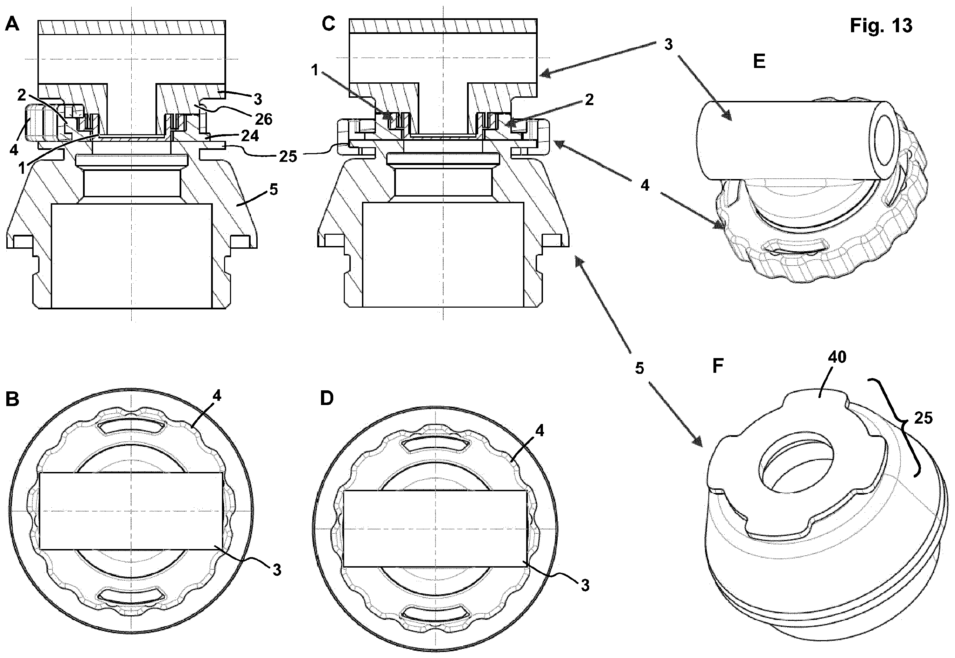

In einigen Ausführungsformen des Verbindungssystems gemäß dem ersten oder dem zweiten Aspekt ist das Verbindungselement durch eine umlaufende Verbindungsklammer, durch eine Tri-Clamp-Klemmverbindung, durch einen Verriegelungsring und/oder durch eine im Wesentlichen U-förmige Klammer definiert. Ein Verriegelungsring kann eine oder mehrere hinterschnittige Haltevorrichtungen aufweisen. Eine solche Ausführungsform ist beispielsweise in Fig. 13 gezeigt.

In einigen Ausführungsformen weist das Verbindungselement ein oder mehrere Spannhebel auf. Ein derartiger Spannhebel ist beweglich an das scheibenförmige Halteelement gekoppelt und umgreift den Flansch des ersten Körpers zumindest teilweise. Das Verbindungselement kann auch aus einem oder mehreren solchen Spannhebeln bestehen. Beispielsweise kann ein entsprechender Spannhebel um eine Achse beweglich sein, die der Position entspricht, an der der Spannhebel an das scheibenförmige Halteelement gekoppelt ist. Der Spannhebel kann mit dem scheibenförmigen Halteelement verbunden sein.

In einigen Ausführungsformen weist das Verbindungselement einen beweglich an das scheibenförmige Halteelement gekoppelten Arm auf. Das Verbindungselement kann auch aus einem beweglich an das scheibenförmige Halteelement gekoppelten Arm bestehen. Der Arm umgreift zumindest teilweise die Außenseite des Flansches des ersten Körpers und den peripheren Außenrand des scheibenförmigen Halteelements. Der Arm kann beispielsweise am peripheren Außenrand des scheibenförmigen Halteelements befestigt sein. Zum Arretieren des Arms kann beispielsweise am peripheren Außenrand des scheibenförmigen Halteelements ein formpassendes Aufnahme- oder Auflageelement wie eine Fuge oder eine niet- oder rahmenförmige Halterung vorgesehen sein, in die der Arm lösbar eingelegt ist. In einigen Ausführungsformen weist das Verbindungselement eine Mehrzahl an beweglich an das scheibenförmige Halteelement gekoppelter Arme auf. Es können beispielsweise zwei oder drei beweglich an das scheibenförmige Halteelement gekoppelte Arme vorhanden sein.

In einigen Ausführungsformen weist das Verbindungselement ein erstes Gewinde auf und das scheibenförmige Halteelement weist ein zweites Gewinde auf. Das erste und das zweite Gewinde passen ineinander und sind verschraubt. Das erste und das zweite Gewinde können eine weibliches und ein männliches Gewinde sein. Beispielsweise können das Verbindungselement und das scheibenförmige Halteelement in Form eines Luer-Lock verbunden sein.

In einigen Ausführungsformen weist das Verbindungselement eine umlaufende Wand auf, die den peripheren Außenrand des scheibenförmigen Halteelements umfasst. Die umlaufende Wand weist ein Paar axial einander gegenüberliegender Öffnungen auf und der periphere Außenrand des scheibenförmigen Halteelements weist ein Paar axial einander gegenüberliegender Vorsprünge auf. Das Paar axial einander gegenüberliegender Vorsprünge wird in dem Paar axial einander gegenüberliegender Öffnungen in Form eines Bajonettverschlusses gehalten.

In einigen Ausführungsformen weist das Verbindungselement eine aus wenigstens zwei beweglichen Elementen bestehende Klemme auf, die den peripheren Außenrand des scheibenförmigen Halteelements und den Flansch des ersten Körpers umgreift. Eine solche Klemme kann aus zwei aufklappbaren Elementen bestehen, die beispielsweise über ein Scharnier verbunden sind. Eine solche Klemme kann nach einem bekannten Mechanismus aufgebaut sein wie er unter den Namen BioClamp^{®}, ReadyClamp^{®}, Tri-clamp oder Tri-clover bekannt ist.

Das Verbindungselement kann durch eine umlaufende Verbindungsklammer, durch eine Tri-Clamp-Klemmverbindung, durch einen Verriegelungsring und/oder durch eine im Wesentlichen U-förmige Klammer definiert sein.

In einigen Ausführungsformen ist das Verbindungselement beweglich, z.B. drehbar oder ein/ausschiebbar mit dem ersten und oder dem zweiten Körper verbunden. So können der erste und/ oder der zweite Körper eine Schiene oder einen Steg aufweisen und das Verbindungselement kann einen Zapfen aufweisen. Ebenso können der erste und/oder der zweite Körper einen Zapfen aufweisen und das Verbindungselement kann eine Schiene oder einen Steg aufweisen. Der Steg bzw. die Schiene kann so dimensioniert und angeordnet sein, dass er/sie eine Führung und Sicherung des Zapfens ermöglicht.

In einigen Ausführungsformen des Verbindungssystems gemäß dem ersten oder dem zweiten Aspekt ist das Verbindungselement durch eine im Wesentlichen U-förmige Klammer mit einem oberen und einem unteren Element definiert. In diesen Ausfiihrungsformen ist das scheibenförmige Halteelement zwischen dem oberen und dem unteren Element der im Wesentlichen U-förmigen Klammer angeordnet. Die im Wesentlichen U-förmige Klammer kann ein Paar Schenkel aufweisen, die parallel zur Ebene des Flansches angeordnet sind. In einigen Ausfiihrungsformen kann die im Wesentlichen U-förmige Klammer durch ein oberes Paar Schenkel, ein unteres Paar Schenkel und eine Seitenwand definiert sein. Die Seitenwand kann jeweils einen oberen und einen unteren Schenkel verbinden.

In einigen Ausführungsformen des Verbindungssystems gemäß dem ersten oder dem zweiten Aspekt ist das Verbindungselement durch eine Kombination von mindestens einem festen Hakenelement und mindestens einem Spannhebel, durch eine Kombination von mindestens einem festen Hakenelements und mindestens einer Schnappverbindung, durch eine Kombination von zwei oder mehr Spannhebeln und/oder eine Kombination von einer im wesentlichen U-förmige Klammer und mindestens einem festen Hakenelement definiert.

In einigen Ausführungsformen des Verbindungssystems gemäß dem ersten oder dem zweiten Aspekt weist das Verbindungselement eine im Wesentlichen U-förmige Klammer auf und die zweite Seite des scheibenförmigen Halteelements weist eine Führungskante auf. Die Führungskante ist dazu angepasst, die Außenseite des Flansches des ersten Körpers zu kontaktieren.

In einigen Ausführungsformen des Verbindungssystems gemäß dem ersten oder dem zweiten Aspekt weist das Verbindungselement eine im Wesentlichen U-förmige Klammer mit einem oberen Paar Schenkel, einem unteren Paar Schenkel und einer jeden oberen und unteren Schenkel verbindenden Seitenwand auf. In einigen Ausführungsformen besteht das Verbindungselement im Wesentlichen aus einer im Wesentlichen U-förmigen Klammer mit einem oberen Paar Schenkel, einem unteren Paar Schenkel und einer jeden oberen und unteren Schenkel verbindenden Seitenwand. Die Seitenwand weist auf der Seite jedes Schenkels eine Öffnung auf. Das scheibenförmige Halteelement weist ein Paar Schenkel auf, die parallel zur Ebene des Flansches des ersten Körpers angeordnet sind. Das Halteelement ist zwischen dem oberen und dem unteren Element der im Wesentlichen U-förmigen Klammer angeordnet. Dadurch ermöglichen die Schenkel eine Halterung und Führung des Verbindungselements.

Das Paar oberer Schenkel der im Wesentlichen U-förmigen Klammer eines solchen Verbindungselements befindet sich relativ zum scheibenförmigen Halteelement auf der Seite, die dem zweiten Körper zugewandt ist. Das Paar unterer Schenkel befindet sich auf der Seite, die dem ersten Körper zugewandt ist. Das Paar oberer Schenkel weist in einigen Ausführungsformen einen Steg auf, der dazu angepasst ist, eine Führung und Sicherung eines Zapfens zu ermöglichen, der auf dem zweiten Körper angebracht ist. In einigen Ausfiihrungsformen weist das Paar unterer Schenkel einen Steg auf, der dazu angepasst ist, eine Führung und Sicherung eines Zapfens zu ermöglichen, der auf dem ersten Körper angebracht ist.

In einigen Ausführungsformen ist das Paar oberer Schenkel zumindest im Wesentlichen parallel zur Ebene des Flansches des ersten Körpers angeordnet. In einigen Ausführungsformen ist das Paar unterer Schenkel zumindest im Wesentlichen parallel zur Ebene des Flansches des zweiten Körpers angeordnet. Das Paar oberer und/oder unterer Schenkel definiert typischerweise eine Ebene, die im Wesentlichen parallel zur Ebene des Flansches des ersten Körpers angeordnet ist. In einigen Ausfiihrungsformen ist das Paar oberer und oder das Paar unterer Schenkel ein Paar zueinander im Wesentlichen symmetrischer Schenkel.

Auch das scheibenförmige Halteelement kann ein Paar Schenkel aufweisen. Auch das Paar Schenkel des scheibenförmigen Halteelements kann zumindest im Wesentlichen parallel zur Ebene des Flansches des ersten Körpers angeordnet sein. Ein solches Paar Schenkel des scheibenförmigen Halteelements kann eine Ebene definieren, die im Wesentlichen parallel zur Ebene des Flansches des ersten Körpers angeordnet ist. Das Paar Schenkel des scheibenförmigen Halteelements zeigt typischerweise parallel von einem peripheren Rand des Halteelements weg. Das Paar Schenkel des scheibenförmigen Halteelements kann zwischen das obere und das untere Element einer im Wesentlichen U-förmigen Klammer des Verbindungselements eingepasst sein, so dass sie eine Halterung und Führung der Klammer ermöglichen. Das Paar Schenkel des scheibenförmigen Halteelements kann zwischen ein Paar oberer und ein Paar unterer Schenkel einer im Wesentlichen U-förmigen Klammer des Verbindungselements eingepasst sein. In einigen Ausführungsformen ist das Paar Schenkel des scheibenförmigen Halteelements ein Paar zweier zueinander im Wesentlichen symmetrischer Schenkel.

In einigen Ausführungsformen ist das Ende mindestens eines der Schenkel des scheibenförmigen Halteelements als Haken geformt. Die Außenseite einer U-förmigen Klammer eines Verbindungselements kann in einer solchen Ausführungsform eine Aussparung oder eine Öffnung aufweist, die dazu angepasst ist, den Haken aufzunehmen.

Eine Montage eines hier offenbarten Verbindungssystems zum lösbar kraftschlüssigen Koppeln eines ersten Körpers an ein fluidführendes System beinhaltet es, das erste Ende des ersten Körpers mit dem zweiten Ende des zweiten Körpers in Kontakt zu bringen. Eine solche Montage beinhaltet auch, das lösbare Verbindungselement so anzuordnen, dass der Flansch des ersten Körpers und der peripheren Außenrand des scheibenförmigen Halteelements (als eigenständiges Bauteil) oder des flanschartigen, scheibenförmigen Halteelements, das das zweite Ende des zweiten Körpers definiert, vom lösbaren Verbindungselement zumindest teilweise umgriffen werden. Zur Montage kann es auch zählen, dass das lösbare Verbindungselement in einer Position, in der es den Flansch des ersten Körpers und den peripheren Außenrand des scheibenförmigen Halteelements bzw. des flanschartigen, scheibenförmigen Halteelements zumindest teilweise umgreift, in eine Position zu bringen, in der der erste Körper kraftschlüssig am zweiten Körper fixiert ist.

Gemäß einem dritten Aspekt wird ein Verfahren zum lösbar kraftschlüssigen Koppeln eines ersten Körpers an ein fluidführendes System über eine fluiddichte Membran bereitgestellt. Der erste Körper enthält ein erstes Ende mit einem Flansch. An das fluidführende System ist ein Innenraum eines zweiten Körpers gekoppelt. Der zweite Körper enthält ein zweites Ende mit einer umlaufenden Wand und einem Innenraum. Das zweite Ende enthält auch eine Öffnung des Innenraums, beispielsweise eine zentrale Öffnung. Ein um die Öffnung des Innenraums umlaufender Bereich der umlaufenden Wand definiert einen Membrankontaktabschnitt. Die Öffnung des Innenraums ist von der umlaufenden Wand umgeben. Der umlaufende Bereich, der den Membrankontaktabschnitt definiert, liegt in der Regel in einer Ebene, die von der Öffnung des Innenraums definiert wird. Typischerweise weist der umlaufende Bereich, der den Membrankontaktabschnitt definiert, in die Richtung, in die auch die Öffnung des Innenraums weist. Der Membrankontaktabschnitt ist in Kontakt mit der Membran. In typischen Ausführungsformen des Verfahrens gemäß dem dritten Aspekt ist der Membrankontaktabschnitt mit der Membran verbunden.

Das zweite Ende des zweiten Körpers ist darüber hinaus mit einem scheibenförmigen Halteelement in Kontakt. In typischen Ausführungsformen ist das zweite Ende des zweiten Körpers mit diesem scheibenförmigen Halteelement verbunden. Das scheibenförmige Halteelement enthält einen Durchlass, eine erste Seite, eine zweite Seite und einen peripheren Außenrand. Dabei ist die erste Seite des Halteelements mit dem zweiten Ende des zweiten Körpers in Kontakt. Die erste Seite und/oder der Durchlass des Halteelements ist/sind darüber hinaus an die Membran gekoppelt. In typischen Ausführungsformen ist/sind die erste Seite und/oder der Durchlass des Halteelements mit der Membran verbunden. Die zweite Seite des Halteelements ist für einen flächigen Kontakt mit dem Flansch des ersten Körpers ausgelegt.

Im Verfahren gemäß dem dritten Aspekt werden das am zweiten Ende des zweiten Körpers befindliche scheibenförmige Halteelement und das erste Ende des ersten Körpers in Kontakt gebracht. Dabei werden das scheibenförmige Halteelement und das erste Ende des ersten Körpers so ausgerichtet, dass die zweite Seite des scheibenförmigen Halteelements auf dem Flansch des ersten Körpers aufliegt. Anschließend wird ein lösbares Verbindungselement so angeordnet, dass es den Flansch des ersten Körpers und den peripheren Außenrand des scheibenförmigen Halteelements zumindest teilweise umgreift. Dadurch wird das scheibenförmige Halteelement mit Hilfe des Verbindungselements kraftschlüssig am ersten Körper reversibel fixiert.

In einigen Ausführungsformen des Verfahrens gemäß dem dritten Aspekt werden zuerst das scheibenförmige Halteelement und das erste Ende des ersten Körpers so angeordnet, dass die zweite Seite des scheibenförmigen Halteelements auf dem Flansch des ersten Körpers aufliegt. Anschließend wird das lösbares Verbindungselement in eine Position gebracht, in der es sich in einer Ebene mit dem Flansch des ersten Körpers und dem peripheren Außenrand des scheibenförmigen Halteelements befindet. Von dieser Position lässt sich das lösbares Verbindungselement in der gleichen Eben in eine Position bringen, in der es den Flansch des ersten Körpers und den peripheren Außenrand des scheibenförmigen Halteelements zumindest teilweise umgreifen kann. In dieser Position sind der erste Körper und das scheibenförmigen Halteelement und somit der erste Körper und der zweite Körper lösbar in Kontakt. Danach wird das lösbares Verbindungselement in die Position gebracht, in der es den Flansch des ersten Körpers und den peripheren Außenrand des scheibenförmigen Halteelements zumindest teilweise umgreift. Dadurch wird das scheibenförmige Halteelement mit Hilfe des Verbindungselements kraftschlüssig am ersten Körper reversibel fixiert.

Der Innenraum des zweiten Körpers weist eine Wandung auf. Diese Wandung ist in der Regel die umlaufende Wand des zweiten Ende des zweite Körpers. In einigen Ausführungsformen des Verfahrens gemäß dem dritten Aspekt ist die Membran wesentlich nachgiebiger als diese Wandung des Innenraums des zweiten Körpers.

Gemäß einem vierten Aspekt wird ein Verfahren zum lösbar kraftschlüssigen Koppeln des Innenraums eines ersten fluidführenden Körpers mit dem Innenraum eines zweiten fluidführenden Körpers bereitgestellt. Der erste fluidführende Körper enthält ein erstes Ende mit einem Flansch. Im ersten Ende des ersten fluidführenden Körpers befindet sich des Weiteren ein Auslass des Innenraums des ersten Körpers. Der zweite fluidführende Körper enthält ein zweites Ende. Dieses zweite Ende definiert ein flanschartiges, scheibenförmiges Halteelement. Das flanschartige, scheibenförmige Halteelement enthält einen peripheren Außenrand und eine zentrale Öffnung. Diese zentrale Öffnung ist eine Öffnung des Innenraums des zweiten fluidführenden Körpers. Das Halteelement enthält weiterhin einen oder mehrere Arme. Zum Verbindungssystem zählt auch ein Verbindungselement, das dazu ausgelegt ist, den peripheren Außenrand des flanschartigen, scheibenförmigen Halteelements und den Flansch des ersten Körpers zumindest teilweise zu umgreifen. Auf diese Weise ist das Halteelement mit Hilfe des lösbaren Verbindungselements kraftschlüssig am ersten Körper fixierbar. Im Verfahren gemäß dem vierten Aspekt wird das flanschartige, scheibenförmige Halteelement verbindbar so an den Flansch des ersten Körpers gekoppelt, dass das Halteelement auf dem Flansch des ersten Körpers aufliegt. Anschließend wird das Verbindungselement so auf das flanschartige, scheibenförmige Halteelement und den Flansch des ersten Körpers platziert, dass das Verbindungselement den peripheren Außenrand des flanschartigen, scheibenförmigen Halteelements und den Flansch des ersten Körpers zumindest teilweise umgreift. Dadurch wird das flanschartige, scheibenförmige Halteelement mit Hilfe des Verbindungselements kraftschlüssig am ersten Körper reversibel fixiert.

In typischen Ausführungsformen des Verfahrens gemäß dem vierten Aspekt enthält das Verbindungselement eine im Wesentlichen U-förmige Klammer mit zwei Schenkeln. Die im Wesentlichen U-förmige Klammer enthält eine oder mehrere Aussparungen, in die der Arm bzw. die Arme des Halteelements einschiebbar ist/sind.

In einigen Ausführungsformen beinhaltet das Verfahren gemäß dem vierten Aspekt Bereitstellen des ersten fluidführenden Körpers und des zweiten fluidführenden Körpers.

In einigen Ausführungsformen ist das Verfahren gemäß dem vierten Aspekt ein Verfahren zum sterilen bzw. aseptischen Koppeln des Innenraums eines ersten fluidführenden Körpers mit dem Innenraum eines zweiten fluidführenden Körpers.

Auch ein hier offenbartes Verbindungssystems zum lösbar kraftschlüssigen Koppeln des Innenraums eines ersten fluidführenden Körpers mit dem Innenraum eines zweiten fluidführenden Körpers kann in einem Zustand vorliegen, in dem das Halteelement noch nicht mit Hilfe des lösbaren Verbindungselements kraftschlüssig am ersten Körper fixiert ist. In diesem Zustand ist beispielsweise der Arm des Halteelements nicht in eine Aussparung einer im Wesentlichen U-förmigen Klammer des Verbindungselements eingeschoben. Eine Montage dieses Verbindungssystems beinhaltet es in der Regel, das einen Flansch aufweisende erste Ende des ersten Körpers mit dem zweiten Ende des zweiten Körpers, das ein flanschartiges, scheibenförmiges Halteelement definiert, in Kontakt zu bringen. Die Montage beinhaltet auch, das lösbare Verbindungselement so anzuordnen, dass der Flansch des ersten Körpers und der periphere Außenrand des Halteelements vom lösbaren Verbindungselement zumindest teilweise umgriffen werden. Dabei wird beispielsweise der Arm des Halteelements in die Aussparung einer im Wesentlichen U-förmigen Klammer des Verbindungselements eingeschoben. Zur Montage kann es auch zählen, dass das lösbare Verbindungselement in einer Position, in der es den Flansch des ersten Körpers und den peripheren Außenrand des Halteelements zumindest teilweise umgreift, in eine Position zu bringen, in der der erste Körper kraftschlüssig am zweiten Körper fixiert ist. Beispielsweise kann der Arm des Halteelements in der Aussparung der Klammer arretiert werden.

Die im Vorangehenden beschriebene Zusammenfassung ist nicht einschränkend und weitere Merkmale und Vorteile der hier beschriebenen Vorrichtung sollten aus der folgenden ausführlichen Beschreibung, den Abbildungen und den Patentansprüchen ersichtlich sein.

### KURZE BESCHREIBUNG DER ZEICHNUNGEN

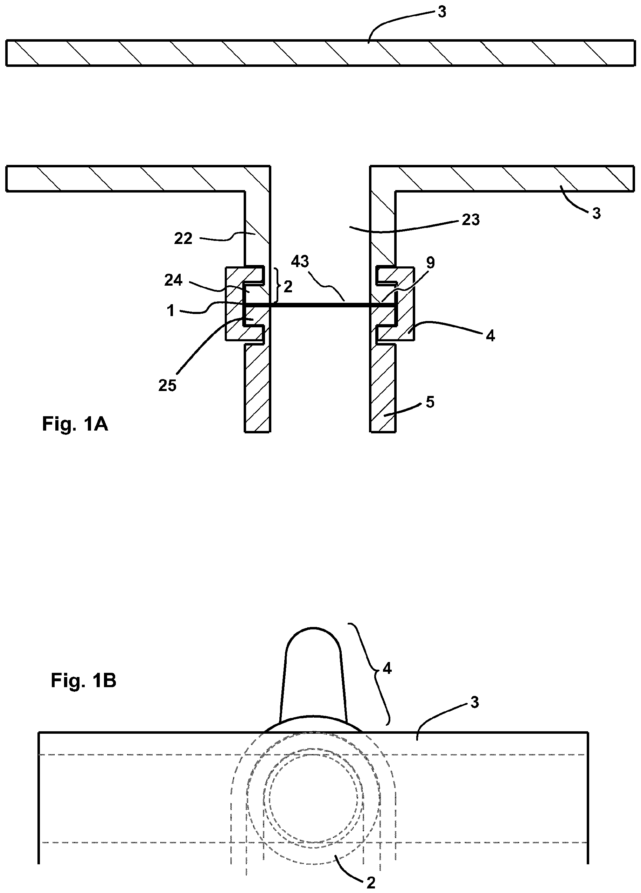

**Figur 1** zeigt ein hier offenbartes Verbindungssystem in allgemeiner schematisierter Darstellung. **Fig. 1A** zeigt das Verbindungssystem im Querschnitt, während **Fig. 1B** das Verbindungssystem in Draufsicht zeigt. Das Verbindungssystem weist eine Membran (1) und einen zweiten Körper (3) mit einem Innenraum auf. Es ist verbunden mit einem ersten Körper (5), der einen Flansch (25) aufweist. Der zweite Körper (3), der an ein fluidführendes System gekoppelt ist, enthält ein zweites Ende mit einem Innenraum (23) und einer umlaufenden Wand (22). Eine Öffnung (43) ist von der Membran (1) bedeckt. Die umlaufende Wand (22) weist einen um die Öffnung (43) umlaufenden Membrankontaktabschnitt (9) auf, der mit der Membran (1) verbunden ist. Das zweite Ende des zweiten Körpers (3) definiert ein flanschartiges, scheibenförmiges Halteelement (2) mit einem peripheren Außenrand (24). Ein lösbares Verbindungselement (4) umgreift den peripheren Außenrand (24) des Halteelements (2) und den Flansch (25) des ersten Körpers (5). Wie **Fig. 1B** zu entnehmen ist, umgreift das Verbindungselement (4) das Halteelement (2) und den Flansch (25) des ersten Körpers (5) nicht vollständig umlaufend um deren gesamten Umfang, sondern nur um einen Abschnitt, der etwa die Hälfte des gesamten Umfangs definiert. Die Dimensionen der einzelnen Bauteile und Merkmale sind nicht maßstabsgerecht, sondern dem Zweck der Anschaulichkeit angepasst.

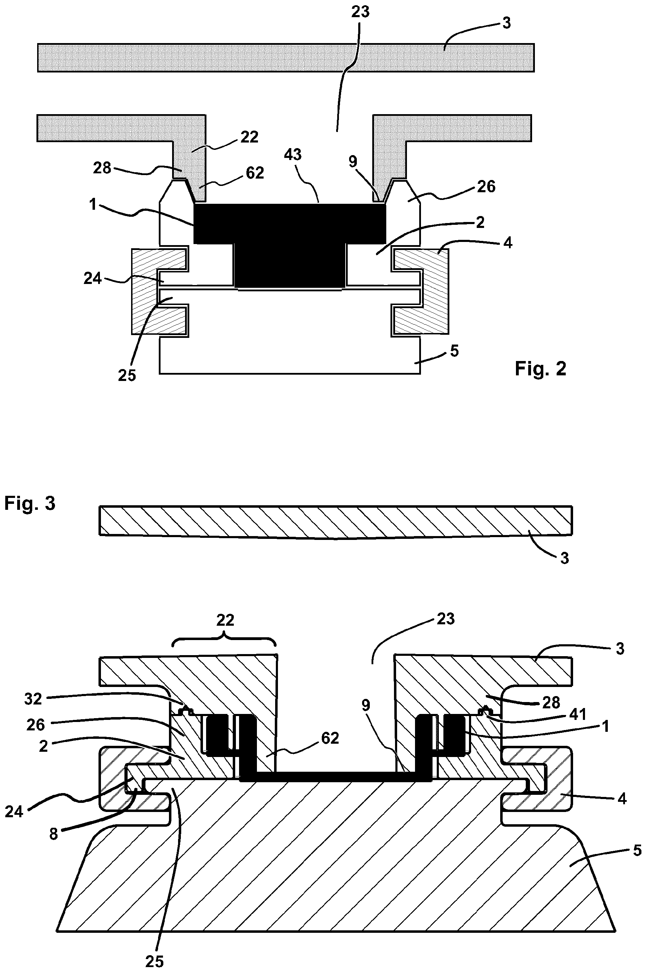

**Figur 2** zeigt ein weiteres Verbindungssystem gemäß einer hier offenbarten Ausführungsform. Es weist einen ersten Körper (5) mit einem Flansch (25) auf. Das Verbindungssystem weist einen zweiten Körper (3) in Form eines Gehäuses auf. Ein Ende des zweiten Körpers (3) mit einem Innenraum (23) weist eine umlaufende Wand (22) mit einer umlaufenden Wulst (62) und einem Rücksprung (28) auf. Die umlaufende Wulst (62) umgibt eine Öffnung (43) und weist einen umlaufenden Membrankontaktabschnitt (9) auf. Die Öffnung (43) des zweiten Körpers (3) wird von der Membran (1) bedeckt. Zwischen dem ersten (5) und dem zweiten Körper (3) sind eine Membran (1) und ein scheibenförmiges Halteelement (2) mit einer ringförmigen Wand (26) angeordnet. Die Wand (26) des scheibenförmigen Halteelements (2) kontaktiert den Rücksprung (28) des zweiten Körpers (3). Ein Verbindungselement (4) umgreift eine periphere Außenwand (24) des Halteelements (2) und den Flansch (25) des ersten Körpers (5). Die Dimensionen der einzelnen Bauteile und Merkmale wurden der Veranschaulichung halber für die Querschnittsansicht angepasst.
**Figur 3** zeigt eine Ausführungsform des Verbindungssystems, in dem der erste Körper (5) ein Messwertaufnehmer ist und der zweite Körper (3) eine Messkammer ist. Ein zylindrisches Ende des zweiten Körpers (3) mit einem Zylinderinnenraum (23) weist eine umlaufende Wand (22) mit einer umlaufenden Wulst (62) auf. Die umlaufende Wulst (62) weist einen umlaufenden Membrankontaktabschnitt (9) auf. Auf einer ersten Seite des scheibenförmigen Halteelements (2), die dem zweiten Körper (3) zugewandt ist, befindet sich eine Wand (26), die einen Bereich (28) der umlaufenden Wand (22) kontaktiert, der dem Zylinderinnenraum (23) abgewandt ist. Weiterhin weist dieser Bereich (28), der auch als Rücksprung aufgefasst werden kann, eine Nut (32) auf. Eine Wulst (41) der Wand (26) des scheibenförmigen Halteelements (2) liegt in der Nut (32). An der peripheren Außenwand (24) des scheibenförmigen Halteelements (2) ist eine Führungskante (8) vorgesehen, die den Flansch (25) von außen kontaktiert. Das Verbindungselement (4) umgreift die Führungskante (8).

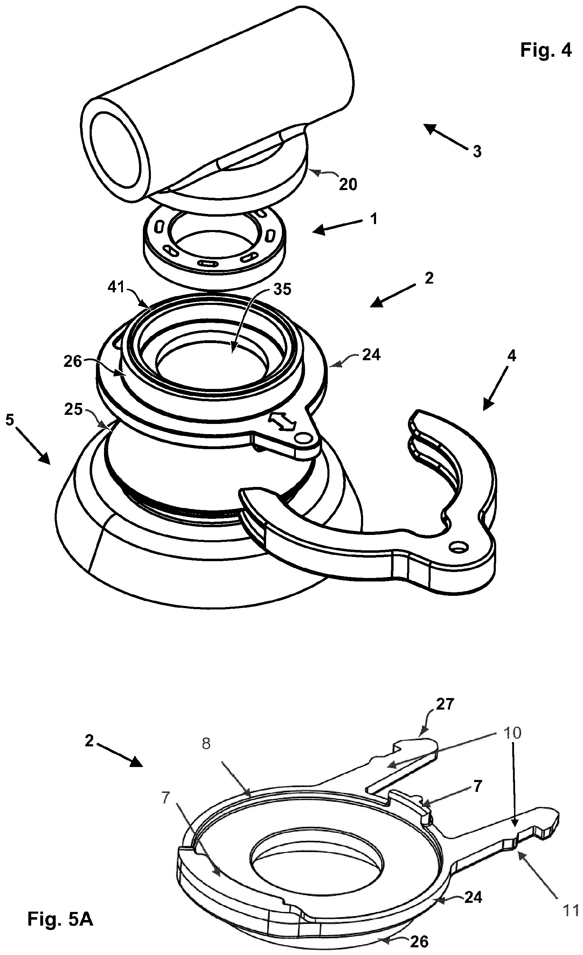

**Figur 4** zeigt in Explosionsansicht eine Ausführungsform des Verbindungssystems mit einem zweiten Körper (3), dessen zylindrisches Ende von schräg oben als Wandabschitt (20) erkennbar ist. Das Verbindungselement (4) ist als halbring-förmige Klammer gestaltet. Die Figur zeigt des Weiteren, dass die Form der Membran so gestaltet ist, dass sie an der umlaufenden Wand des Durchlasses (35) des scheibenförmigen Halteelements (2) anliegt. Eine Wulst (41) der Wand (26) des scheibenförmigen Halteelements (2) trägt zum Fixieren und Abdichten der Membran (1) bei. In diesem Bereich der Wand (26) kann ein dauerhaftes Verbinden, also ein Fügen mit dem zweiten Körper und/oder der Membran vorgenommen sein, beispielsweise durch Kleben oder Verschweißen. Der Flansch (25) des ersten Körpers (5), die Membran (1) und das scheibenförmige Halteelement (2) definieren Ebenen, die parallel zueinander liegen.
**Figur 5A** zeigt eine Ausführungsform des scheibenförmigen Halteelements (2) von schräg unten, also aus Richtung des ersten Körpers (5). Das scheibenförmige Halteelement ist als Haltering mit zwei Armen (10) ausgebildet. Eine Führungskante (8) setzt sich in die beiden Arme (10) fort. Die Arme (10) weisen Vorsprünge (11) und Haken (27) auf. Die Führungskante (8) setzt sich weiterhin in zwei gegenüberliegende Hakenelemente (7) fort.

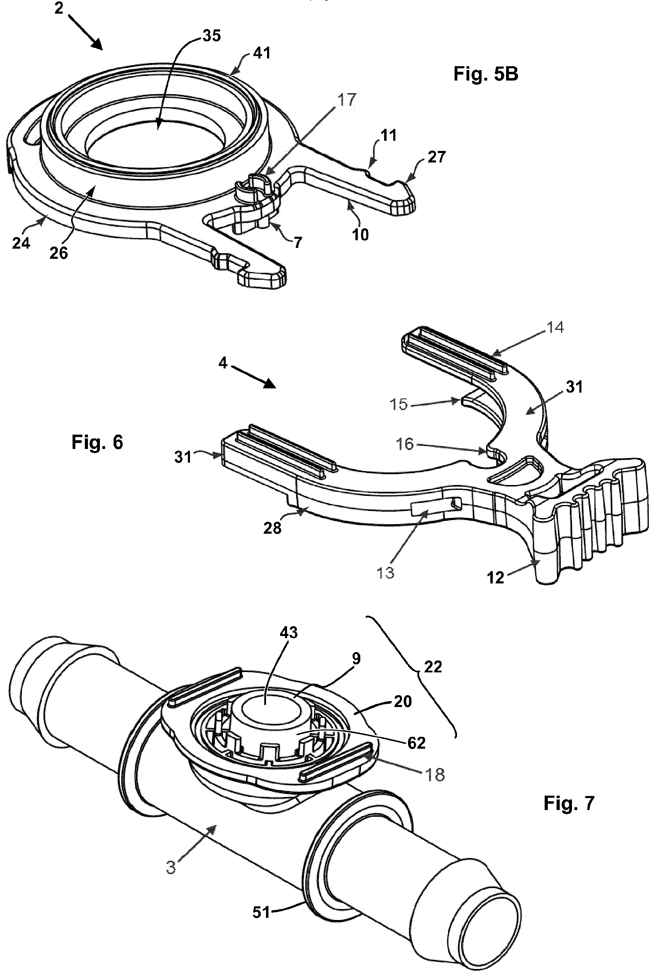

**Figur 5B** zeigt die Ausführungsform des in Figur 5A gezeigten scheibenförmigen Halteelements (2) mit einem Durchlass (35) von schräg oben, also aus Richtung des zweiten Körpers (3). Der periphere Außenrand (24) des Halteelements definiert eine Wand. Aus der peripheren Außenwand (24) entspringen die beiden Arme (10) mit jeweils einem Vorsprung (11) und einem Haken (27). Auf der dem zweiten Körper (3) zugewandten Seite befindet sich eine umlaufende Wand (26) mit einer Wulst (41). Im Bereich zwischen den beiden Armen (10) ist eines der auf der dem zweiten Körper (3) abgewandten Seite angeordneten Hakenelemente (7) erkennbar. Gegenüber auf der dem zweiten Körper (3) zugewandten Seite ist eine Verklemmung (17) angeordnet, die in die Aussparung (16) des Verbindungselements (4) einschiebbar ist.
**Figur 6** zeigt eine Ausführungsform des Verbindungselements (4) als U-förmige Klammer mit einem Griffelement (12) und zwei Schenkeln (31) von schräg oben, also aus Richtung des zweiten Körpers (3). Die Schenkel weisen eine Seitenwand (28) mit einer Aussparung (13) auf. Auf der dem zweiten Körper zugewandten Seite befinden sich auf den Schenkeln Stege (14) und im Bereich des Verbindungselements, in dem sich die Schenkel (31) treffen, ist eine Aussparung (16) vorgesehen. Eine Einlaufkante (15) liegt auf der den Stegen (14) gegenüberliegenden Seite, d.h. der dem ersten Körper (5) zugewandten Seite, der Schenkel (31).
**Figur 7** zeigt eine Ausführungsform des zweiten Körpers (3) als Messkammer mit zwei Ringbunden (51) von schräg unten, also aus Richtung des scheibenförmigen Halteelements (2). Erkennbar sind somit der Wandabschnitt (20) mit der Öffnung (43) des zweiten Körpers (3) und dem umlaufenden Membrankontaktabschnitt (9) auf der umlaufenden Wulst (62). Führungsstege (18) sind auf der Seite der umlaufenden Wand (22) angeordnet, an der das scheibenförmige Halteelement (2) anliegt.

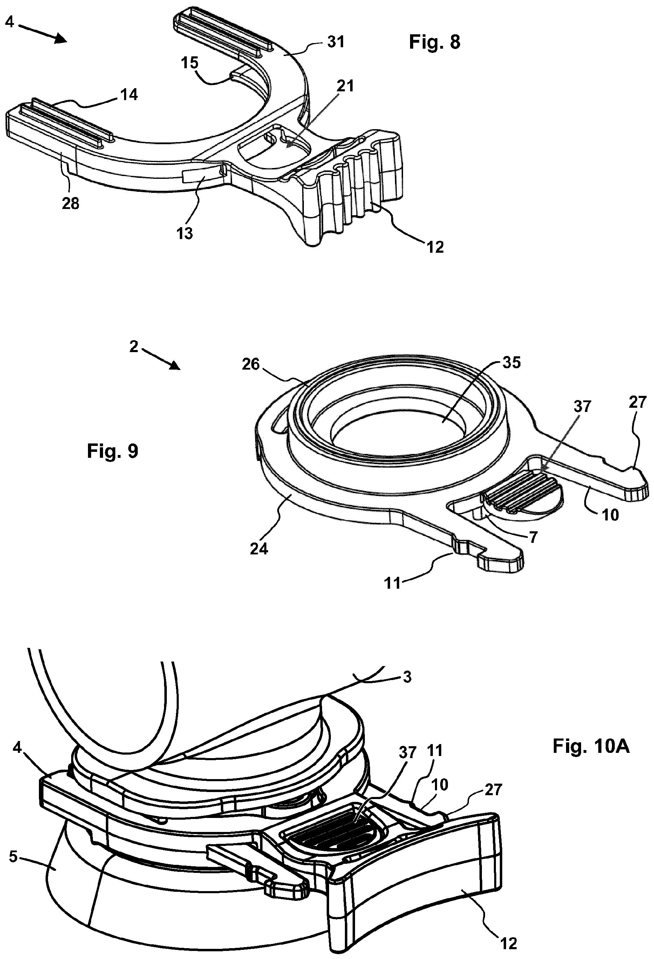

**Figur 8** zeigt eine weitere Ausführungsform des Verbindungselements (4) als U-förmige Klammer mit einem Griffelement (16) und zwei Schenkeln (31). Die gewählte Perspektive ist erneut von schräg oben, also aus Richtung des zweiten Körpers (3). Die Schenkel weisen eine Seitenwand (28) mit einer Aussparung (13) in Form einer Öffnung auf. Der Bereich des Verbindungselements, in dem sich die Schenkel (31) treffen, ist durchgehend, so dass in dieser Ausführungsform keine Aussparung vorgesehen ist. In dieser Ausführungsform befindet sich im Bereich des Griffelements (12) ein Durchlass durch eine Ebene, die parallel zu der vom Flansch des ersten Körpers (5), vgl. z.B. Fig. 10A, definierten Ebene liegt. Das Verbindungselement hat in diesem Fall eine durchgehende Öffnung (21) durch diese Ebene hindurch.
**Figur 9** zeigt eine weitere Ausführungsform des scheibenförmigen Halteelements (2) mit zwei Armen (10), von schräg oben, also aus Richtung des zweiten Körpers (3). Auch hier definiert der periphere Außenrand (24) des Halteelements eine Wand. Im Bereich zwischen den beiden Armen (10) befindet sich ein Einschübling (37), der nachgebend aus der Außenwand (24) entspringt. Die Form des Einschüblings (37) ist an die Form der durchgehenden Öffnung (21) des in Fig. 8 gezeigten Verbindungselements (4) angepasst.
**Figur 10A** zeigt einen Ausschnitt eines Verbindungssystems, in dem eine der in Fig. 8 ähnelnde Ausführungsform des Verbindungselements (4) mit der in Fig. 9 gezeigten Ausführungsform des scheibenförmigen Halteelements (2) zwischen dem ersten Körper (5) und dem zweiten Körper (3) angeordnet ist. Das Verbindungselement (4) und damit das gesamte Verbindungssystem befindet sich in einer geschlossenen Stellung, in der die Arme (10) mit Haken (27) des scheibenförmigen Halteelements (2) so weit wie möglich aus den Aussparungen des Verbindungselements (4) herausragen. Dadurch umgreift das Verbindungselements (4) den durch das Verbindungselements (4) verdeckten Flansch des ersten Körpers (5). Der Einschübling (37) des scheibenförmigen Halteelements (2) ist in einer Aussparung des Verbindungselements (4) arretiert.

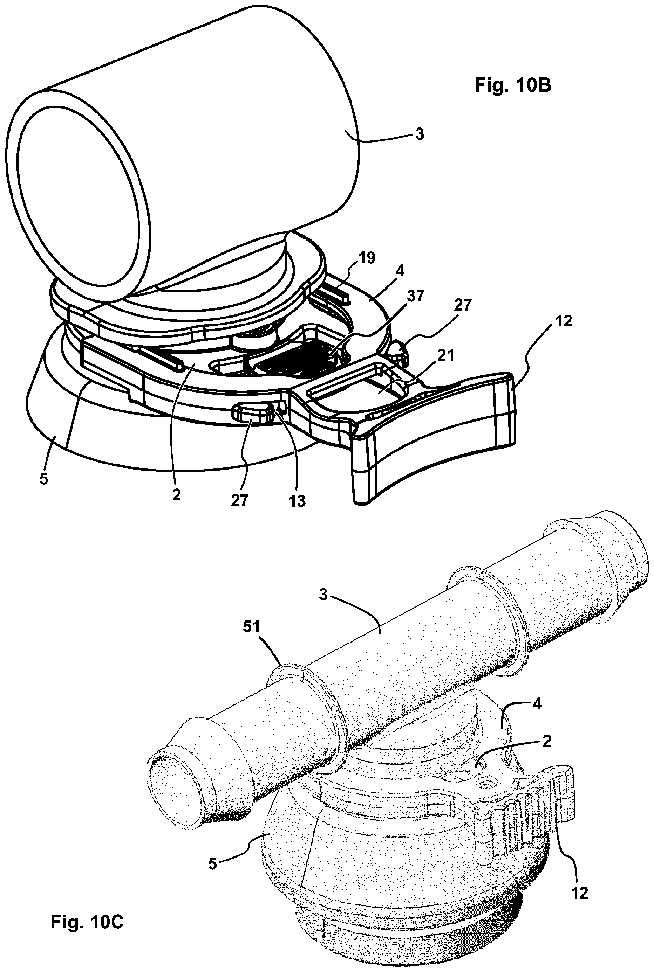

**Figur 10B** zeigt das in Fig. 10A gezeigte Verbindungssystem in offener Stellung. Relativ zum Betrachter ist das Verbindungselement (4) nach vorne verschoben, sodass der Einschübling (37) des scheibenförmigen Halteelements (2) und die Aussparung (21) des Verbindungselements (4) gegeneinander versetzt angeordnet sind. Das Verbindungselement (4) weist u.a. im Gegensatz zur Ausführungsform von Fig. 8 nur einen Steg (19) anstelle von zwei Stegen (14) auf.
**Figur 10C** zeigt eine weitere Ausführungsform des Verbindungssystems, in dem das scheibenförmige Halteelement (2) keine Arme aufweist und das Verbindungselement (4) keine Stege (14) aufweist, vgl. z.B. Fig. 4. Der zweite Körper (3) besitzt zwei Ringbunde (51). Das Verbindungssystem befindet sich in geschlossener Stellung.

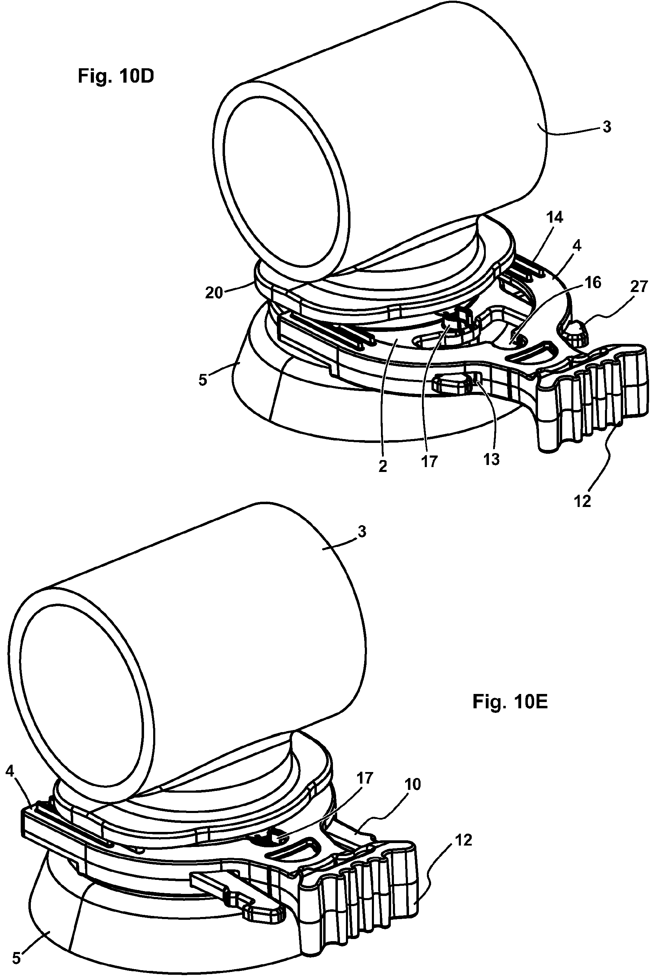

**Figur 10D** zeigt eine Ausführungsform eines Verbindungssystems, in dem die in Fig. 6 gezeigte Ausführungsform des scheibenförmigen Halteelements (2) zwischen dem ersten Körper (5) und dem zweiten Körper (3) angeordnet ist. Die die in Fig. 5A und 5B gezeigte Ausführungsform des Verbindungselements (4) ist bereits teilweise so zwischen dem ersten Körper (5) und dem zweiten Körper (3) platziert, dass durch Druck auf das Griffelement (12) eine Arretierung erfolgen kann. Die Stege (14) auf dem Verbindungselement (4) definieren eine bidirektionale Führung am Wandabschnitt (20) des zweiten Körpers (3). Das Verbindungselement (4) und damit das gesamte Verbindungssystem befindet sich somit in einer offenen Stellung, in der im Wesentlichen kein Umgreifen des peripheren Außenrands des scheibenförmigen Halteelements (2) und des Flanschs (25) des ersten Körpers (5) vorliegt. Die Verklemmung (17) des scheibenförmigen Halteelements (2) und die Aussparung (16) des Verbindungselements (4) sind gegeneinander versetzt angeordnet. Die Stege (14) auf dem Verbindungselement (4) erfahren eine Führung durch in Fig. 7 gezeigte Zapfen. Von den Schenkeln des scheibenförmigen Halteelements (2) sind Haken (27) erkennbar, die in Aussparungen (13) des Verbindungselements (4) liegen. Durch Druck auf das Griffelement (12) kann ein Verschieben des Verbindungselements (4) erfolgen, bei der sich die Schenkel des scheibenförmigen Halteelements (2) durch die jeweilige Aussparung (13) bewegen.
**Figur 10E** zeigt die in Fig. 10D gezeigte Ausführungsform des Verbindungssystems in geschlossener Stellung. Die Verklemmung (17) des scheibenförmigen Halteelements (2) ist in die Aussparung (16) des Verbindungselements (4) eingeschoben und arretiert. Das Verbindungselement (4) umgreift eine in dieser Perspektive verdeckte periphere Außenwand des Halteelements (2) und den ebenfalls verdeckten Flansch des ersten Körpers (5).

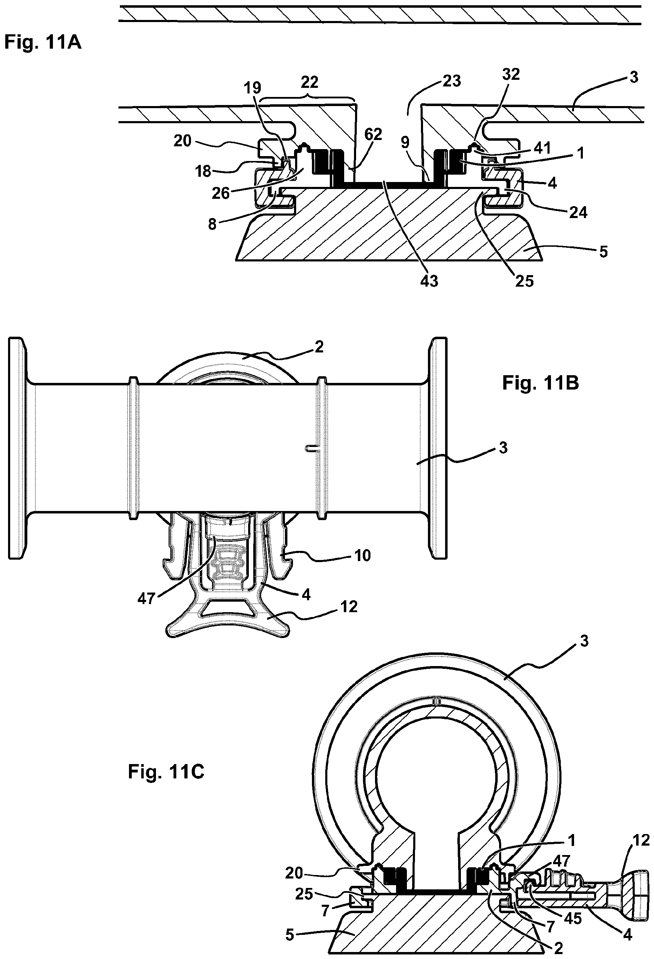

**Figur 11A** zeigt eine weitere Ausführungsform des Verbindungssystems, in dem ein erster Körpers (5), eine fluiddichte Membran (1), ein scheibenförmiges Halteelement (2) und ein lösbares Verbindungselement (4) enthalten sind. Das Verbindungselement (4) ist als U-förmige Klammer ausgeführt und befindet sich in geschlossener Stellung, in der die U-förmige Klammer einen umlaufenden Abschnitt des scheibenförmigen Halteelements (weiß dargestellt) kontaktiert (vgl. Fig. 10). Das Verbindungssystem ist mit einem ersten Körper (5) verbunden, der einen Flansch (25) aufweist. Der zweite Körper (3) hat einen Innenraum (23), der in einer Öffnung (43) endet. Die Öffnung (43) wird von einer umlaufenden Wand (22) eingefasst, die eine an die Öffnung (43) angrenzende umlaufende Wulst (62) aufweist. Die umlaufende Wulst (62) weist einen umlaufenden Membrankontaktabschnitt (9) auf, auf dem die Membran (1) aufliegt. Weiterhin weist die umlaufende Wand (22) eine Nut (32) auf. Eine Wulst (41) einer Wand (26) des scheibenförmigen Halteelements liegt in der Nut (32). Die umlaufende Wand (22) weist weiterhin einen vorspringenden Wandabschitt (20) mit einem Führungssteg (18) auf. Der Führungssteg (18) kontaktiert einen Steg (19) des Verbindungselements (4). Die periphere Außenwand (24) des scheibenförmigen Halteelements setzt sich in eine in Richtung des ersten Körpers (5) weisende Führungskante (8) auf, die den Flansch (25) von außen kontaktiert. Das Verbindungselement (4) umgreift die Führungskante (8) und den Flansch (25). Die Dimensionen des Innenraums des fluidführenden Systems im zweiten Körper (3) sind nicht maßstabsgetreu, sondern zur Anschaulichkeit angepasst. Die Verhältnisse der Dimensionen von umlaufender Wand (22), Membran (1), Flansch (25), Verbindungselement (4) und Halteelement sind im Wesentlichen maßstabsgetreu und lediglich für die Widergabe als Figur verkleinert dargestellt.
**Figur 11B** zeigt die in Fig. 11A gezeigte Ausführungsform von oben. Auch hier befindet sich das Verbindungselement (4) in geschlossener Stellung, in der die U-förmige Klammer desselben einen umlaufenden Abschnitt des scheibenförmigen Halteelements (2) kontaktiert (vgl. Fig. 10). Die dargestellten Verhältnisse entsprechen im Wesentlichen dem Original der gezeigten Ausführungsform, die Größe wurde lediglich für die Widergabe als Figur verkleinert. Wie sich der Figur entnehmen lässt, reichen die Arme (10) des scheibenförmigen Halteelements (2) durch die Aussparungen des Verbindungselements (4), das das Griffelement (12) aufweist, hindurch. Ein Bügel (47) des Halteelements (2) ist in Draufsicht erkennbar, in der er teilweise das Verbindungselement (4) verdeckt.
**Figur 11C** zeigt die in Fig. 11A und Fig. 11B gezeigte Ausführungsform aus einer seitlichen Perspektive, nämlich entlang einer Achse des zweiten Körpers (3). Das als U-förmige Klammer ausgeführte Verbindungselement (4) befindet sich wieder in geschlossener Stellung, in der es einen umlaufenden Abschnitt des scheibenförmigen Halteelements (2) kontaktiert. Zu beachten ist, dass in dieser Perspektive das Verbindungselement (4) den Flansch (25) nicht umgreift, da dieses Umgreifen nur auf der dem Betrachter zugewandten und abgewandten Seite vorhanden ist. Erkennbar sind stattdessen Hakenelemente (7) des scheibenförmigen Halteelements (2). Ein Bügel (47) des scheibenförmigen Halteelements (2) arretiert einen Halter (45) des Verbindungselements (4). Die dargestellten Verhältnisse entsprechen im Wesentlichen dem Original der gezeigten Ausführungsform, die Größe wurde lediglich für die Widergabe als Figur verkleinert.

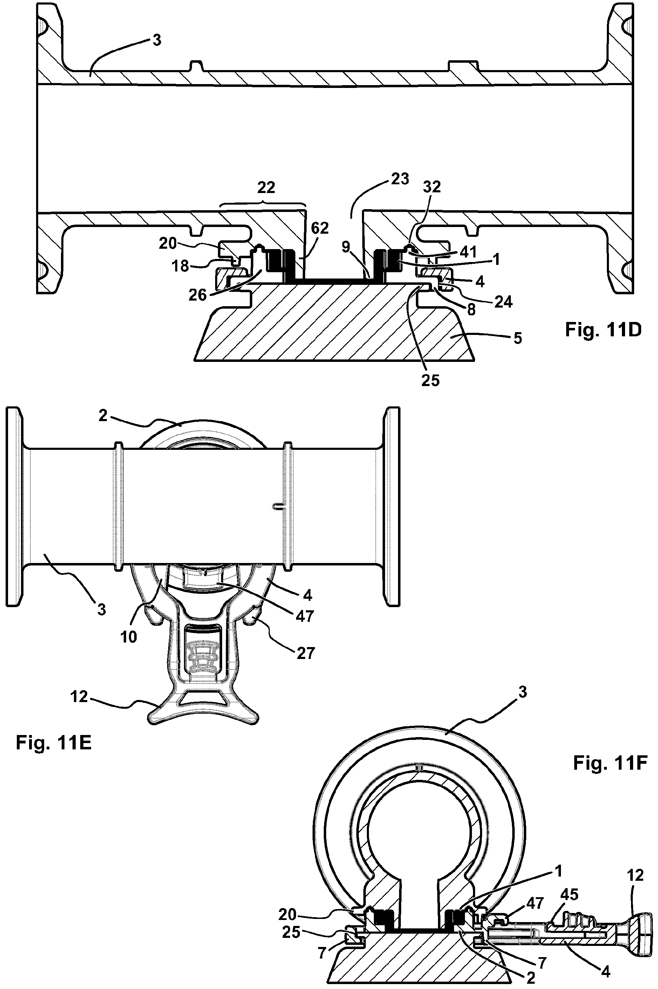

In **Figur 11D** ist die in Fig. 11A schematisch wiedergegebene Ausführungsform des Verbindungssystems maßstabsgetreu gezeigt. Das Verbindungselement (4) ist als U-förmige Klammer ausgeführt und befindet sich in offener Stellung, in der die U-förmige Klammer vom umlaufenden Abschnitt des scheibenförmigen Halteelements (2) im Wesentlichen gelöst ist (vgl. Fig. 10). Der Bereich des Verbindungselements (4), der einen Steg (19) aufweist, ist relativ zum zweiten Körper (3) und insbesondere zum Wandabschnitt (20) so verschoben, dass er im gezeigten Querschnitt nicht erkennbar ist (vgl. z.B. Fig. 10B). In dieser Stellung umgreift das Verbindungselement (4) nicht den peripheren Außenrand (24) des Halteelements und die Führungskante (8) des Halteelements kontaktiert im Bereich des gezeigten Querschnitts die Umgebung, nicht jedoch das Verbindungselement (4).
**Figur 11E** zeigt die in Fig. 11A bis D und F gezeigte Ausführungsform in offener Stellung, vgl. Fig. 11B. Von den Armen (10) des scheibenförmigen Halteelements (2) reicht lediglich deren Ende mit Haken (27) durch die Aussparungen des Verbindungselements (4) hindurch.
**Figur 11F** zeigt die in Fig. 11A bis E gezeigte Ausführungsform in offener Stellung. Im Vergleich zu Fig. 11C ist das als U-förmige Klammer ausgeführte Verbindungselement (4) vom Betrachter aus nach rechts verschoben. Der Bügel (47) des scheibenförmigen Halteelements (2) und der Halter (45) des Verbindungselements (4) sind gegeneinander versetzt angeordnet. Auf Grund der Perspektive umgreift die Klammer (4) im gezeigten Querschnitt nach wie vor nicht den Flansch (25), da ein solches Umgreifen nur auf der dem Betrachter zugewandten und abgewandten Seite erfolgen kann. Erkennbar sind stattdessen Hakenelemente (7) des scheibenförmigen Halteelements (2).

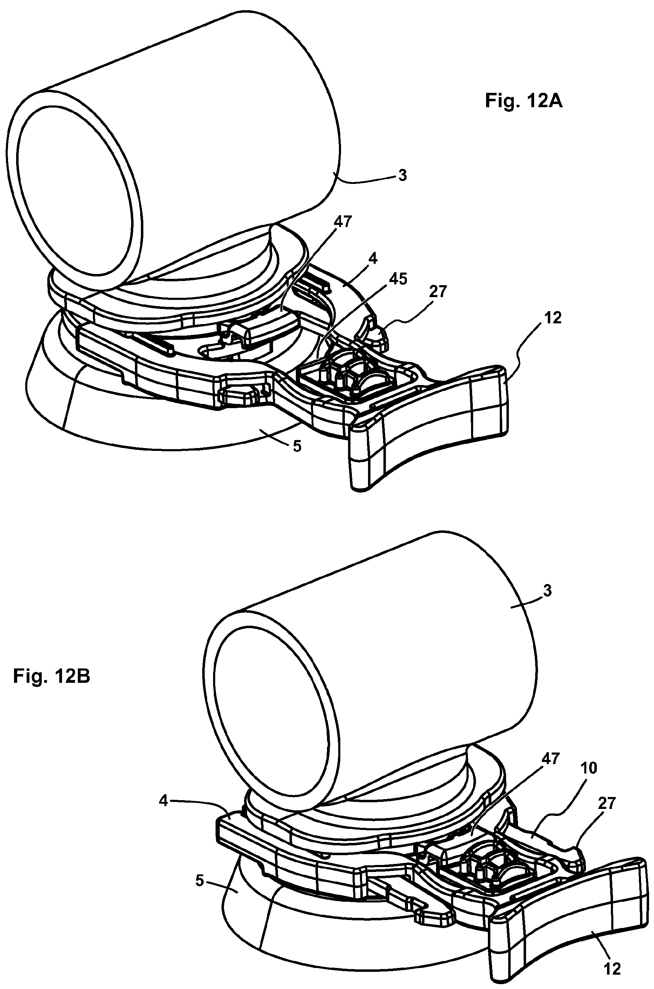

**Figur 12** zeigt die in Fig. 11 gezeigte Ausführungsform des Verbindungssystems perspektivisch in offener **(A)** und geschlossener **(B)** Stellung. In offener Stellung halten die Haken (27) des scheibenförmigen Halteelements (2) die Arme (10) in den Aussparungen (13) und verhindern ein vollständiges Trennen von Verbindungselement (4) und scheibenförmigem Halteelement (2). In geschlossener Stellung hält der Bügel (47) des scheibenförmigen Halteelements (2) den Halter (45) des Verbindungselements (4) und verhindert ein Verschieben von Verbindungselement (4) und Halteelement (2) gegeneinander.
**Figur 13** zeigt eine Ausführungsform des Verbindungssystems, in dem das Verbindungselement (4) einen segmentierten Verriegelungsring definiert. Figur **13C** zeigt einen Querschnitt des Verbindungssystems in arretierter Form, während in Fig. **13A** der Querschnitt des Verbindungselements (4) entsprechend dem Arretierungsmechanismus seitlich verdreht ist. Figuren 11B und 11D zeigen dementsprechend eine erste entriegelte Stellung und eine zweite arretierte Stellung des Verriegelungsrings. Fig. **13E** zeigt den zweiten Körper (3) mit dem Verbindungselement (4) von schräg oben, also aus der Perspektive dem ersten Körper (5) schräg gegenüber. Fig. **13F** zeigt den ersten Körper (5) von schräg oben, also aus einer Perspektive seitlich schräg durch den zweiten Körper (3). Der Flansch (25) des ersten Körpers (5) weist Vorsprünge (40) zum Arretieren des Verriegelungsrings auf.

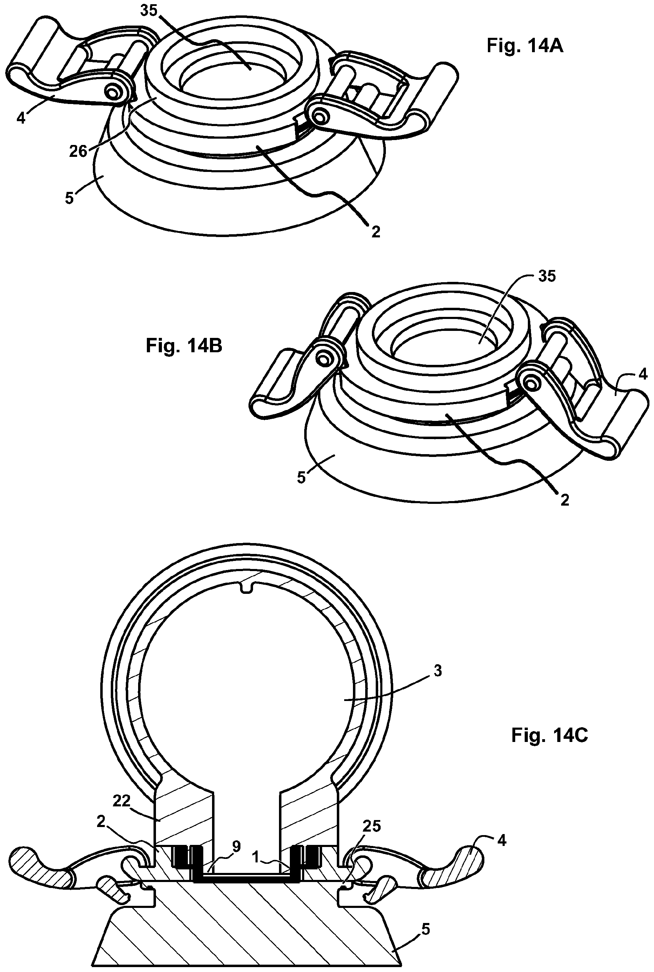

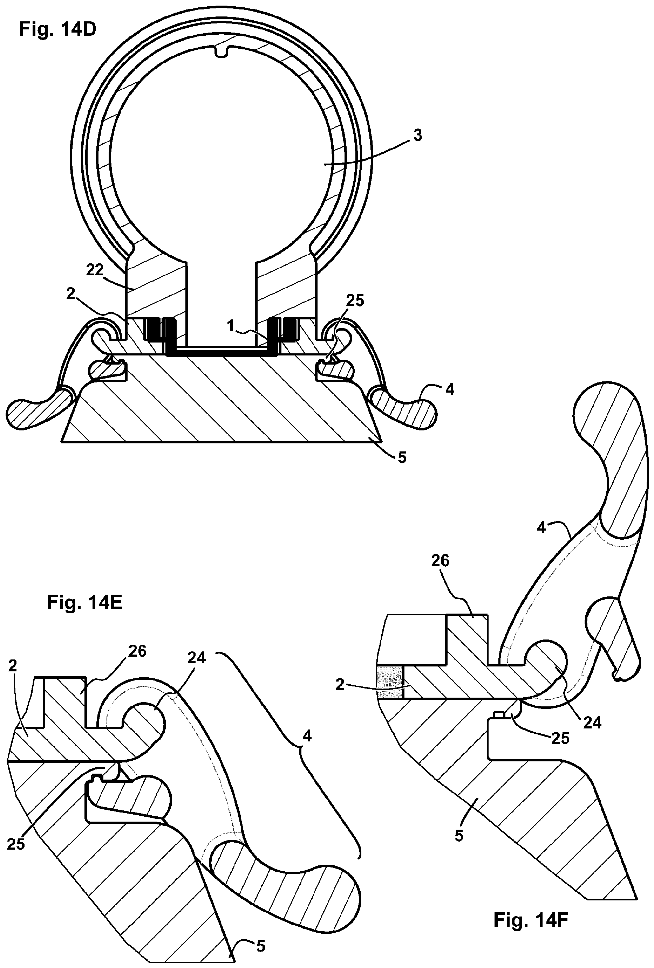

**Figur 14** zeigt eine Ausführungsform des Verbindungssystems, in dem das Verbindungselement (4) durch zwei einander gegenüberliegende Klemmhebel definiert wird. Die Klemmhebel sind in dieser Ausführungsform an den peripheren Außenrand (24) des Halteelements (2) drehbar gekoppelt. Fig. **14A** und Fig. **14C** zeigen das Verbindungssystem mit gelösten Klemmhebeln, während Fig. **14B** und Fig. **14D** das Verbindungssystem mit arretierten Klemmhebeln zeigen. Fig. **14F** und Fig. **14E** zeigen jeweils einen vergrößerten Ausschnitt als Querschnitt der in Fig. **14A** und Fig. **14C** bzw. in Fig. **14B** und Fig. **14D** gezeigten Positionen. Die Klemmhebel (4) umgreifen den peripheren Außenrand (24) des Halteelements (2) und umgreifen im arretierten Zustand den Flansch (25) des ersten Körpers (5).

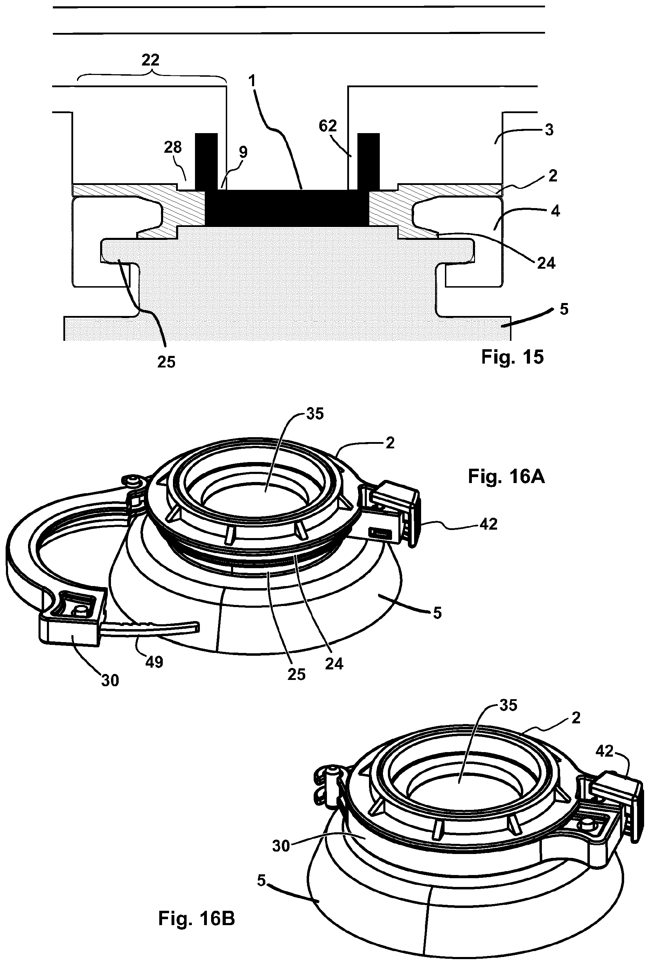

**Figur 15** zeigt eine Ausführungsform des Verbindungssystems, in dem das Verbindungselement (4) eine an den Flansch (25) angrenzende leicht gewinkelte Fläche aufweist. Der Flansch (25) weist eine an das Verbindungselement (4) angrenzende Fläche auf, die konisch zuläuft. Beim Aufschieben des Verbindungselements (4) auf den Flansch (25) ergibt sich dadurch eine Spannung, die dazu beiträgt, eine feste Verbindung herzustellen.

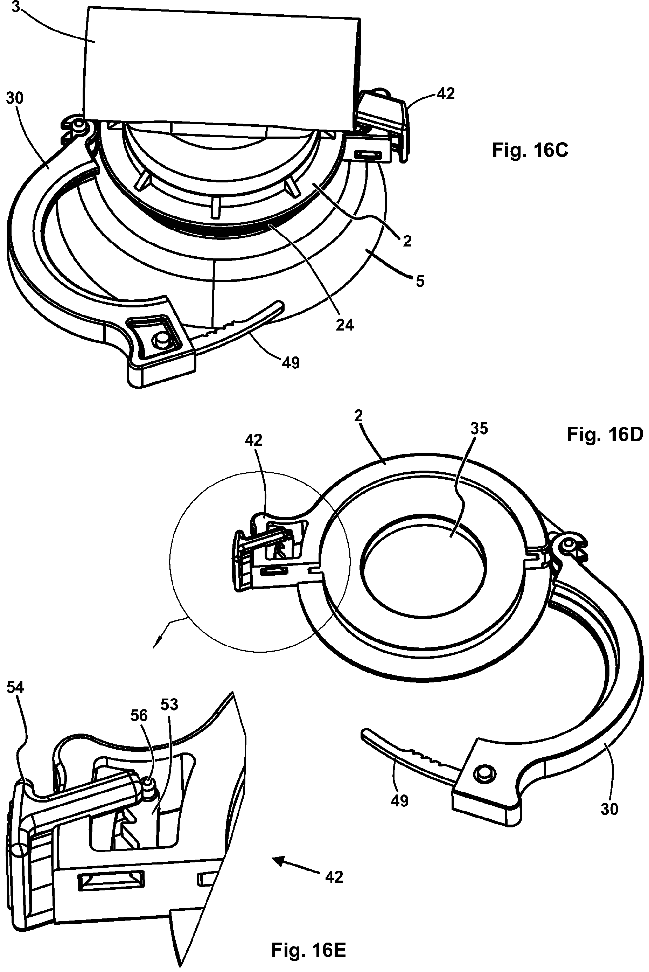

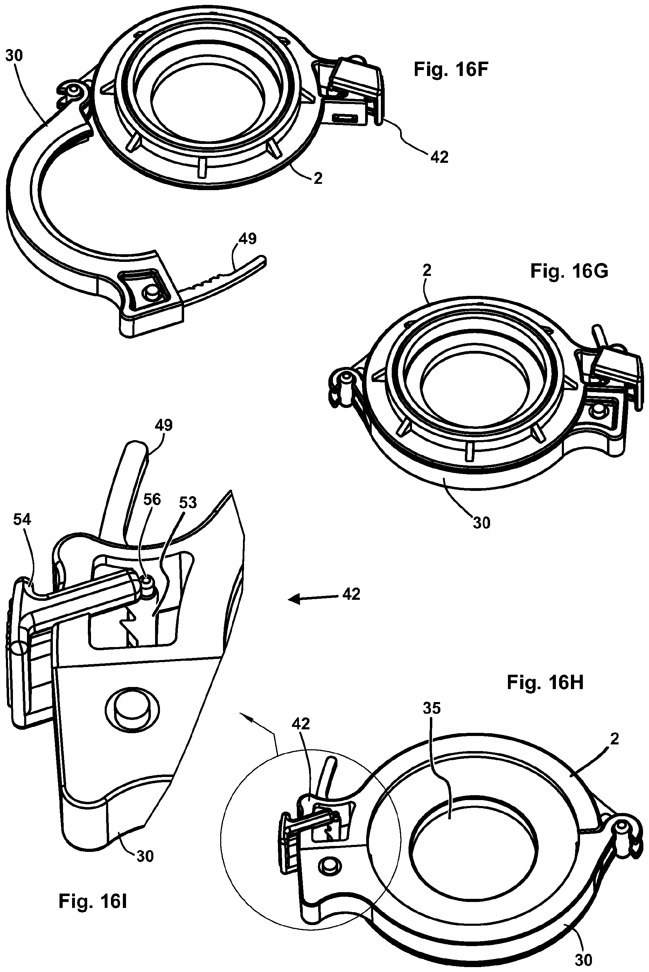

**Figur 16** zeigt eine Ausführungsform des Verbindungssystems, in dem das Verbindungselement (4) von einem schwenkbaren Arm (30) mit einer Zunge (49) definiert wird, die in einen nietenförmigen Vorsprung (42) einrasten kann, der am peripheren Außenrand des scheibenförmigen Halteelements (2) angebracht ist. **Fig. 16A** zeigt das Verbindungselement (4) in gelöstem Zustand, während in **Fig. 16B** die Zunge (49) des Verbindungselements (4) im Vorsprung (42) fixiert ist. In Fig. 16A ist erkennbar, dass das Halteelement (2) auf dem Flansch (25) des ersten Körpers (5) aufliegt. **Fig. 16C** ist eine perspektivische Ansicht des zwischen dem zweiten Körper (3) und dem ersten Körper (5) angebrachten Verbindungselements (4), das von einem schwenkbaren Arm (30) mit einer Zunge (49) definiert wird. Der Arm ist in diesem Zustand nicht im nietenförmigen Vorsprung (42) eingerastet. Die Ansicht ist von schräg oben, also aus Richtung des zweiten Körpers (3). Die Ansicht ist von schräg oben, also aus Richtung des zweiten Körpers (3). Die Verhältnisse der Dimensionen von Arm (22), erstem Körper (5), zweitem Körper (5), Zunge (49), nietenförmigem Vorsprung (42) und Halteelement (2) sind im Wesentlichen maßstabsgetreu und lediglich für die Widergabe als Figur verkleinert dargestellt. **Fig. 16D** zeigt das scheibenförmige Halteelement (2) mit daran befindlichem Arm (30) und nietenförmigem Vorsprung (42) von unten, also aus Richtung des ersten Körpers (5). Der durch einen Kreis markierte nietenförmige Vorsprung (42) ist in **Fig. 16E** in etwa fünffacher Vergrößerung gezeigt. Ein Griffelement (54) ist mit einer Zahnstange (53) über einen Zapfen (56) gekoppelt. **Fig. 16F** zeigt das scheibenförmige Halteelement (2) mit daran befindlichem Arm (30) und nietenförmigem Vorsprung (42) von schräg oben, also aus Richtung des zweiten Körpers (3). Gezeigt ist erneut ein Zustand, in dem der Arm (30) nicht im nietenförmigen Vorsprung (42) eingerastet ist. **Fig. 16G** zeigt aus gleicher Perspektive wie Fig. 16F das scheibenförmige Halteelement (2) mit daran befindlichem Arm (3), der jedoch im nietenförmigen Vorsprung (42) eingerastet ist. **Fig. 16H** zeigt das scheibenförmige Halteelement (2) mit Arm (30) und nietenförmigem Vorsprung (42) im gleichen eingerasteten Zustand von schräg unten, also aus Richtung des ersten Körpers (5). Der durch einen Kreis markierte nietenförmige Vorsprung (42) von Fig. 16H ist in **Fig. 16E** in etwa fünffacher Vergrößerung gezeigt. Das Griffelement (54) ist mit der Zahnstange (53) über einen Zapfen (56) gekoppelt.

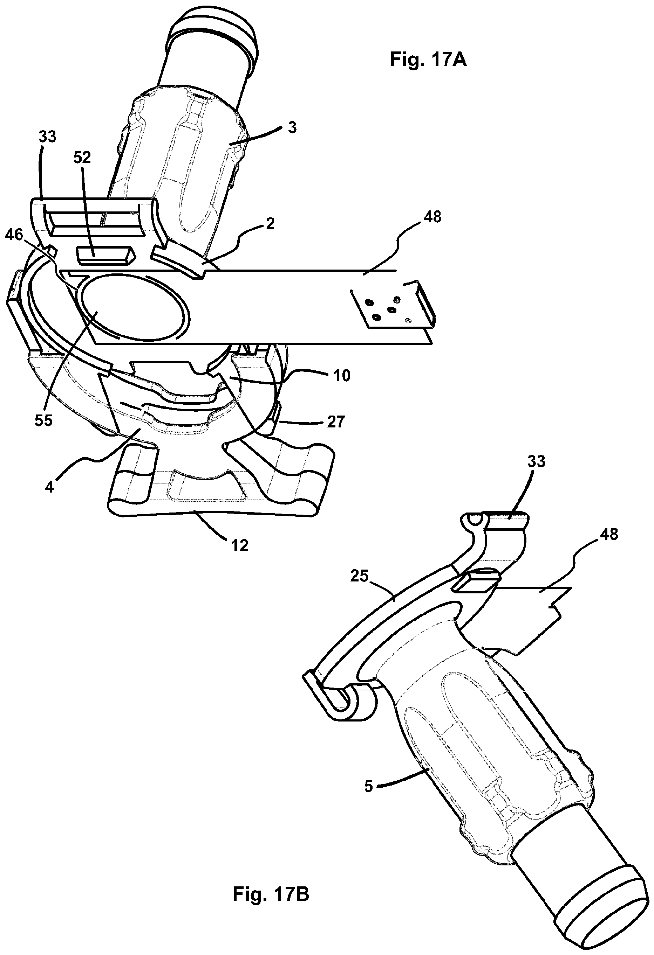

**Figur 17** zeigt ein Verbindungssystem zum lösbar kraftschlüssigen Koppeln eines ersten in **Fig. 17B** gezeigten fluidführenden Körpers (5) mit einem zweiten in **Fig. 17A** gezeigten fluidführenden Körper (3). Der erste und der zweite Körper sind durch ein Scharnier (33) beweglich verbunden. Die Öffnung des Innenraums des ersten Körpers (5) und die zentralen Öffnung (55) des Innenraums des zweiten Körpers (3) sind jeweils durch eine lösbare Schutzfolie (48) bedeckt. Die lösbare Schutzfolie (48) die den Innenraums des zweiten fluidführenden Körpers (3) verschließt, liegt auf einem umlaufenden Vorsprung (46) auf, s. Fig. 17A. Der erste Körper (5) weist ein erstes zylindrisches Ende mit einem Flansch (25) auf. Der zweite Körper (3) weist ein zweites zylindrisches Ende auf, das in ein flanschartiges, scheibenförmiges Halteelement (2) mit einem Führungssteg (52) und zwei Schenkeln (10) mündet. Am scheibenförmigen Halteelement (2) ist ein Verbindungselement (4) mit einem Griffelement (12) angebracht. Aus der peripheren Außenwand des Halteelements (2) entspringen zwei Arme mit Haken (27), die in entsprechende Aussparungen oder Durchlässe des Verbindungselements (4) ein- bzw. durchschiebbar sind.

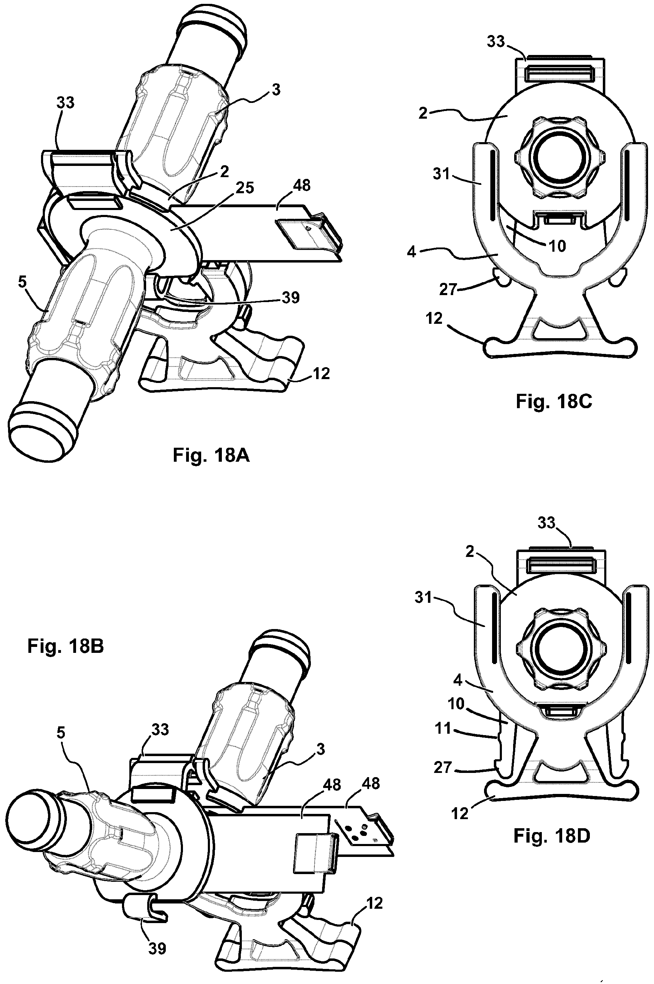

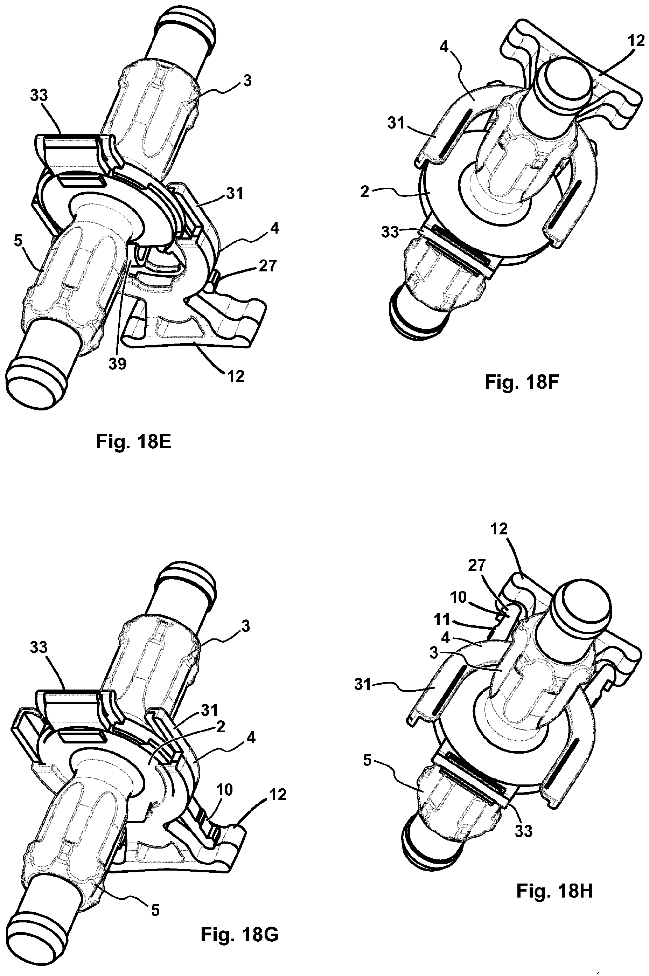

**Figur 18A** zeigt das in Fig. 17 gezeigte Verbindungssystem in einem arretierbar offenen Zustand, in dem das flanschartige, scheibenförmige Halteelement (2) des zweiten Körpers (3) auf dem Flansch (25) des ersten Körpers (5) aufliegt. Ein Haken (39) fixiert den ersten und den zweiten Körper relativ zueinander. Lösbare Schutzfolien (48) versperren den jeweiligen Innenraum des ersten (5) und zweiten (3) Körpers. **Fig. 18B** zeigt einen aufgeklappten Zustand, in dem der erste Körper (5) und der zweite Körpers (3) lediglich durch das Scharnier schwenkbar aneinander gekoppelt sind. Der Haken (39) fixiert nicht den ersten und den zweiten Körper aneinander. **Fig. 18C** zeigt eine der in Fig. 18A im Wesentlichen entsprechende Anordnung schematisch von oben, also aus der Richtung des zweiten Körpers (3). Der Zustand des Verbindungssystems unterscheidet sich vom in Fig. 18A gezeigten Zustand dadurch, dass die Schutzfolien (48) entfernt sind. Es lassen sich Schenkel (10) des flanschartigen, scheibenförmigen Halteelements (2) und Arme (31) des Verbindungselements (4) erkennen. Fig. 18E und Fig. 18F zeigen den in Fig. 18C gezeigten Zustand aus zwei verschiedenen seitlichen Perspektiven. **Fig. 18D** zeigt die Anordnung der Fig. 18C in einem geschlossenen arretierten Zustand, in dem das Verbindungselement (4) den ersten und den zweiten Körper fixiert, indem es den Flansch (25) und den peripheren Außenrand des Halteelements zu etwa der Hälfte oder einige Grad mehr als die Hälfte (s.u.) des jeweiligen Umfangs umgreift. **Fig. 18G** und **Fig. 18H** zeigen den in Fig. 18D gezeigten Zustand aus zwei verschiedenen seitlichen Perspektiven.

### AUSFÜHRLICHE BESCHREIBUNG

Ein hier offenbartes Verbindungssystem erlaubt in der Regel eine robuste und definierte mechanische Ankopplung eines ersten Körpers an einen fluidführenden zweiten Körper wie z.B. eines Messaufnehmers an eine Einwegkomponente. Dabei ist die Verbindung typischerweise durch eine einfache Montage möglich. In einigen Ausfiihrungsformen ist diese Montage sogar mit einer Hand sicher und zuverlässig durchführbar. Zur Verbindung trägt insbesondere ein Verbindungselement bei, das an ein am zweiten Körper befindliches Halteelement und an den ersten Körper fixierbar ist. Das Fluid ist in einigen Ausfiihrungsformen eine Flüssigkeit wie beispielsweise ein wässriges Medium.

Dies lässt sich einerseits dadurch erreichen, dass die Kopplung des ersten und des zweiten Körpers typischerweise auf engstem Raum durch direktes Aufeinanderpressen des scheibenförmigen Halteelements mit dem Flansch des ersten Körpers, z.B. des Messwertaufnehmers durch das Verbindungselement (4) erfolgt. Das scheibenförmige Halteelement verbindet wiederum die Membran auf engem Raum direkt mit dem zweiten Körper, z.B. der Messkammer. In Ausführungsformen, in denen ein Messwertaufnehmer und eine Messkammer gekoppelt werden, ist dadurch ein sicherer Kraftschluss und damit eine sichere Kraftmessung der auf die Membranfläche wirkenden Druckkraft gewährleistet.

Darüber hinaus bietet die Ausgestaltung des Verbindungselementes eines hier offenbarten Verbindungssystems die Möglichkeit einer einfachen und sicheren Montage. Bei dem hier offenbarten Verbindungssystem kann auch eine sichere De- und Remontage sichergestellt werden.

Die Membran ist so dimensioniert, dass sie den Innenraum des zweiten Körpers gegenüber der Umgebung verschließt, indem sie die Öffnung des zweiten Endes des zweiten Körpers bedeckt. Dabei kann die Membran in einigen Ausführungsformen passgenau im Durchlass des Halteelements positioniert sein und diesen dabei verschließen. In einigen Ausführungsformen kann die Membran so dimensioniert sein, dass sie mit einem umlaufenden Membrankontaktabschnitt des zweiten Endes des zweiten Körpers überlappt. Dabei kann die Membran in einer Ebene der Öffnung des zweiten Endes des zweiten Körpers eine größere Weite aufweisen als diese Öffnung des zweiten Endes des zweiten Körpers.

In einigen Ausführungsformen kann das zweite Ende des zweiten Körpers ein Element aufweisen, das sich zumindest teilweise in den Durchlass des Halteelements erstreckt, beispielsweise in Form einer umlaufenden Wulst der umlaufenden Wand des zweiten Endes des zweiten Körpers. Ein solches Element, wie eine Wulst oder ein Steg, kann dabei in einigen Ausführungsformen mit einer dem Innenraum des zweiten Endes des zweiten Körpers zugewandten Fläche des scheibenförmigen Halteelements in Kontakt sein, beispielsweise passgenau anliegen. Ein solches Element kann in einigen Ausführungsformen mit der Membran in Kontakt sein. So kann beispielsweise in einigen Ausfiihrungsformen eine Wulst einen um die Öffnung des zweiten Endes des zweiten Körpers umlaufenden Membrankontaktabschnitt definieren, der im montierten Zustand dem ersten Körper zugewandt ist. Dieser Membrankontaktabschnitt kann mit der Membran in Kontakt sein. Dieser Membrankontaktabschnitt kann mit der Membran fest verbunden sein, beispielsweise durch Verschweißen, Verkleben oder Versiegeln. In einigen Ausführungsformen kann eine Wulst eine dem Innenraum des zweiten Endes des zweiten Körpers abgewandte Fläche, beispielsweise eine umlaufende Fläche, aufweisen. Eine solche dem Innenraum des zweiten Endes des zweiten Körpers abgewandte Fläche kann in einigen Ausfiihrungsformen mit der Membran in Kontakt sein. Diese Fläche kann mit der Membran fest verbunden sein, beispielsweise durch Verschweißen, Verkleben oder Versiegeln. In einigen Ausführungsformen kann die Membran zwischen einer solchen dem Innenraum des zweiten Endes des zweiten Körpers abgewandte Fläche und dem scheibenförmigen Halteelement angeordnet sein, beispielsweise in Sandwich-Anordnung. Dabei kann die Membran passgenau zwischen der betreffenden Fläche, z.B. einer Wulst oder eines Stegs, und dem scheibenförmigen Halteelement positioniert sein.

Wie bereits im Vorangehenden erläutert, enthält das Verbindungssystem in einigen Ausführungsformen, in denen das scheibenförmige Halteelement eine endständige Ausgestaltung des zweiten Endes des zweiten Körpers definiert, keine Membran. In derartigen Ausführungsformen dient das Verbindungssystem dem lösbar kraftschlüssigen Koppeln eines Innenraums des ersten fluidführenden Körpers mit einem Innenraum des zweiten fluidführenden Körpers.

Soweit nicht anders angegeben oder aus dem Zusammenhang eindeutig eine andere Bedeutung ersichtlich ist, haben die folgenden Begriffe und Ausdrücke wenn sie in diesem Dokument, inklusive Beschreibung und Patentansprüchen, verwendet werden, die im Folgenden angegebenen Bedeutungen.

Der Ausdruck "bestehend aus" wie in diesem Dokument verwendet, bedeutet einschließend und begrenzt auf das, was auf den Begriff "bestehend aus" folgt. Der Begriff "bestehend aus", gibt somit an, dass aufgeführte Elemente erforderlich oder notwendig sind und dass keine weiteren Elemente vorhanden sein dürfen. Der Begriff "im Wesentlichen bestehend aus" wird dahingehend verstanden, dass er bedeutet, dass jedwede Elemente, die nach dem Ausdruck definiert sind, umfasst sind und dass weitere Elemente, beispielsweise in einer Probe oder einer Zusammensetzung zugegen sein können, die die Aktivität oder Wirkung, die für die betreffenden Elemente in diesem Dokument angegeben sind, nicht verändern, also sie nicht beeinträchtigen und nicht zu ihr beitragen. Anders gesagt gibt der Ausdruck "im Wesentlichen bestehend aus" an, dass die definierten Elemente notwendig oder erforderlich sind, dass aber weitere Elemente optional sind und zugegen sein können oder nicht, je nachdem, ob sie für die Wirkung oder Wirksamkeit der definierten Elemente von Belang sind oder nicht.

Das Wort "etwa" bezieht sich wenn hier verwendet auf einen Wert, der für einen bestimmten Wert, wie von einem Durchschnittsfachmann bestimmt, innerhalb eines akzeptablen Fehlerbereichs liegt. Dies wird teilweise davon abhängig sein, wie der jeweilige Wert ermittelt oder gemessen worden ist, d.h. von den Einschränkungen des Messsystems. "Etwa" kann beispielsweise innerhalb einer Standardabweichung von 1 oder mehr bedeuten, je nach Gebrauch im jeweiligen Gebiet. Der Begriff "etwa" wird auch verwendet um anzugeben, dass der Betrag oder Wert der bezeichnete Wert sein kann oder ein anderer Wert, der näherungsweise gleich ist. Der Begriff soll ausdrücken, dass ähnliche Werte gleichwertige Ergebnisse oder Wirkungen, wie in diesem Dokument offenbart, begünstigen. In diesem Zusammenhang kann "etwa" sich auf einen Bereich von bis zu 10 % über und/oder unter einem bestimmten Wert beziehen. In einigen Ausführungsformen bezieht "etwa" sich auf einen Bereich von bis zu 5 % über und/oder unter einem bestimmten Wert, wie etwa 2 % über und/oder unter einem bestimmten Wert. In einigen Ausfiihrungsformen bezieht "etwa" sich auf einen Bereich von bis zu 1 % über und/oder unter einem bestimmten Wert. In einigen Ausführungsformen bezieht "etwa" sich auf einen Bereich von bis zu 0,5 % über und/oder unter einem bestimmten Wert. In einer Ausführungsform bezieht sich "etwa" auf einen Bereich von bis zu 0,1 % über und/oder unter einem bestimmten Wert.

Der Konjunktionalausdruck "und/oder" zwischen mehreren Elementen, wenn hier verwendet, wird als sowohl individuelle als auch kombinierte Optionen umfassend verstanden. Sind beispielsweise zwei Elemente durch "und/oder" verknüpft, betrifft eine erste Option den Einsatz des ersten Elements ohne das zweite. Eine zweite Option betrifft den Einsatz des zweiten Elements ohne das erste. Eine dritte Option betrifft den Einsatz des ersten und des zweiten Elements zusammen. Es wird verstanden, dass jede beliebige dieser Optionen unter die Bedeutung des Ausdrucks fällt und somit die Bedingungen des Begriffs "und/oder", wie in diesem Dokument verwendet, erfüllt.

Singularformen wie "eine", "ein", "der", "die" oder "das" schließen die Pluralform ein, wenn sie in diesem Dokument verwendet werden. So bezeichnet beispielsweise eine Bezugnahme auf "eine Zelle" sowohl eine individuelle Zelle als auch eine Mehrzahl an Zellen. In einigen Fällen wird explizit der Ausdruck "ein oder mehrere" verwendet, um im jeweiligen Fall darauf hinzuweisen, dass die Singularform die Pluralform mit umfasst. Derartige explizite Hinweise schränken die allgemeine Bedeutung der Singularform nicht ein. Falls nicht anders angegeben, werden die Begriffe "zumindest", "mindestens" und "wenigstens", wenn sie eine Abfolge von Elementen vorangehen, dahingehend verstanden, dass sie sich auf jedes dieser Elemente beziehen. Die Begriffe "zumindest ein", "mindestens ein(e)", "wenigstens einer" oder "wenigstens eine(r) von" schließen beispielsweise ein, zwei, drei, vier oder mehr Elemente ein.

In allen hier offenbarten Ausfiihrungsformen weist das scheibenförmige Halteelement einen peripheren Außenrand auf, der von einem lösbaren Verbindungselement zumindest teilweise zu umgriffen werden kann. Das scheibenförmige Halteelement kann eine Mehrzahl peripherer Außenränder aufweisen. Einer oder mehrere solcher peripherer Außenränder kann/können axial weiter nach außen reichen als andere periphere Außenränder. In solchen Ausfiihrungsformen kann jeder beliebige periphere Außenrand von einem lösbaren Verbindungselement zumindest teilweise umgriffen werden, um eine Verbindung zu erzielen. In einigen Ausführungsformen, in denen mehr als ein peripherer Außenrand vorhanden ist, können mehr als ein peripherer Außenränder von einem lösbaren Verbindungselement zumindest teilweise umgriffen werden. In einigen Ausführungsformen ist ein entsprechend umgriffener peripherer Außenrand der äußerste periphere Außenrand, den das lösbare Verbindungselement aufweist. In einigen Ausführungsformen ist ein entsprechend umgriffener peripherer Außenrand nicht der äußerste periphere Außenrand, den das lösbare Verbindungselement aufweist, vgl. z.B. Fig. 15 oder Fig. 16. In einigen Ausführungsformen, z.B. der von Fig. 16, grenzt der periphere Außenrand des scheibenförmigen Halteelements, der vom lösbaren Verbindungselement zumindest teilweise umgriffen wird, im montierten Zustand unmittelbar an den Flansch des ersten Körpers an. Fig. 16A zeigt beispielsweise ein Verbindungselement, das von einem beweglichen Arm (30) mit einer Zunge (49) und einem nietenförmigen Vorsprung (42) definiert wird. Über dem Flansch (25) des ersten Körpers (5) ist gerade noch eine Wand erkennbar, die einen peripheren Außenrand (24) definiert. Wie Fig. 16B zeigt, ist dieser peripheren Außenrand (24) der peripheren Außenrand, der vom Verbindungselement teilweise umgriffen wird.

Bereitgestellt wird durch diese Offenbarung ein Verbindungssystem zum lösbar kraftschlüssigen Koppeln eines ersten Körpers (z.B. eines ersten Körpers (5)) an einen zweiten fluidführenden Körper (z.B. einen zweiten Körper (3)). Der erste Körper weist ein erstes Ende mit einem Flansch (25), beispielsweise mit einem ringförmigen Flansch, auf.

Kraftschlüssiges Koppeln, wenn hier verwendet, bedeutet ein Koppeln eines ersten an einen zweiten Körper, bei den ein Zustand hergestellt wird, in dem der erste und der zweite Körper daran gehindert sind, sich gegeneinander zu bewegen. Beispielsweise ist verhindert, dass sich der erste und der zweite Körper gegeneinander verschieben oder verdrehen. Typischerweise wird der Zustand der kraftschlüssigen Kopplung in einem hier offenbarten System durch ein Arretieren erreicht. Beispielsweise kann eine Schiebe- oder Drehbewegung eines Verbindungselements bis zu einer vorgesehenen Endposition möglich sein, in der ein Verriegelungsmechanismus ausgelöst wird. Beispielsweise kann ein Verriegelungselement einrasten und dadurch eine weitere oder rückwärtige Schiebe- oder Drehbewegung verhindern. Es sollte somit ersichtlich sein, das der Kraftschluss beispielsweise an einen oder mehreren Elementen des Verbindungssystems einen Formschluss beinhalten kann.

In typischen Ausführungsformen erzielt ein hier offenbartes Verbindungssystem eine kraftschlüssige Verbindung des entsprechenden ersten und des zweiten Körpers. In einigen Ausführungsformen weist der Flansch des ersten Körpers eine dem zweiten Körper zugewandte Kontaktfläche auf. Das scheibenförmige Halteelement kann in solchen Ausfiihrungsformen eine erste Seite aufweisen, die dem zweiten Körper zugewandt ist. Das scheibenförmige Halteelement kann eine zweite Seite aufweisen, die dem zweiten Körper zugewandt ist. Diese zweite Seite kann eine Kontaktfläche aufweisen, die für einen flächigen Kontakt mit dem Flansch des ersten Körpers ausgelegt ist bzw. dazu ausgelegt ist, auf dem Flansch des ersten Körpers aufzuliegen. Ein hier offenbartes Verbindungssystem ist dazu ausgelegt, eine Bewegung einer entsprechenden Kontaktfläche des scheibenförmigen Halteelements relativ zur Kontaktfläche, inklusive entlang der Kontaktfläche, des Flansches des ersten Körpers zu verhindern. Dabei ist zum Überwinden des Kraftschlusses in der Regel eine Kraft erforderlich, die ausreicht, um eine mechanische Beschädigung an einem oder mehreren Bauteilen des Verbindungssystems, typischerweise an einem oder mehreren Komponenten des lösbaren Verbindungselements, zu bewirken.

Das hier beschriebene Verbindungssystem weist den zweiten Körper und ein lösbares Verbindungselement (z.B. ein Verbindungselement (4)) auf. Dabei kann der zweite Körper entweder a) ein zweites Ende mit einem Innenraum (z.B. einem Innenraum (23)) und einer umlaufenden Wand (z.B. einer Wand (22)) aufweisen oder b) ein zweites Ende aufweisen, das in ein flanschartiges, scheibenförmiges Halteelement (z.B. ein Halteelement (2)) mit einen peripheren Außenrand (z.B. einen Außenrand (24)) mündet. Im zweiten Fall definiert das scheibenförmige Halteelement somit ein Ende des zweiten Körpers, während es im ersten Fall ein eigenständiges Bauteil definiert.

Wenn der zweite Körper ein zweites Ende mit einem Innenraum und einer umlaufenden Wand aufweist, weist das Verbindungssystem des Weiteren ein scheibenförmiges Halteelement (z.B. ein Halteelement (2)) auf. Das scheibenförmige Halteelement enthält einen Durchlass (z.B. einen Durchlass (35)), eine erste Seite, eine zweite Seite und einen peripheren Außenrand (z.B. einen Außenrand (24)). Die erste Seite und/oder der Durchlass des Halteelements ist/sind an die Membran gekoppelt. Auf der ersten Seite des Halteelements ist eine umlaufende Wand (z.B. eine Wand (26)) angeordnet, die dazu ausgelegt ist, mit der umlaufenden Wand des zweiten Körpers verbunden zu werden. Die zweite Seite des Halteelements ist dazu ausgelegt, mit dem Flansch des ersten Körpers verbunden zu werden. Die zweite Seite des Halteelements kann beispielsweise für einen flächigen Kontakt mit dem Flansch des ersten Körpers ausgelegt sein bzw. dazu ausgelegt sein, auf dem Flansch des ersten Körpers aufzuliegen.

In Ausführungsformen in denen der zweite Körper ein zweites Ende mit einem Innenraum und einer umlaufenden Wand aufweist, weist der zweite Körper typischerweise eine Mündung wie beispielsweise einen Auslass (z.B. eine Öffnung (43)) auf. In einigen derartigen Ausführungsformen weist die umlaufende Wand einen um die Mündung umlaufenden Membrankontaktabschnitt (z.B. einen Membrankontaktabschnitt (9)) auf, der mit der Membran verbunden werden kann oder mit der Membran verbunden ist.

Wenn der zweite Körper ein zweites Ende aufweist, das in ein flanschartiges, scheibenförmiges Halteelement mündet, weist dieses Halteelement eine zentrale Mündung wie beispielsweise einen Auslass (z.B. eine Öffnung (43)) des Innenraums des zweiten fluidführenden Körpers auf.

Das Verbindungselement ist dazu ausgelegt, den peripheren Außenrand des Halteelements und den Flansch des ersten Körpers zumindest teilweise zu umgreifen, sodass das Halteelement mit Hilfe des Verbindungselements kraftschlüssig am ersten Körper fixiert ist.

In einigen Ausführungsformen ist der erste Körper nicht fluidführend. In einigen Ausführungsformen weist der erste Körper keinen Innenraum auf. In einigen Ausführungsformen weist der erste Körper einen Innenraum auf, ist jedoch nicht fluidführend. Beispielsweise kann in Ausführungsformen, in denen das Verbindungssystem ein scheibenförmiges Halteelement als eigenständiges Bauteil aufweist, der erste Körper keinen Innenraum aufweisen. Auch kann in Ausführungsformen, in denen das Verbindungssystem ein scheibenförmiges Halteelement aufweist, der erste Körper ein nicht fluidführender Körper sein.

In einigen Ausfiihrungsformen sind sowohl der erste als auch der zweite Körper fluidführend. Beispielsweise können in Ausführungsformen, in denen der zweite Körper ein zweites Ende aufweist, das in ein flanschartiges, scheibenförmiges Halteelement mündet, sowohl der erste als auch der zweite Körper fluidführend sein. In einigen Ausführungsformen erfolgt das Koppeln des ersten Körpers an den zweiten fluidführenden Körper über eine fluiddichte Membran. Das Verbindungssystem weist in solchen Ausführungsformen die Membran und den zweiten Körper mit einem Innenraum auf. In einigen Ausführungsformen ist auf der ersten Seite des Halteelements oder auf dem flanschartigen scheibenförmigen Halteelement ein umlaufender Steg oder eine umlaufende Wulst (z.B. ein Steg oder eine Wulst (62)) angeordnet.

In einigen Ausführungsformen weist das zweite Ende des zweiten Körpers einen Membrankontaktabschnitt auf. Dieser Membrankontaktabschnitt ist in einigen Ausführungsformen dem ersten Körper zugewandt. Wie bereits zuvor angegeben, kann beispielsweise eine umlaufende Wand des zweiten Körpers, beispielsweise eine ringförmig umlaufende Wand, einen solchen Membrankontaktabschnitt aufweisen. In einigen Ausführungsformen weist das zweite Ende des zweiten Körpers unabhängig vom Membrankontaktabschnitt einen Abschnitt auf, der dem ersten Körper zugewandt ist und der vom Membrankontaktabschnitt verschieden ist. Beispielsweise kann ein solcher Abschnitt, der dem ersten Körper zugewandt ist, an den Membrankontaktabschnitt angrenzen. Als illustratives Beispiel können in einer Ausführungsform, in denen der zweite Körper ein zweites Ende aufweist, das in ein flanschartiges, scheibenförmiges Halteelement mündet, auf dem flanschartigen, scheibenförmigen Halteelement ein Membrankontaktabschnitt und ein weiterer Wandabschnitt vorgesehen sein. Dieser weitere Wandabschnitt kann dem ersten Körper zugewandt sein. Für weitere Ausführungsformen wird auch auf die vorangehenden Abschnitte dieses Dokuments verwiesen.

In einem montierten Zustand ist der zweite Körper lösbar an den ersten Körper gekoppelt. Die Öffnung im zweiten Ende des zweiten Körpers ist dem ersten Körper zugewandt. In diesem Zustand weist ein hier beschriebenes Verbindungssystem in einigen Ausführungsformen den ersten Körper, die Membran und den zweiten Körper mit einem Innenraum auf. Der zweite Körper weist neben dem zweiten Ende, das zylindrisches sein kann, typischerweise einen oder mehrere Anschlüsse für ein fluidführendes System auf. Das zweite Ende des zweiten Körpers weist einen Innenraum, eine umlaufenden Wand und eine Öffnung auf. Der Innenraum ist typischerweise an das fluidführende System koppelbar. Die umlaufende Wand weist auf der dem ersten Körper zugewandten Seite einen um die Öffnung umlaufenden Membrankontaktabschnitt auf, an dem die Membran anliegt. Auch in einem montierten Zustand weist das Verbindungssystem ein scheibenförmiges Halteelement und ein Verbindungselement auf. Das scheibenförmige Halteelement kann auch in einem montierten Zustand mit dem zweiten Ende des zweiten Körpers ein einstückiges Bauteil definieren. Ebenso kann das Halteelement ein eigenständiges Bauteil definieren, das lösbar mit dem zweiten Körper verbunden ist. Das Verbindungselement ist in einem montierten Zustand lösbar oder fest mit dem ersten Körper und mit dem scheibenförmigen Halteelement verbunden. Dabei bewirkt das Verbindungselement ein kraftschlüssiges Fixieren des scheibenförmigen Halteelements an den ersten Körper. In Ausführungsformen, in denen das scheibenförmige Halteelement mit dem zweiten Ende des zweiten Körpers ein einstückiges Bauteil definiert, sind auf diese Weise der erste und der zweite Körper lösbar miteinander verbunden. In Ausführungsformen, in denen das scheibenförmige Halteelement ein eigenständiges Bauteil definiert, ist dieses in der Regel mit dem zweiten Körper verbunden, beispielsweise formschlüssig verbunden, verschraubt, verklebt oder verschweißt. Auch in diesen Fällen sind somit der erste und der zweite Körper lösbar miteinander verbunden.

Typischerweise ist das Verbindungselement so ausgelegt, dass es im fixierten Zustand eine Anpresskraft auf einen Flansch des ersten Körpers und eine diesem Flansch zugewandte Fläche des scheibenförmigen Halteelements ausübt. Dabei liegt die betreffende Fläche des scheibenförmigen Halteelements auf dem Flansch des ersten Körpers auf. Die dem Flansch des ersten Körpers zugewandte Fläche des scheibenförmigen Halteelements kann die gesamte Seite des scheibenförmigen Halteelements einnehmen, die dem Flansch zugewandt ist. Diese Seite ist im Vorangehenden auch bereits als zweite Seite des scheibenförmigen Halteelements bezeichnet worden. Die dem Flansch des ersten Körpers abgewandte Seite des scheibenförmigen Halteelements, im Vorangehenden auch bereits als erste Seite des scheibenförmigen Halteelements bezeichnet, ist in der Regel dem zweiten Körper zugewandt. Das scheibenförmigen Halteelement weist darüber einen zentralen Durchlass und einem dem Durchlass abgewandten peripheren Außenrand auf. Der Durchlass kann in einigen Ausführungsformen die Membran enthalten und an diese gekoppelt sein. In einigen Ausfiihrungsformen ist die Seite des scheibenförmigen Halteelements, die dem Flansch zugewandt ist, d.h. die zweite Seite, an die Membran gekoppelt. In einigen Ausführungsformen ist die Seite des scheibenförmigen Halteelements, die dem Flansch abgewandt ist, d.h. die erste Seite, an die Membran gekoppelt. Die umlaufenden Wand des zweiten Endes des zweiten Körpers weist einen um die Öffnung desselben umlaufenden Membrankontaktabschnitt auf. Dieser Membrankontaktabschnitt ist ebenfalls an die Membran gekoppelt. In Ausführungsformen, in denen das scheibenförmige Halteelement ein eigenständiges Bauteil definiert, ist die Seite des scheibenförmigen Halteelements, die dem Flansch zugewandt ist, d.h. die zweite Seite, in der Regel nicht an die Membran gekoppelt. In Ausführungsformen, in denen das scheibenförmige Halteelement eine endständige Ausgestaltung des zweiten Endes des zweiten Körpers definiert, weist eine dem Flansch des ersten Körpers abgewandte Seite des scheibenförmigen Halteelements typischerweise eine Außenfläche auf. Eine solche dem Flansch des ersten Körpers abgewandte Seite eines derartigen scheibenförmigen Halteelements ist in der Regel nicht an die Membran gekoppelt.

In einigen Ausführungsformen, in denen das scheibenförmige Halteelement mit dem zweiten Ende des zweiten Körpers ein einstückiges Bauteil definiert, ist der erste Körper ein fluidführender Körper. Der erste fluidführende Körper weist in diesem Fall einen Innenraum auf und das erste Ende des ersten Körpers weist zusätzlich zu einem Flansch einen Auslass des betreffenden Innenraums auf, typischerweise eine Öffnung. In derartigen Ausführungsformen weist der zweite Körper, nämlich das zweite Ende des zweiten Körpers, eine Öffnung auf, beispielsweise eine zentrale Öffnung, und der erste Körper weist eine Öffnung auf, beispielsweise eine zentrale Öffnung. Diese Öffnung ist eine Öffnung der ersten Endes des ersten Körpers. Die Öffnung des zweiten Körpers ist eine Öffnung des Innenraums des zweiten Endes des zweiten Körpers, der an das fluidführende System gekoppelt ist. Das scheibenförmige Halteelement weist einen Durchlass auf, der in Ausführungsformen, in denen das scheibenförmige Halteelement mit dem zweiten Ende des zweiten Körpers ein einstückiges Bauteil definiert, mit der Öffnung des zweiten Körpers identisch ist. Die Öffnung des ersten Körpers kann ebenso eine Öffnung eines Innenraums des ersten Endes des ersten Körpers sein. Der Innenraum des ersten Endes des ersten Körpers kann ebenfalls an ein fluidführendes System gekoppelt sein. Die Öffnungen des ersten Körpers und des zweiten Körpers sind im montierten Zustand einander zugewandt.

In Ausführungsformen, in denen das scheibenförmige Halteelement mit dem zweiten Ende des zweiten Körpers ein einstückiges Bauteil definiert, kann ein hier offenbartes Verbindungssystem auch in einem noch nicht montierten Zustand bereits den ersten Körper aufweisen. An den ersten Körper kann in diesem Fall das scheibenförmige Halteelement gekoppelt sein, beispielsweise lösbar gekoppelt. Dabei kann das scheibenförmige Halteelement beweglich an den ersten Körper gekoppelt sein. Beispielsweise kann die Öffnung des zweiten Körpers auf die Öffnung des ersten Körpers orientierbar sein.

Die Dimensionen der Öffnung des ersten Körpers und der Öffnung des zweiten Körpers können so vorgesehen sein, dass eine der beiden Öffnungen die maximale Ausdehnung der anderen Öffnung zu nicht mehr als 10 Prozent überschreitet. Sind beide Öffnungen kreisförmige Öffnungen, kann beispielsweise der Durchmesser einer der beiden Öffnungen den Durchmesser der anderen Öffnung zu nicht mehr als 10 Prozent überschreiten. Die Dimensionen der Öffnung des ersten Körpers und der Öffnung des zweiten Körpers können in einigen Ausführungsformen von im Wesentlichen gleicher maximaler Ausdehnung sein, inklusive gleicher maximaler Ausdehnung. Die Formen der beiden Öffnungen können aufeinander abgestimmt sein. Beispielsweise können die Formen der beiden Öffnungen zueinander spiegelbildlich sein. Derartig aufeinander abgestimmte Öffnungen können so aufeinander ausrichtbar sein, dass die Umrisse der Öffnungen deckungsgleich sind. Im montierten Zustand können derartige Öffnungen sich deckungsgleich gegenüber liegen. Sind beide Öffnungen kreisförmige Öffnungen, können beispielsweise die Durchmesser der beiden Öffnungen im Wesentlichen gleich, inklusive identisch, sein.

Zum besseren Verständnis werden anhand der Figuren im Folgenden einige Ausführungsformen des Verbindungssystems erläutert.

In der in Fig. 4 gezeigten Ausführungsform ist das Verbindungselement (4) eine Halbring-förmige Klammer und das scheibenförmige Halteelement (2) ein Haltering. Die Halbring-förmige Klammer (4) presst den Haltering (2) auf den Flansch des ersten Körpers (5). Eine Klammer kann dabei Halbring-förmig im Sinne von etwa 180° eines Kreises umfassend sein. Eine Klammer kann dabei auch Halbring-förmig im Sinne von mehr als etwa 180° eines Kreises umfassend sein, wie beispielsweise etwa 190° oder etwa 195° eines Kreisumfangs. Ein Design mit einem Umfang von einigen Grad mehr als 180° eines Kreisumfangs wie etwa 185° kann dabei vorteilhaft sein, um eine zusätzliche Verankerung zu bewirken. So kann das Verbindungselement (4) aus einem Material wie z.B. Kunststoff gefertigt sein, das in einem bestimmten Maße nachgiebig ist, so dass sich eine entsprechende Klammer als das Verbindungselement (4) bei der Montage verbiegen lässt. Auf diese Weise lässt sich die Klammer zu einem gewissen Grad auseinander dehnen. Eine Klammer, die einige Grad mehr als 180° eines Kreisumfangs umgibt, lässt sich so und über das scheibenförmiges Halteelement (2) schieben. Es schnappt dann über das scheibenförmige Halteelement und nimmt seine vorherige Form wieder ein, in der es mehr als 180° des Umfangs des scheibenförmigen Halteelements umgibt.

In den in beispielsweise Fig. 10A und Fig. 10E gezeigten Ausführungsformen ist das Verbindungselement (4) eine U-förmige Klammer mit Armen (31) und das scheibenförmige Halteelement (2) ein Haltering mit Schenkeln (10). Die Abbildungen 5 bis 6, 8 und 9 zeigen weitere Ausfiihrungsformen eines Verbindungselementes mit einer U-förmigen Klammer, die eine einfache Montage mit Führungen ermöglichen, so dass ggf. auch ohne Sichtkontakt bzw. ohne Betrachten des Verbindungssystems montiert werden kann. Weiterhin sind an Hand dieser Ausfiihrungsformen Möglichkeiten für eine Vormontage des Verbindungselements (4) veranschaulicht, wodurch auch eine Einhandmontage des Verbindungssystems möglich wird, vgl. z.B. Fig. 10 und Fig. 12.

Das scheibenförmige Halteelement (2) kann in z.B. Ausführungsformen der Fig. 5 so über dem ersten Körper (5) orientiert werden, dass sich der Durchlass (35) des scheibenförmigen Halteelements (2) über dem Flansch (25) des ersten Körpers (5) befindet, vgl. Fig. 6. Dabei weisen die Führungskante b) und die Hakenelemente (7) des Halteelements (2) in Richtung des Flansches (25). Am Flansch (25) des ersten Körpers (5) kann dann das scheibenförmige Halteelement (2) durch leichte Kippbewegung mit dem größeren der beiden Hakenelemente (7) eingehakt werden und auf den des ersten Körper (5) gekippt werden.

Auf die gleiche Weise ist ein bereits zuvor mit dem zweiten Körper (3) verbundenes scheibenförmiges Halteelement (2) an den ersten Körper (5) koppelbar. Dabei ist der erste Körper auch an den zweiten Körper fixierbar. Auf den zweiten Körper (3) kann mit eingesetzter Membran (1) das scheibenförmige Halteelement (2) so aufgesetzt sein, dass die Wand (26) des Halteelements (2) am Rücksprung (28) des zweiten Körpers (3) anliegt. Dazu kann zuvor die Membran (1) auf die umlaufende Wand (22) des zweiten Körpers (3) aufgeschoben worden sein und die Wand (26) des Halteelements (2) auf die Membran (1) geschoben worden sein, sodass die Membran (1) den Zylinderinnenraum (23) von der Außenwelt trennt. Der ersten Körper (5) kann so unter dem mit dem zweiten Körper (3) verbundene scheibenförmige Halteelement (2) orientiert werden, dass der Flansch der Führungskante (8) und den Hakenelementen (7) des Halteelements (2) gegenüberliegt. Durch leichte Kippbewegung in das größere der beiden Hakenelemente (7) des Halteelements (2) kann der Flansch eingehakt und auf das Halteelement (2) und damit den zweiten Körper (3) gekippt werden. Eine solche Kippmontage ist vorteilhaft gegenüber einer schiebenden Montage, da auf diese Weise keine Reibung zwischen Membran (1) und dem ersten Körper (5) entsteht. Damit besteht keine Beschädigungsgefahr oder Beeinflussung der mechanischen Eigenschaften beispielsweise einer Membran-Messwertaufnehmer-Verbindung, wenn der ersten Körper (5) einen Messwertaufnehmer umfasst oder einen solchen definiert.

In einigen Ausführungsformen weist das scheibenförmige Halteelement (2) ein Element auf, das dem Benutzer die Montage erleichtern kann. Dies trägt dazu bei, zuverlässig sicherzustellen, dass Beschädigungen des Halteelements und des Verbindungselements vermieden werden. So kann das scheibenförmige Halteelement (2) ein Element aufweisen, das Fehlstellungen bei der Montage vermeidet. So zeigen Fig. 3 und Fig. 5A eine umlaufende Führungskante (8). Andere Bauteile des Verbindungssystems wie z.B. das lösbare Verbindungselement oder ggf. der zweite Körper können passgenaue Elemente aufweisen, die ein entsprechendes Element des scheibenförmigen Halteelements (2) kontaktieren können. Die in Fig. 3 und Fig. 5A gezeigte umlaufende Führungskante (8) hat ein auf den Flansch des ersten Körpers (5) abgestimmtes Design, das es erleichtert, das scheibenförmige Halteelement (2) auf den ersten Körpers (5) zu montieren. Durch das Kontaktieren zueinander passender Elemente kann nach dem Schlüssel-Schlüsselloch-Prinzip bzw. dem Prinzip des Poka Yoke verhindert werden, das unbeabsichtigte Fehler, beispielsweise bei Bewegungen oder beim Ausrichten von Bauteilen, zu Beschädigungen führen. Auf diese Weise kann auch zusätzlicher Halt geschaffen werden. Als weiteres Beispiel in dieser Hinsicht zeigt Fig. 17A einen konischen Führungssteg (52), der beim Zusammenführen des flanschartigen, scheibenförmigen Halteelements (2) mit dem Flansch (25) des ersten Körpers ein Arretieren bewirken kann. Der erste Körper (5) kann ein Element aufweisen, das geometrisch dazu angepasst ist, den konischen Führungssteg (52) zu kontaktieren und dadurch eine Selbstarretierung zu bewirken. Auf diese Weise kann verhindert werden, dass beim Zusammenführen der erste und der zweite Körper zueinander versetzt angeordnet sind.

Eine umlaufende Führungskante (8) und ein zweites, typischerweise kleineres Hakenelement (7) ermöglichen eine einfache, sichere und definierte Positionierung beispielsweise einer Messkammer auf einem Messwertaufnehmer. Das zweite Hakenelement (7) kann dabei als Führungssegment verstanden werden. Insbesondere wird die für die Kraftübertragung wichtige definierte Positionierung der Membran auf dem Kraftsensor (z.B. Piezo-Element) des Messwertaufnehmers sichergestellt.

In einigen Ausführungsformen weist das Verbindungselement (4) Paare sich gegenüberliegender Wände, Vorsprünge - inklusive Schenkeln - oder Auslassungen auf. Dabei können die sich gegenüberliegender Wände oder Vorsprünge eines Paars an Wänden bzw. Vorsprüngen gegeneinander eine Nachgiebigkeit aufweisen, die es ermöglicht, Bereiche des scheibenförmigen Halteelements (2) aufzunehmen. Beispielsweise kann das Verbindungselement (4) zwei übereinander liegende Paare Schenkel aufweisen, wobei jedes Schenkelpaar eine Hälfte einer U-förmigen Klammer definiert. Die beiden Schenkel eines solchen Schenkelpaars können so gestaltet sein, dass Arme des scheibenförmigen Halteelements (2) zwischen die Schenkel eines Schenkelpaars der U-förmigen Klammer geschoben werden können. Dieser Vorgang des Einschiebens kann ein Nachgeben der Schenkel des Verbindungselements bedingen.

In einigen Ausführungsformen ist das Verbindungselement (4) als U-förmige Klammer ausgebildet, wobei die U-Form durch zwei Schenkel (31) definiert ist, die ebenfalls als Schenkelpaar bezeichnet werden können. In diesem Fall liegen die beiden Schenkel an einander gegenüberliegenden Seiten des scheibenförmigen Halteelements (2) an, typischerweise an gegenüberliegenden Seiten des peripheren Außenrands desselben. Eine solche Ausführungsform ist unter anderem in Figur 6 gezeigt. In dieser Ausführungsform weist jeder Schenkel (31) des Verbindungselements (4) eine Seitenwand (28) auf. Die beiden Seitenwände (28) liegen an jeweils gegenüberliegenden Seiten eines peripheren Außenrands (24) des Halteelements (2) an. Die Seitenwände (28) der beiden Schenkel (31) ermöglichen ein Umgreifen sowohl des scheibenförmigen Halteelements (2) als auch des Flansches (25). Eine Einlaufkante (15), die sowohl konkav als auch konvex sein kann, erleichtert dabei die Montage auf dem ersten Körper (5).

Das scheibenförmige Halteelement (2) und/oder das Verbindungselement (4) können Merkmale aufweisen, die es ermöglichen, das Verbindungselement (4) relativ zum Halteelement (2) in einer zumindest teilweise kontrollierten Weise zu bewegen. Entsprechende Merkmale können analog einer Schiene eine Führung für eine Bewegung des Verbindungselements (4) vorgeben. Diesem Zweck kann beispielsweise eine Kombination von einem oder mehreren Vorsprüngen - inklusive Schenkeln - auf dem scheibenförmigen Halteelement (2) und einem oder mehreren Aussparungen des Verbindungselements (4) dienen. Ebenso kann eine Kombination von einem oder mehreren Vorsprüngen auf dem Verbindungselement (4) und eine oder mehrere Aussparungen des scheibenförmigen Halteelements (2) vorgesehen sein. Beispielsweise kann das Verbindungselement (4) eine umlaufende Wand aufweisen, die zumindest ein Segment des Umfangs des Verbindungselements (4) umläuft. Je nach Form des Verbindungselements kann die umlaufende Wand eine einheitliche oder eine uneinheitliche Dicke aufweisen. Typischerweise ist die Form dieser umlaufenden Wand so an die Form des peripheren Außenrands des scheibenförmigen Halteelements (2) angepasst, dass eine oder mehrere Kontaktflächen von Verbindungselement (4) und scheibenförmigem Halteelement (2) aneinander anliegen können. In einigen Ausfiihrungsformen ist ein Formschluss von scheibenförmigem Halteelement (2) und Verbindungselement (4) möglich. Die umlaufende Wand des Verbindungselements (4) kann die Form eines Halbkreises oder eines Halbovals aufweisen. Die umlaufende Wand des Verbindungselements (4) kann auch V-förmig, U-förmig oder M-förmig sein. Die umlaufende Wand des Verbindungselements kann in einigen ausführungsformen die Form eines offenen Rechtecks oder eines offenen Quadrats aufweisen. Die Figuren 6 und 8 zeigen Ausführungsformen, in denen eine umlaufende Wand (28) des Verbindungselements (4) U-förmig ist.

In den in den Figuren 6 und 8 gezeigten Ausführungsformen weist die umlaufende Wand (28) des Verbindungselements (4) eine Mehrzahl, in diesem Fall zwei, axial gegeneinander versetzte Aussparungen auf. Die Dimensionen der Aussparungen (13) erlauben es, Arme (10) eines scheibenförmigen Halteelements (2) aufzunehmen. Die Dimensionen derartiger Aussparungen können es auch erlauben, für Vorsprünge wie Arme (10) eines scheibenförmigen Halteelements (2) zwei verschiedene Positionen vorzusehen. Auf diese Weise können beispielsweise entsprechend axial gegeneinander versetzte Vorsprünge des scheibenförmigen Halteelements (2) in Form eines Bajonettverschlusses in Aussparungen (13) des Verbindungselements (4) gehalten werden. Die umlaufende Wand (28) kann beispielsweise auch ein Paar axial einander gegenüberliegender Aussparungen oder drei axial gegeneinander versetzter Aussparungen aufweisen. Die Form der Aussparungen kann so gewählt sein, dass durch eine Drehbewegung des Verbindungselements (4) ein Fixieren der Vorsprünge des scheibenförmigen Halteelements (2) in Form eines Bajonettverschlusses erfolgen kann.

Zur Unterstützung und/oder Erleichterung einer Bewegung des Verbindungselements (4) relativ zum scheibenförmigen Halteelement (2) kann ein Griffelement (12) vorgesehen sein. In den in den Figuren 6 und 8 gezeigten Ausführungsformen ist ein Griffelement (12) zwischen den Aussparungen (13) angeordnet.

Das scheibenförmige Halteelement (2), das beispielsweise zwei Arme (10) aufweisen kann, kann als Arretierung einen Vorsprung (11) aufweisen, durch den das Halteelement (2) im Verbindungselement (4), das beispielsweise die Form einer Klammer haben kann, in einer Montageposition gehalten wird. Somit ist eine Vormontage von Halteelement (2) und Verbindungselement (4) möglich. Dadurch ist es möglich, mit einer Daumenbewegung am Griffelement (12) das Verbindungselement (4) auf dem scheibenförmigen Halteelement (2) zu verschieben, ohne dass sich die Klammer des Verbindungselements (4) vom Halteelement (2) löst. Per Daumenbewegung kann das Verbindungselement (4) am Griffelement (12) über das scheibenförmige Halteelement (2) und den Flansch (25) des ersten Körpers verschoben werden, so dass beide fest miteinander verpresst werden.

Ein wie in den Figuren 6, 8, 10 bis 12 gezeigtes Griffelement (12) des Verbindungselements (4) kann nach Gebrauch (oder bei Fehlmontage) auch zur Trennung der Verbindung dienen, wobei das Klammer-förmige Verbindungselement (4) wieder vom scheibenförmigen Halteelement (2) gezogen wird. Zur Sicherheit kann eine definierte Endposition des Verbindungselements (4) auf dem Halteelement (2) kenntlich gemacht werden. Des Weiteren kann übe eine Verklemmung (17), beispielsweise in Form eines Stegs, inklusive eines Paars Stege, eine Sicherung der Montageposition erfolgen. Ein Paar Stege, beispielsweise in einer Gestaltung, die der Form von Kreissegmenten entspricht, kann in eine Ausführungsform in einer Endposition in eine formpassende Aussparung (16) eines Verbindungselements (4) eingeklemmt werden. Auf diese Weise kann eine definierte Verklemmung und damit Betriebssicherheit sichergestellt werden.

Diese Art des Verbindungs-, z.B. Klemm-, Mechanismus weist eine Zwischenposition auf, in der das Halteelement (2) und das Verbindungselement (4) bereits verbunden sind, jedoch noch keine Verpressung mit dem ersten Körper (5) erfolgt ist. Dies ermöglicht eine Bewegbarkeit, inklusive Drehbarkeit, des ersten Körpers auf dem zweiten Körper. Dadurch wird beispielsweise die Montage einer Messkammer mit den typischerweise angeschlossenen Schläuchen und die Ausrichtung der Schläuche erleichtert. Fertig ausgerichtet kann die Klemmung dann in die definierte Endposition gedrückt und verriegelt werden.

Als weiteres Beispiel kann das scheibenförmige Halteelement (2), wie in Fig. 9 gezeigt, einen Einschübling (37) enthalten, der wie in der gezeigten Ausführungsform beispielsweise zwischen Armen (10) des Halteelements (2) angeordnet sein kann. Das Verbindungselement (4) kann eine Aussparung wie beispielsweise eine durchgehende Öffnung (21) aufweisen, die den Einschübling (37) aufnehmen kann, siehe Fig. 8. Der Einschübling (37) kann so weit nachgebend sein, dass sich das Verbindungselement (4) ggf. über den Einschübling hinweg schieben lässt. Wie in Figur 10 gezeigt, kann auf diese Weise eine Arretierung des Verbindungselements (4) in einem Zustand erfolgen. Figur 10B zeigt einen offenen Zustand, in dem das Verbindungselement (4) eine schräg nach vorne zum Betrachter hin verschobene Position einnimmt. Durch Druck auf das Griffelement (12) des Verbindungselements (4) kann das Verbindungselement (4) in Richtung vom Betrachter weg schräg nach hinten geschoben werden. Figur 10A zeigt einen geschlossenen Zustand, in dem der Einschübling (37) in der Aussparung (21) des Verbindungselements (4) gehalten wird.

Auch kann auf dem scheibenförmigen Halteelement (2) ein in Richtung des zweiten Körpers weisender Vorsprung, ein Steg oder ähnliches vorgesehen sein. Eine entsprechende Aussparung, Vertiefung oder Nut auf dem Verbindungselement (4) kann so ausgebildet sein, dass sie den Vorsprung, Steg etc. auf dem scheibenförmigen Halteelement (2) aufnehmen kann. Figur 5 zeigt eine auf dem scheibenförmigen Halteelement (2) angeordnete Verklemmung (17). Eine von den Dimensionen her angepasste Aussparung (16) auf dem Verbindungselements (4) ist in Fig. 6 gezeigt. Figuren 10D und 10 E zeigen den offenen Zustand eines entsprechenden Verbindungssystems und das Ineinanderpassen der Elemente (17) und (16) im geschlossenen Zustand (Fig. 10E).

Als weiteres Beispiel zeigen Fig. 11B, Fig. 11C, Fig. 11F und Fig. 12 einen auf dem scheibenförmigen Halteelement (2) angeordneten Bügel (47). Ein beispielsweise als Steg ausgebildeter Halter (45) kann auf dem Verbindungselement (4) vorgesehen sein, wie in den Figuren 11C, 11F und 12 gezeigt. Der Bügel (47) kann so gestaltet sein, dass er in der Lage ist, den Halter (45) zumindest teilweise zu umfassen und damit zu arretieren. Dabei kann der Bügel (47) so weit nachgebend sein, dass es möglich ist, den Halter (45) unter den Bügel (47) zu schieben. Fig. 12A zeigt ein entsprechendes Verbindungssystem im offenen Zustand, in dem das Verbindungselement (4) durch Druck auf das Griffelement (12) des Verbindungselements (4) in Richtung vom Betrachter weg schräg nach hinten geschoben werden kann. Der entsprechende geschlossene Zustand, in dem der Bügel (47) den Halter (45) teilweise umgreift, ist in Fig. 12B gezeigt.

In der in Fig. 13 gezeigten Ausführungsform erfolgt die Verriegelung durch Drehen des Verbindungselements (4), das die Form eines Montagerings hat. Der zu Grunde liegende Mechanismus kann als Variante eines Bajonettverschlusses verstanden werden. Hier wird die Verpressung durch Drehbewegung des Montagerings (4), ggf. mit Hinterschnitt-Elementen, über den Flansch (25) des ersten Körpers (5) sichergestellt. Auch bei dieser Ausführungsform ist eine Einhandmontage möglich.

Fig. 14 zeigt eine Verpressung durch zwei Spannhebel als Verbindungselement (4). Ein oder mehr weitere Spannhebel können vorgesehen werden. Wiederum ist eine Einhandmontage möglich.

Fig. 16 zeigt eine Ausführungsform, in der das Verbindungselement (4) einen schwenkbaren Arm (30) und einen nietenförmigen Vorsprung (42) aufweist. Der schwenkbare Arm kann dabei, wie in der Figur gezeigt, ein Element aufweisen, das geometrisch dazu angepasst ist, im nietenförmigen Vorsprung (42) lösbar fixiert zu werden. Dabei kann es sich beispielsweise um eine Riffelung, um einen oder mehrere Zapfen, einen oder mehrere Rippen oder einen oder mehrere Stege handeln. Derartige geometrische Elemente, die als Verzahnung dienen können, können auf einem Fortsatz des Arms wie z.B. einer Zunge (49) oder einem Band vorgesehen sein. Der nietenförmige Vorsprung (42) kann ein geometrisches Element aufweisen, dass dazu angepasst ist, das am schwenkbaren Arm (30) vorgesehene geometrische Element zu kontaktieren. Dabei kann ein Ineinandergreifen oder Einrasten ähnlich einem Kabelverbinder erfolgen. Beispielsweise kann im nietenförmigen Vorsprung (42) ein Keil oder ein Dorn angebracht sein, der auf eine Riffelung oder einen Zapfen auf der Zunge (49) passt und eine Arretierung bewirkt. Auf diese Weise kann beispielsweise verhindert werden, dass die Zunge (49) ohne äußere Einwirkung den nietenförmigen Vorsprung (42) verlassen kann.

Die Arretierung des in Fig. 16 gezeigten Arms (30) in den nietenförmigen Vorsprung (42) lässt sich den Vergrößerungen von Fig. 16I (arretierter Zustand) und Fig. 16E (nicht- arretierter Zustand) entnehmen. Die Zunge (49) des Arms (30) geht eine Verzahnung mit einer Zahnstange (53) ein, die im nietenförmigen Vorsprung (42) vorgesehen ist. Auf diese Weise wird verhindert, dass sich der Arm (30) ohne äußeren Eingriff vom scheibenförmigen Halteelement (2) löst. Die Zahnstange (53) weist an ihrem unteren Ende, also am dem ersten Körper (5) zugewandten Ende, einen Zapfen (56) auf, über den sie an ein Griffelement (54) gekoppelt ist. Der Zapfen (56) dient als Angriffspunkt für eine auf das Griffelement (54) einwirkende Kraft. Durch seitlichen Druck auf das Griffelement (54) kann die Verzahnung mit der Zunge (49) gelöst werden, so dass sich der Arm (30) vom scheibenförmigen Halteelement (2) lösen kann. In einigen Ausführungsform besteht das Verbindungselement (4) im Wesentlichen aus einen schwenkbaren Arm (30) und einem nietenförmigen Vorsprung (42), wie etwa in Fig. 16 gezeigt. Wie in Fig. 16 gezeigt, kann das Verbindungselement (4) ausschließlich aus einen schwenkbaren Arm (30) und einem nietenförmigen Vorsprung (42) bestehen.

Ein Verbindungssystem gemäß der vorliegenden Offenbarung kann eine Steril-Barriere aufweisen, um Kontaminationen vor und ggf. während der Montage zu vermeiden. Eine derartige Steril-Barriere kann ein entfernbares Element aufweisen oder aus einem solchen Element bestehen, das den Innenraum eines Körpers verschließt. Eine derartige Steril-Barriere kann auch ein nichtentfernbares Element aufweisen oder aus einem nicht-entfernbaren Element bestehen, das den Innenraum eines Körpers verschließt. Dies kann insbesondere dazu dienen, den Innenraum eines fluidführenden Körpers steril zu halten. In einigen Ausführungsformen bedeckt eine entfernbare Absperrung wie beispielsweise ein Film, eine Mündung eines Innenraums des ersten und/oder des zweiten Körpers. Bei einer solchen Absperrung kann es sich z.B. um eine Schutzfolie oder eine Membran handeln.

So kann beispielsweise ein wie in den Figuren 17 und 18 gezeigtes Verbindungssystem zum Koppeln zweier fluidführender Körper mit einem Film ausgestattet sein. Figuren 17 und 18 zeigen beispielhaft lösbare Schutzfolien (48), die dazu angepasst ist, den Innenraum des ersten und des zweiten Körpers von der Umgebung zu isolieren. Eine zentralen Öffnung (55) des Innenraums des zweiten fluidführenden Körpers (3) ist in Fig. 17A von einem umlaufenden Vorsprung (46) umgeben. Diesen umlaufenden Vorsprung (46) kontaktiert umlaufend eine lösbare Schutzfolie (48). Werden der erste und der zweite Körper in dieser Ausführungsform durch Schwenken, siehe das Scharnier (33), in eine Position gebracht, in der der Flansch (25) des ersten Körpers und das flanschartige, scheibenförmige Halteelement (2) des zweiten Körpers flächig aneinander anliegen, können die Schutzfolien (48) entfernt werden. Insbesondere nach Arretieren durch den Haken (39) kann durch den umlaufenden Vorsprung (46) eine sterile Verbindung der Innenräume des ersten und des zweiten Körpers sichergestellt werden. In einer so konnektierten Stellung können daher die Schutzfolien (48) entfernt werden. Figuren 18C bis 18H zeigen das Verbindungssystem nach Entfernen der Schutzfolien (48).

## Patentansprüche

1. Verbindungssystem zum lösbar kraftschlüssigen Koppeln eines ersten Körpers (5) an ein fluidführendes System über eine fluiddichte Membran (1),
wobei das Verbindungssystem die Membran (1) und einen zweiten Körper (3) mit einem Innenraum aufweist, der an das fluidführende System gekoppelt ist, und wobei der erste Körper (5) ein erstes Ende mit einem Flansch (25) aufweist,
wobei der zweite Körper (3) ein zweites Ende mit einem Innenraum (23), einer umlaufenden Wand (22) und einer Öffnung (43) aufweist, wobei die umlaufende Wand (22) einen um die Öffnung (43) umlaufenden Membrankontaktabschnitt (9) aufweist, der mit der Membran (1) in Kontakt ist,
**dadurch gekennzeichnet, dass**
(a) das Verbindungssystem ein scheibenförmiges Halteelement (2) mit einem Durchlass (35), einer ersten Seite, einer zweiten Seite und einem peripheren Außenrand (24) aufweist, wobei die erste Seite und/oder der Durchlass (35) des Halteelements (2) an die Membran (1) gekoppelt ist/sind und wobei die zweite Seite des Halteelements (2) für einen flächigen Kontakt mit dem Flansch (25) des ersten Körpers (5) ausgelegt ist, oder
(b) das zweite Ende des zweiten Körpers (3) ein flanschartiges, scheibenförmiges Halteelement (2) mit einem peripheren Außenrand (24) definiert, wobei das flanschartige, scheibenförmige Halteelement (2) mit der Membran (1) in Kontakt ist und für einen flächigen Kontakt mit dem Flansch (25) des ersten Körpers (5) ausgelegt ist und wobei der erste Körper fluidführend ist, und
wobei das Verbindungssystem in (a) und (b) weiterhin ein lösbares Verbindungselement (4) aufweist, das dazu ausgelegt ist, den peripheren Außenrand (24) des Halteelements (2) und den Flansch (25) des ersten Körpers (5) zumindest teilweise zu umgreifen, sodass das Halteelement (2) mit Hilfe des Verbindungselements (4) kraftschlüssig am ersten Körper (5) fixierbar ist.

2. Verbindungssystem nach Anspruch 1, wobei das Verbindungssystem ein scheibenförmiges Halteelement (2) mit einem Durchlass (35), einer ersten Seite, einer zweiten Seite und einem peripheren Außenrand (24) aufweist,
**dadurch gekennzeichnet,**
**dass** a) auf der ersten Seite des Halteelements (2) eine umlaufende Wand (26) angeordnet ist, die mit der umlaufenden Wand (22) des zweiten Körpers (3) verbunden ist, und/oder b) das scheibenförmige Halteelement (2) und das Verbindungselement (4) arretierbar ineinandergreifen.

3. Verbindungssystem nach einem der vorhergehenden Ansprüche, **dadurch gekennzeichnet, dass**
(a) der um die Öffnung (43) umlaufenden Membrankontaktabschnitt (9) mit der Membran (1) verbunden ist; und/oder
(b) die erste Seite und/oder der Durchlass (35) des scheibenförmigen Halteelements (2) mit der Membran (1) verbunden ist/sind.

4. Verbindungssystem nach einem der vorhergehenden Ansprüche, **dadurch gekennzeichnet, dass** der erste Körper (5) ein Messwertaufnehmer ist und der zweite Körper (3) eine mit einem Fluidsystem durchströmbar verbindbare Messkammer ist,
wobei die Membran (1) wesentlich nachgiebiger als die Wandungen des Innenraums des zweiten Körpers (3) ist und eine Übertragung von Druckkräften aus der Messkammer auf den Messwertaufnehmer (5) ermöglicht,
wobei die erste Seite und der Durchlass (35) des Halteelements (2) mit derMembran (1) in Kontakt sind,
wobei die erste Seite des Halteelements (2) an die Membran (1) und die umlaufende Wand (22) der Messkammer (3) gekoppelt ist und die zweite Seite für einen flächigen Kontakt mit dem Flansch (25) des Messwertaufnehmers (5) ausgelegt sind.

5. Verbindungssystem nach einem der vorhergehenden Ansprüche, **dadurch gekennzeichnet, dass**
(a) die Membran (1) mit der umlaufenden Wand (22) des zweiten Körpers (3) und/oder dem Halteelement (2) verschweißt, verklebt oderversiegelt, oder an die umlaufende Wand (22) angeklemmt ist und/oder durch Mehrkomponentenspritzguss und/oder Flüssig-Silikon oder durch eine Kombination daraus mit der umlaufenden Wand (22) verbunden ist; und/oder
(b) dass das Halteelement (2) mit der umlaufenden Wand (22) des zweiten Körpers verschweißt, verklebt oder versiegelt, oder an diesen angeklemmt ist und/oder durch Mehrkomponentenspritzguss und/oder Flüssig-Silikon oder durch eine Kombination daraus mit der umlaufenden Wand (22) des zweiten Körpers (3) verbunden ist.

6. Verbindungssystem nach einem der vorhergehenden Ansprüche, **dadurch gekennzeichnet, dass**
- das Halteelement (2) mindestens einen Arm (10) aufweist und wobei das Verbindungselement (4) eine im Wesentlichen U-förmige Klammer mit zwei Schenkeln (31) aufweist und wobei die im Wesentlichen U-förmige Klammer eine Öffnung oder einen Auslass aufweist, in die/den der Arm (10) aufgenommen ist und/oder
- das Verbindungselement (4) ein oder mehrere Spannhebel aufweist, die beweglich an das Halteelement (2) gekoppelt sind und dazu ausgelegt sind, den Flansch (25) des ersten Körpers (5) zumindest teilweise zu umgreifen und/oder
- das Verbindungselement (4) einen beweglich an das Halteelement (2) gekoppelten Arm (30) aufweist, der dazu ausgelegt ist, die Außenseite des Flansches (25) des ersten Körpers (5) und den peripheren Außenrand (24) des Halteelements (2) zumindest teilweise zu umgreifen und/oder
- das Verbindungselement (4) ein erstes Gewinde aufweist und das das Halteelement (2) ein zweites Gewinde aufweist, wobei das erste und das zweite Gewinde ineinander passen und verschraubt sind und/oder
- das Verbindungselement (4) eine umlaufende Wand aufweist, die den peripheren Außenrand (24) des Halteelements (2) umfasst, wobei die umlaufende Wand eine Mehrzahl axial gegeneinander versetzter Aussparungen aufweist und wobei der periphere Außenrand (24) des Halteelements (2) eine Mehrzahl axial gegeneinander versetzter Vorsprünge aufweist und wobei die Mehrzahl axial gegeneinander versetzter Vorsprünge in der Mehrzahl gegeneinander versetzter Aussparungen in Form eines Bajonettverschlusses gehalten werden.

7. Verbindungssystem zum lösbar kraftschlüssigen Koppeln des Innenraums eines ersten fluidführenden Körpers (5) mit dem Innenraum eines zweiten fluidführenden Körpers (3), wobei das Verbindungssystem den zweiten fluidführenden Körper (3) aufweist,
wobei der erste Körper (5) ein erstes Ende mit einem Flansch (25) und einem Auslass des Innenraums des ersten fluidführenden Körpers (5) aufweist und der zweite Körper (3) ein zweites Ende aufweist, das ein flanschartiges, scheibenförmiges Halteelement (2) definiert, das einen peripheren Außenrand (24) und eine zentralen Öffnung (55) des Innenraums des zweiten fluidführenden Körpers (3) aufweist,
wobei das Halteelement (2) an den Flansch (25) des ersten Körpers (5) so verbindbar gekoppelt ist, dass das Halteelement (2) auf dem Flansch (25) des ersten Körpers (5) aufliegen kann,
**dadurch gekennzeichnet,**
**dass** das Verbindungssystem weiterhin ein Verbindungselement (4) aufweist, das dazu ausgelegt ist, den peripheren Außenrand (24) des Halteelements (2) und den Flansch (25) des ersten Körpers (5) zumindest teilweise zu umgreifen, so dass das Halteelement (2) mit Hilfe eines lösbaren Verbindungselements (4) kraftschlüssig am ersten Körper (5) fixiert ist,
wobei das Halteelement (2) mindestens einen Arm (10) aufweist und wobei das Verbindungselement (4) eine im Wesentlichen U-förmige Klammer mit zwei Schenkeln (31) aufweist und wobei die im Wesentlichen U-förmige Klammer eine Aussparung (13) aufweist, in die der Arm (10) einschiebbar ist.

8. Verbindungssystem nach Anspruch 7, **dadurch gekennzeichnet, dass** das Halteelement (2) eine erste Seite und eine zweite Seite aufweist, wobei die erste Seite des Halteelements (2) mit dem Flansch (25) des ersten Körpers (5) in Kontakt ist und wobei auf der ersten Seite des Halteelements (2) ein umlaufender Vorsprung (46) angeordnet ist.

9. Verbindungssystem nach einem der Ansprüche 6 oder 7, weiterhin eine Membran (1) aufweisend, die zwischen dem Flansch (25) des ersten Körpers (5) und der ersten Seite des Halteelements (2) des zweiten Körpers (3) angeordnet ist.

10. Verbindungssystem nach einem der Ansprüche 7 bis 9, **dadurch gekennzeichnet, dass** der erste fluidführende Körper (5) und der zweite fluidführende Körper (3) verschwenkbar miteinander verbunden sind.

11. Verbindungssystem nach einem der vorhergehenden Ansprüche, **dadurch gekennzeichnet, dass** die zweite Seite des Halteelements (2) ein peripheres Hakenelement (7) aufweist, wobei das Hakenelement (7) dazu angepasst ist, die Außenseite des Flansches (25) des ersten Körpers (5) zumindest teilweise zu umgreifen.

12. Verbindungssystem nach einem der vorhergehenden Ansprüche, **dadurch gekennzeichnet, dass** es
- ein Verbindungselement (4) mit einem oder mehr Spannhebeln und ein Halteelement (2) mit mindestens einem Hakenelement (7) und/oder
- ein Verbindungselement (4) mit einer oder mehr Schnappverbindungen und ein Halteelement (2) mit einem oder mehr Hakenelementen (7) und/oder
- ein Verbindungselement (4) mit zwei oder mehr Spannhebeln und/oder
- ein Verbindungselement (4) mit einer im Wesentlichen U-förmigen Klammer und ein Halteelement (2) mit einem oder mehr Hakenelementen (7)
aufweist.

13. Verbindungssystem nach einem der vorhergehenden Ansprüche, **dadurch gekennzeichnet, dass** das Verbindungselement (4) durch eine im Wesentlichen U-förmige Klammer mit einem oberen Paar Schenkel, einem unteren Paar Schenkel und einer jeden oberen und unteren Schenkel verbindenden Seitenwand (28) definiert ist, wobei die Seitenwand (28) auf der Seite jedes Schenkels eine Aussparung (13) aufweist,
wobei das Halteelement (2) ein Paar Arme (10) aufweist, die parallel zur Ebene des Flansches (25) angeordnet sind,
wobei das Halteelement (2) zwischen dem oberen und dem unteren Element der im Wesentlichen U-förmigen Klammer (6) so angeordnet ist, dass die Enden der Arme (10) in die Öffnungen (13) einschiebbar sind.

14. Verfahren zum lösbar kraftschlüssigen Koppeln a) eines ersten Körpers (5) an ein fluidführendes System über eine fluiddichte Membran (1) mit Hilfe eines lösbaren Verbindungselements (4), oder b) des Innenraums eines ersten fluid-führenden Körpers (5) mit dem Innenraum (23) eines zweiten fluidführenden Körpers (3) mit Hilfe eines lösbaren Verbindungselements (4), wobei der erste Körper (5) ein erstes Ende mit einem Flansch (25) aufweist und sich im ersten Ende des ersten Körpers (5) weiterhin ein Auslass des Innenraums des ersten Körpers befindet,
wobei in a) der erste Körper (5) ein erstes Ende mit einem Flansch (25) aufweist und wobei an das fluidführende System ein Innenraum (23) eines zweiten Körpers (3) gekoppelt ist,
wobei in a) der zweite Körper (3) ein zweites Ende mit einer umlaufenden Wand (22), sowie den Innenraum (23) mit einer Öffnung (43) desselben aufweist, wobei die umlaufende Wand (22) einen um die Öffnung (43) umlaufenden Membrankontaktabschnitt (9) aufweist, der mit der Membran (1) in Kontakt ist,
**dadurch gekennzeichnet, dass** in a) das zweite Ende des zweiten Körpers (3) mit einem scheibenförmigen Halteelement (2) in Kontakt ist, das ein eigenständiges Bauteil definiert, wobei das scheibenförmige Halteelement (2) einen Durchlass (35), eine erste Seite, eine zweite Seite und einen peripheren Außenrand (24) aufweist, wobei die erste Seite des Halteelements (2) mit dem ersten Ende des ersten Körpers (5) in Kontakt ist und die erste Seite und/oder der Durchlass (35) des scheibenförmigen Halteelements (2) weiterhin an die Membran (1) gekoppelt ist, und wobei die zweite Seite des Halteelements (2) für einen flächigen Kontakt mit dem Flansch (25) des ersten Körpers (5) ausgelegt ist,
wobei das Verfahren in a) aufweist:
das am zweiten Ende des zweiten Körpers (3 befindliche scheibenförmige Halteelement (2) und das erste Ende des ersten Körpers (5) so ausrichten und in Kontakt bringen, dass die zweite Seite des scheibenförmigen Halteelements (2) auf dem Flansch (25) des ersten Körpers (5) aufliegt, und
anschließend in (a) das lösbare Verbindungselement (4) so anordnen, dass es den Flansch (25) des ersten Körpers (5) und den peripheren Außenrand (24) des scheibenförmigen Halteelements zumindest teilweise umgreift, sodass das Halteelement (2) mit Hilfe des Verbindungselements (4) kraftschlüssig am ersten Körper (5) reversibel fixiert wird, oder
**dadurch gekennzeichnet, dass** in b) der zweite fluidführende Körper (3) ein zweites Ende aufweist, das ein flanschartiges, scheibenförmiges Halteelement (2) mit einem peripheren Außenrand (24) und einer zentralen Öffnung (43) definiert, wobei die zentrale Öffnung eine Öffnung des Innenraums (23) des zweiten fluidführenden Körpers (3) ist,
wobei das flanschartige, scheibenförmige Halteelement (2) weiterhin einen oder mehrere Arme (10) aufweist,
wobei das Verfahren in b) aufweist:
das flanschartige, scheibenförmige Halteelement (2) verbindbar so an den Flansch (25) des ersten Körpers (5) koppeln, dass das Halteelement (2) auf dem Flansch (25) des ersten Körpers (5) aufliegt,
das lösbare Verbindungselement (4) anschließend in b) so auf das flanschartige, scheibenförmige Halteelement (2) und den Flansch (25) des ersten Körpers (5) platzieren, dass es den peripheren Außenrand (24) des flanschartigen, scheibenförmigen Halteelements (2) und den Flansch (25) des ersten Körpers (5) zumindest teilweise umgreift, sodass das flanschartige, scheibenförmige Halteelement (2) mit Hilfe des Verbindungselements (4) kraftschlüssig am ersten Körper (5) reversibel fixiert wird.

## Claims

1. Connection system for releasably force-fittingly coupling a first body (5) to a fluid-carrying system via a fluid-tight membrane (1),
wherein the connection system comprises the membrane (1) and a second body (3) with an interior space which is coupled to the fluid-carrying system, and wherein the first body (5) comprises a first end with a flange (25),
wherein the second body (3) comprises a second end with an interior space (23), a circumferential wall (22) and an opening (43), wherein the circumferential wall (22) comprises a membrane contact portion (9) which extends around the opening (43) and which is in contact with the membrane (1),
**characterized in that**
(a) the connection system comprises a disk-shaped holding member (2) with a passage (35), a first side, a second side and a peripheral outer edge (24), wherein the first side and/or the passage (35) of the holding member (2) is/are coupled to the membrane (1), and wherein the second side of the holding member (2) is designed for surface-to-surface contact with the flange (25) of the first body (5), or
(b) the second end of the second body (3) defines a flange-like, disk-shaped holding member (2) having a peripheral outer edge (24), wherein the flange-like, disk-shaped holding member (2) is in contact with the membrane (1) and is designed for surface-to-surface contact with the flange (25) of the first body (5), and wherein the first body is fluid-carrying, and
wherein the connection system in (a) and (b) further comprises a releasable connection member (4) which is designed to at least partially engage around the peripheral outer edge (24) of the holding member (2) and the flange (25) of the first body (5), such that the holding member (2) can be releasably force-fittingly fixed to the first body (5) by means of the connection member (4).

2. Connection system according to claim 1, wherein the connection system comprises a disk-shaped holding member (2) with a passage (35), a first side, a second side and a peripheral outer edge (24),
**characterized in that**
a) on the first side of the holding member (2) a circumferential wall (26) is arranged, which is connected to the circumferential wall (22) of the second body (3), and/or
b) the disk-shaped holding member (2) and the connection member (4) lockably engage each other.

3. Connection system according to any one of the preceding claims, **characterized in that**
(a) the membrane contact portion (9) which extends around the opening (43) is connected to the membrane (1); and/or
(b) the first side and/or the passage (35) of the disk-shaped holding member (2) is/are connected to the membrane (1).

4. Connection system according to any one of the preceding claims, **characterized in that** the first body (5) is a measuring element and the second body (3) is a measuring chamber connectable to a fluid system such that passage of fluid is allowed through the connection,
wherein the membrane (1) is substantially more flexible than the walls of the interior space of the second body (3) and allows a transmission of compressive forces from the measuring chamber to the measuring element (5),
wherein the first side and the passage (35) of the holding member (2) are in contact with the membrane (1),
wherein the first side of the holding member (2) is coupled to the membrane (1) and to the circumferential wall (22) of the measuring chamber (3), and the second side is designed for surface-to-surface contact with the flange (25) of the measuring element (5).

5. Connection system according to any one of the preceding claims, **characterized in that**
(a) the membrane (1) is welded, glued or sealed to the circumferential wall (22) of the second body (3) and/or to the holding member (2), or is clamped to the circumferential wall (22), and/or is connected to the circumferential wall (22) by means of multi-component injection molding and/or liquid silicone or by a combination thereof; and/or
(b) the holding member (2) is welded, glued or sealed to the circumferential wall (22) of the second body, or is clamped thereto, and/or is connected to the circumferential wall (22) of the second body (3) by means of multi-component injection molding and/or liquid silicone or by a combination thereof.

6. Connection system according to any one of the preceding claims, **characterized in that**
- the holding member (2) comprises at least one arm (10), and wherein the connection member (4) comprises a substantially U-shaped clamp with two legs (31), and wherein the substantially U-shaped clamp comprises an opening or an outlet, in which the arm (10) is received, and/or
- the connection member (4) comprises one or more clamping levers which are movably coupled to the holding member (2) and are designed to at least partially engage around the flange (25) of the first body (5) and/or
- the connection member (4) comprises an arm (30), which is movably coupled to the holding member (2) and is designed to at least partially engage around the outer side of the flange (25) of the first body (5) and the peripheral outer edge (24) of the holding member (2) and/or
- the connection member (4) comprises a first thread and the holding member (2) comprises a second thread, wherein the first and the second thread fit into one another and are screwed together and/or
- the connection member (4) comprises a circumferential wall which encompasses the peripheral outer edge (24) of the holding member (2), wherein the circumferential wall comprises a plurality of axially offset recesses, and wherein the peripheral outer edge (24) of the holding member (2) comprises a plurality of axially offset protrusions, and wherein the plurality of axially offset protrusions are held in the plurality of offset recesses in the form of a bayonet closure.

7. Connection system for releasably force-fittingly coupling the interior space of a first fluid-carrying body (5) to the interior space of a second fluid-carrying body (3),
wherein the connection system comprises the second fluid-carrying body (3),
wherein the first body (5) comprises a first end with a flange (25) and an outlet of the interior space of the first fluid-carrying body (5), and the second body (3) comprises a second end which defines a flange-like, disk-shaped holding member (2) that comprises a peripheral outer edge (24) and a central opening (55) of the interior space of the second fluid-carrying body (3),
wherein the holding member (2) is connectably coupled to the flange (25) of the first body (5) in such a way that the holding member (2) can rest on the flange (25) of the first body (5),
**characterized in that**
the connection system further comprises a connection member (4) which is designed to at least partially engage around the peripheral outer edge (24) of the holding member (2) and the flange (25) of the first body (5), such that the holding member (2) is force-fittingly fixed to the first body (5) by means of a releasable connection member (4),
wherein the holding member (2) comprises at least one arm (10), and wherein the connection member (4) comprises a substantially U-shaped clamp with two legs (31), and wherein the substantially U-shaped clamp comprises a recess (13), into which the arm (10) can be slid.

8. Connection system according to claim 7, **characterized in that** the holding member (2) comprises a first side and a second side, wherein the first side of the holding member (2) is in contact with the flange (25) of the first body (5), and wherein a circumferential protrusion (46) is arranged on the first side of the holding member (2).

9. Connection system according to any one of claims 6 or 7, further comprising a membrane (1) which is arranged between the flange (25) of the first body (5) and the first side of the holding member (2) of the second body (3).

10. Connection system according to any one of claims 7 to 9, **characterized in that** the first fluid-carrying body (5) and the second fluid-carrying body (3) are pivotably connected to each other.

11. Connection system according to any one of the preceding claims, **characterized in that** the second side of the holding member (2) comprises a peripheral hook element (7), wherein the hook element (7) is adapted to at least partially engage around the outer side of the flange (25) of the first body (5).

12. Connection system according to any one of the preceding claims, **characterized in that** it comprises
- a connection member (4) with one or more clamping levers and a holding member (2) with at least one hook element (7) and/or
- a connection member (4) with one or more snap connections and a holding member (2) with one or more hook elements (7) and/or
- a connection member (4) with two or more clamping levers and/or
- a connection member (4) with a substantially U-shaped clamp and a holding member (2) with one or more hook elements (7) .

13. Connection system according to any one of the preceding claims, **characterized in that** the connection member (4) is defined by a substantially U-shaped clamp having an upper pair of legs, a lower pair of legs and a side wall (28) connecting each upper and lower leg, wherein the side wall (28) comprises a recess (13) on the side of each leg,
wherein the holding member (2) comprises a pair of arms (10) which are arranged parallel to the plane of the flange (25),
wherein the holding member (2) is arranged between the upper and the lower element of the substantially U-shaped clamp (6) in such a way that the ends of the arms (10) can be slid into the openings (13).

14. Method for releasably force-fittingly coupling a) a first body (5) to a fluid-carrying system via a fluid-tight membrane (1) by means of a releasable connection member (4), or b) the interior space of a first fluid-carrying body (5) to the interior space (23) of a second fluid-carrying body (3) by means of a releasable connection member (4), wherein the first body (5) comprises a first end with a flange (25), and an outlet of the interior space of the first body is also located in the first end of the first body (5),
wherein in a) the first body (5) comprises a first end with a flange (25), and wherein an interior space (23) of a second body (3) is coupled to the fluid-carrying system,
wherein in a) the second body (3) comprises a second end with a circumferential wall (22) as well as the interior space (23) with an opening (43) thereof, wherein the circumferential wall (22) comprises a membrane contact portion (9) which extends around the opening (43) and which is in contact with the membrane (1),
**characterized in that** in a) the second end of the second body (3) is in contact with a disk-shaped holding member (2), which defines a separate component,
wherein the disk-shaped holding member (2) comprises a passage (35), a first side, a second side and a peripheral outer edge (24), wherein the first side of the holding member (2) is in contact with the first end of the first body (5), and the first side and/or the passage (35) of the disk-shaped holding member (2) is also coupled to the membrane (1), and wherein the second side of the holding member (2) is designed for surface-to-surface contact with the flange (25) of the first body (5),
wherein the method in a) comprises:
aligning and bringing into contact the disk-shaped holding member (2), which is located at the second end of the second body (3), and the first end of the first body (5) in such a way that the second side of the disk-shaped holding member (2) rests on the flange (25) of the first body (5), and
thereafter in (a) arranging the releasable connection member (4) in such a way that it at least partially engages around the flange (25) of the first body (5) and the peripheral outer edge (24) of the disk-shaped holding member, such that the holding member (2) is reversibly force-fittingly fixed to the first body (5) by means of the connection member (4), or
**characterized in that** in b) the second fluid-carrying body (3) comprises a second end which defines a flange-like, disk-shaped holding member (2) with a peripheral outer edge (24) and a central opening (43), wherein the central opening is an opening of the interior space (23) of the second fluid-carrying body (3),
wherein the flange-like, disk-shaped holding member (2) further comprises one or more arms (10),
wherein the method in b) comprises
connectably coupling the flange-like, disk-shaped holding member (2) to the flange (25) of the first body (5) in such a way that the holding member (2) rests on the flange (25) of the first body (5),
thereafter in b) placing the releasable connection member (4) on the flange-like, disk-shaped holding member (2) and the flange (25) of the first body (5) in such a way that the connection member at least partially engage around the peripheral outer edge (24) of the flange-like, disk-shaped holding member (2) and the flange (25) of the first body (5), such that the flange-like, disk-shaped holding member (2) is reversibly force-fittingly fixed to the first body (5) by means of the connection member (4).

## Revendications

1. Système de liaison pour l'accouplement à force libérable d'un premier corps (5) à un système conducteur de fluide par l'intermédiaire d'une membrane (1) étanche au fluide,
dans lequel le système de liaison présente la membrane (1) et un deuxième corps (3) avec un espace intérieur, qui est accouplé au système conducteur de fluide, et dans lequel le premier corps (5) présente une première extrémité avec une bride (25),
dans lequel le deuxième corps (3) présente une deuxième extrémité avec un espace intérieur (23), une paroi périphérique (22) et une ouverture (43), dans lequel la paroi périphérique (22) présente une section de contact de membrane (9) faisant le tour de l'ouverture (43), qui est en contact avec la membrane (1),
**caractérisé en ce que**
(a) le système de liaison présente un élément de retenue (2) en forme de disque avec un passage (35), un premier côté, un deuxième côté et un bord extérieur périphérique (24), dans lequel le premier côté et/ou le passage (35) de l'élément de retenue (2) est/sont accouplés à la membrane (1) et dans lequel le deuxième côté de l'élément de retenue (2) est configuré pour un contact à plat avec la bride (25) du premier corps (5), ou
(b) la deuxième extrémité du deuxième corps (3) définit un élément de retenue (2) en forme de disque du type bride avec un bord extérieur périphérique (24), dans lequel l'élément de retenue (2) en forme de disque du type bride est en contact avec la membrane (1) et est configuré pour un contact à plat avec la bride (25) du premier corps (5) et dans lequel le premier corps est conducteur de fluide, et
dans lequel le système de liaison dans (a) et (b) présente en outre un élément de liaison (4) détachable, qui est configuré pour entourer au moins en partie le bord extérieur périphérique (24) de l'élément de retenue (2) et la bride (25) du premier corps (5), de sorte que l'élément de retenue (2) peut être fixé à force sur le premier corps (5) à l'aide de l'élément de liaison (4) .

2. Système de liaison selon la revendication 1, dans lequel le système de liaison présente un élément de retenue (2) en forme de disque avec un passage (35), un premier côté, un deuxième côté et un bord extérieur périphérique (24),
**caractérisé en ce**
**que** a) sur le premier côté de l'élément de retenue (2) est disposée une paroi périphérique (26), qui est reliée à la paroi périphérique (22) du deuxième corps (3), et/ou b) l'élément de retenue (2) en forme de disque et l'élément de liaison (4) s'interpénètrent avec possibilité de blocage.

3. Système de liaison selon l'une quelconque des revendications précédentes, **caractérisé en ce que**
(a) la section de contact de membrane (9) faisant le tour de l'ouverture (43) est reliée à la membrane (1) ; et/ou
(b) le premier côté et/ou le passage (35) de l'élément de retenue (2) en forme de disque est/sont reliés à la membrane (1) .

4. Système de liaison selon l'une quelconque des revendications précédentes, **caractérisé en ce**
**que** le premier corps (5) est un transducteur et le deuxième corps (3) est une chambre de mesure pouvant être reliée à un système de fluide de manière à pouvoir être traversée par un écoulement,
dans lequel la membrane (1) est sensiblement plus flexible que les parois de l'espace intérieur du deuxième corps (3) et permet une transmission de forces de compression à partir de la chambre de mesure sur le transducteur (5),
dans lequel le premier côté et le passage (35) de l'élément de retenue (2) sont en contact avec la membrane (1),
dans lequel le premier côté de l'élément de retenue (2) est accouplé à la membrane (1) et la paroi périphérique (22) de la chambre de mesure (3) et le deuxième côté est configuré pour un contact à plat avec la bride (25) du transducteur (5).

5. Système de liaison selon l'une quelconque des revendications précédentes, **caractérisé en ce que**
(a) la membrane (1) est soudée, collée ou scellée à la paroi périphérique (22) du deuxième corps (3) et/ou l'élément de retenue (2), ou attachée à la paroi périphérique (22) avec des pinces et/ou est reliée à la paroi périphérique (22) par moulage par injection à plusieurs composants et/ou silicone liquide ou par une combinaison de ceux-ci ; et/ou
(b) que l'élément de retenue (2) est soudé, collé ou scellé à la paroi périphérique (22) du deuxième corps (3), ou attaché à celui-ci avec des pinces et/ou est relié à la paroi périphérique (22) du deuxième corps (3) par moulage par injection à plusieurs composants et/ou silicone liquide ou par une combinaison de ceux-ci.

6. Système de liaison selon l'une quelconque des revendications précédentes, **caractérisé en ce que**
- l'élément de retenue (2) présente au moins un bras (10) et dans lequel l'élément de liaison (4) présente une pince sensiblement en forme de U avec deux branches (31) et dans lequel la pince sensiblement en forme de U présente une ouverture ou une sortie, dans laquelle le bras (10) est reçu et/ou
- l'élément de liaison (4) présente un ou plusieurs leviers de serrage, qui sont accouplés de manière mobile à l'élément de retenue (2) et sont configurés pour entourer au moins en partie la bride (25) du premier corps (5) et/ou
- l'élément de liaison (4) présente un bras (30) accouplé de manière mobile à l'élément de retenue (2), qui est configuré pour entourer au moins en partie le côté extérieur de la bride (25) du premier corps (5) et le bord extérieur périphérique (24) de l'élément de retenue (2) et/ou
- l'élément de liaison (4) présente un premier filetage et l'élément de retenue (2) présente un deuxième filetage, dans lequel le premier et le deuxième filetage s'adaptent et sont vissés et/ou
- l'élément de liaison (4) présente une paroi périphérique, qui englobe le bord extérieur périphérique (24) de l'élément de retenue (2), dans lequel la paroi périphérique présente une pluralité d'évidements décalés les uns des autres axialement et dans lequel le bord périphérique extérieur (24) de l'élément de retenue (2) présente une pluralité de saillies décalées les unes des autres axialement et dans lequel la pluralité de saillies décalées les unes des autres axialement sont retenues dans la pluralité d'évidements décalés les uns des autres sous forme d'une fermeture à baïonnette.

7. Système de liaison pour l'accouplement à force libérable de l'espace intérieur d'un premier corps conducteur de fluide (5) à l'espace intérieur d'un deuxième corps conducteur de fluide (3),
dans lequel le système de liaison présente le deuxième corps conducteur de fluide (3),
dans lequel le premier corps (5) présente une première extrémité avec une bride (25) et une sortie de l'espace intérieur du premier corps conducteur de fluide (5) et le deuxième corps (3) présente une deuxième extrémité, qui définit un élément de retenue (2) en forme de disque du type bride, qui présente un bord extérieur périphérique (24) et une ouverture centrale (55) de l'espace intérieur du deuxième corps conducteur de fluide (3),
dans lequel l'élément de retenue (2) est accouplé à la bride (25) du premier corps (5) avec possibilité de liaison, de sorte que l'élément de retenue (2) peut reposer sur la bride (25) du premier corps (5),
**caractérisé en ce**
**que** le système de liaison présente en outre un élément de liaison (4), qui est configuré pour entourer au moins en partie le bord extérieur périphérique (24) de l'élément de retenue (2) et la bride (25) du premier corps (5), de sorte que l'élément de retenue (2) est fixé à force sur le premier corps (5) à l'aide d'un élément de liaison (4) détachable,
dans lequel l'élément de retenue (2) présente au moins un bras (10) et dans lequel l'élément de liaison (4) présente une pince sensiblement en forme de U avec deux branches (31) et dans lequel la pince sensiblement en forme de U présente un évidement (13) dans lequel le bras (10) peut être introduit.

8. Système de liaison selon la revendication 7, **caractérisé en ce que** l'élément de retenue (2) présente un premier côté et un deuxième côté, dans lequel le premier côté de l'élément de retenue (2) est en contact avec la bride (25) du premier corps (5) et dans lequel une saillie (46) périphérique est disposée sur le premier côté de l'élément de retenue (2).

9. Système de liaison selon l'une quelconque des revendications 6 ou 7, présentant en outre une membrane (1), qui est disposée entre la bride (25) du premier corps (5) et le premier côté de l'élément de retenue (2) du deuxième corps (3).

10. Système de liaison selon l'une quelconque des revendications 7 à 9, **caractérisé en ce que** le premier corps conduisant un fluide (5) et le deuxième corps conduisant un fluide (3) sont reliés l'un à l'autre de manière pivotante.

11. Système de liaison selon l'une quelconque des revendications précédentes, **caractérisé en ce que** le deuxième côté de l'élément de retenue (2) présente un élément de crochet (7) périphérique, dans lequel l'élément de crochet (7) est adapté pour entourer au moins en partie le côté extérieur de la bride (25) du premier corps (5).

12. Système de liaison selon l'une quelconque des revendications précédentes, **caractérisé en ce qu'**il présente
- un élément de liaison (4) avec un ou plusieurs leviers de serrage et un élément de retenue (2) avec au moins un élément de crochet (7) et/ou
- un élément de liaison (4) avec une ou plusieurs liaisons par encliquetage et un élément de retenue (2) avec un ou plusieurs éléments de crochet (7) et/ou
- un élément de liaison (4) avec deux leviers de serrage ou plus et/ou
- un élément de liaison (4) avec une pince sensiblement en forme de U et un élément de retenue (2) avec un ou plusieurs éléments de crochet (7).

13. Système de liaison selon l'une quelconque des revendications précédentes, **caractérisé en ce que** l'élément de liaison (4) est défini par une pince sensiblement en forme de U avec une paire supérieure de branches, une paire inférieure de branches et une paroi latérale (28) reliant chaque branche supérieure et inférieure, dans lequel la paroi latérale (28) présente un évidement (13) sur le côté de chaque branche,
dans lequel l'élément de retenue (2) présente une paire de bras (10), qui sont disposés parallèlement au plan de la bride (25),
dans lequel l'élément de retenue (2) est disposé entre l'élément supérieur et l'élément inférieur de la pince (6) sensiblement en forme de U, de sorte que les extrémités des bras (10) peuvent être introduites dans les ouvertures (13).

14. Procédé pour l'accouplement à force libérable a) d'un premier corps (5) à un système conducteur de fluide par l'intermédiaire d'une membrane (1) étanche au fluide à l'aide d'un élément de liaison (4) détachable, ou b) de l'espace intérieur d'un premier corps (5) conduisant un fluide à l'espace intérieur (23) d'un deuxième corps conducteur de fluide (3) à l'aide d'un élément de liaison (4) détachable, dans lequel le premier corps (5) présente une première extrémité avec une bride (25) et en outre une sortie de l'espace intérieur du premier corps se trouve dans la première extrémité du premier corps (5),
dans lequel dans a) le premier corps (5) présente une première extrémité avec une bride (25) et dans lequel un espace intérieur (23) d'un deuxième corps (3) est accouplé au système conducteur de fluide,
dans lequel dans a) le deuxième corps (3) présente une deuxième extrémité avec une paroi périphérique (22), ainsi que l'espace intérieur (23) avec une ouverture (43) de celui-ci, dans lequel la paroi périphérique (22) présente une section de contact de membrane (9) faisant le tour de l'ouverture (43), qui est en contact avec la membrane (1),
**caractérisé en ce que** dans a) la deuxième extrémité du deuxième corps (3) est en contact avec un élément de retenue (2) en forme de disque, qui définit un composant indépendant, dans lequel l'élément de retenue (2) en forme de disque présente un passage (35), un premier côté, un deuxième côté et un bord extérieur périphérique (24), dans lequel le premier côté de l'élément de retenue (2) est en contact avec la première extrémité du premier corps (5) et le premier côté et/ou le passage (35) de l'élément de retenue (2) en forme de disque est en outre accouplé à la membrane (1), et dans lequel le deuxième côté de l'élément de retenue (2) est configuré pour un contact à plat avec la bride (25) du premier corps (5),
dans lequel le procédé dans a) présente :
le fait d'orienter l'élément de retenue (2) en forme de disque se trouvant sur la deuxième extrémité du deuxième corps (3) et la première extrémité du premier corps (5) et de les amener en contact, de sorte que le deuxième côté de l'élément de retenue (2) en forme de disque repose sur la bride (25) du premier corps (5), et
ensuite dans (a) le fait de disposer l'élément de liaison (4) détachable, de sorte qu'il entoure au moins en partie la bride (25) du premier corps (5) et le bord extérieur périphérique (24) de l'élément de retenue en forme de disque, de sorte que l'élément de retenue (2) est fixé de manière réversible à force sur le premier corps (5) à l'aide de l'élément de liaison (4), ou **caractérisé en ce que** dans b) le deuxième corps conduisant un fluide (3) présente une deuxième extrémité, qui définit un élément de retenue (2) en forme de disque du type bride avec un bord extérieur périphérique (24) et une ouverture centrale (43), dans lequel l'ouverture centrale est une ouverture de l'espace intérieur (23) du deuxième corps conducteur de fluide (3),
dans lequel l'élément de retenue (2) en forme de disque du type bride présente en outre un ou plusieurs bras (10),
dans lequel le procédé dans b) présente :
le fait d'accoupler l'élément de retenue (2) en forme de disque du type bride à la bride (25) du premier corps (5) avec possibilité de liaison, de sorte que l'élément de retenue (2) repose sur la bride (25) du premier corps (5),
le fait de placer ensuite l'élément de liaison (4) détachable dans b) sur l'élément de retenue (2) en forme de disque du type bride et la bride (25) du premier corps (5) de sorte qu'il entoure au moins en partie le bord extérieur périphérique (24) de l'élément de retenue (2) en forme de disque du type bride et la bride (25) du premier corps (5), de sorte que l'élément de retenue (2) en forme de disque du type bride est fixé de manière réversible à force sur le premier corps (5) à l'aide de l'élément de liaison (4).
